(19) Europäisches Patentamt
European Patent Office
Office européen des brevets

(11) Publication number : **0 232 075 B1**

# (12) EUROPEAN PATENT SPECIFICATION

(45) Date of publication of patent specification : **13.03.91 Bulletin 91/11**

(51) Int. Cl.[5] : **C07C 243/38,** C07C 327/00, A01N 37/28

(21) Application number : **87300534.2**

(22) Date of filing : **22.01.87**

(54) **Insecticidal N'-substituted-N-alkylcarbonyl-N'-acyl hydrazines.**

The file contains technical information submitted after the application was filed and not included in this specification

(30) Priority : **22.01.86 US 821187**

(43) Date of publication of application : **12.08.87 Bulletin 87/33**

(45) Publication of the grant of the patent : **13.03.91 Bulletin 91/11**

(84) Designated Contracting States : **AT BE CH DE ES FR GB GR IT LI LU NL SE**

(56) References cited :
**DD-A- 212 889**
**FR-A- 2 042 302**
**FR-A- 2 342 959**

(56) References cited :
**HELVETICA CHIMICA ACTA, vol. 61, no. 4, 7th June 1978, pages 1477-1510; M. MÄRKY et al.: "Zum photochemischen Verhalten von Sydnonen und 1,3,4-Oxadiazolin-2-onen"**
**CHEMICAL ABSTRACTS, vol. 76, no. 21, 22nd May 1972, page 417, abstract no. 125990y, Columbus, Ohio, US; Q.N. PORTER et al.: "Mass spectrometric studies. XI. Skeletal rearrangements in acrylhydrazines"**

(73) Proprietor : **ROHM AND HAAS COMPANY**
**Independence Mall West**
**Philadelphia Pennsylvania 19105 (US)**

(72) Inventor : **Murphy, Raymond August**
**Forge Gate Apartments Apt. 38-B-1**
**Lansdale Pennsylvania 19446 (US)**
Inventor : **Hsu, Adam Chi-Tung**
**1686 Heebner Way**
**Landsdale Pennsylvania 19446 (US)**

(74) Representative : **Angell, David Whilton et al**
**ROHM AND HAAS (UK) LTD. European Operations Patent Department Lennig House**
**2 Mason's Avenue**
**Croydon CR9 3NB (GB)**

EP 0 232 075 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to N'-substituted-N-alkyl-carbonyl-N'-acylhydrazines which are useful as insecticides, compositions containing those compounds and their use as insecticides.

The search for compounds which have a combination of excellent insecticidal activity and low desirable toxicity is a continuing one because of factors such as the need for compounds exhibiting greater activity, better selectivity, low undesirable environmental impact, low production cost and effectiveness against insects resistant to many known insecticides.

Compounds of the present invention are particularly suitable for controlling plant-destructive insects in crops of cultivated plants, ornamentals and forestry.

Certain hydrazine and/or hydrazide derivatives have been disclosed in the literature.

In 25 Aust. J. Chem., 523-529 (1972), several N,N'-dibenzoylhydrazine derivatives are disclosed in which one or both nitrogen atoms are alkylated or phenylated. No biological activity is disclosed for those compounds.

In 61 Helv. Chim. Acta, 1477-1510 (1978), several N,N'-dibenzoylhydrazine and hydrazide derivatives are disclosed. No biological activity is disclosed for those compounds.

In 44 J.A.C.S., 2556-2567 (1922), isopropyl hydrazine $(CH_3)_2CH-NH-NH_2$, symmetrical diisopropyl hydrazine, dibenzoylisopropyl hydrazine and certain derivatives are disclosed. No biological activity is disclosed for those compounds.

In 44 J.A.C.S., 1557-1564 (1972), isopropyl, menthyl and bornyl semicarbazides are disclosed. No biological activity is disclosed for those compounds.

In 48 J.A.C.S., 1030-1035 (1926), symmetrical di-methylphenylmethyl hydrazine and certain related compounds including 1,2-bis-methylphenylmethyl-4-phenyl-semicarbazide are disclosed. No biological activity is disclosed for those compounds.

In 27 Bull. Chem. Soc. Japan, 624-627 (1954), certain hydrazine derivatives including alpha, beta-dibenzoylphenyl hydrazine are disclosed. No biological activity is disclosed for those compounds.

In J. Chem. Soc. (C), 1531-1536 (1966), N,N'-dibenzoylphenyl hydrazine and N-acetyl-N'-benzoyl-p-nitrophenyl hydrazine are disclosed. No biological activity is disclosed for those compounds.

In 56B Chem. Berichte, 954-962 (1923), symmetrical di-isopropyl hydrazines, symmetrical diisobutyl and certain derivatives including N,N'-di-isobutyldibenzoyl hydrazine are disclosed. No biological activity is disclosed for those compounds.

In 590 Annalen der Chemie, 1-36 (1954), certain N,N'-dibenzoyl hydrazine derivatives are disclosed including N'-methyl- and N'-(2-phenyl)-isopropyl-N,N'-dibenzoyl hydrazine. No biological activity is disclosed for those compounds.

In J. Chem. Soc., 4191-4198 (1952), N,N'-di-n-propyl hydrazine and the bis-3,5-dinitrobenzoyl derivatives are disclosed. No biological activity is disclosed for those compounds.

In 32 Zhur. Obs. Khim., 2806-2809 (1962), N'-2,4-methyl-2,4-pentadiene-N,N'-dibenzoyl hydrazine is disclosed. No biological activity is disclosed.

In 17 Acta. Chim. Scand., 95-102 (1963), 2-benzoyl-thiobenzhydrazide ($C_6H_5-CS-NHNH-CO-C_6H_5$) and certain hydrazone and hydrazine derivatives are disclosed including 1,2-dibenzoyl-benzyl hydrazine. No biological activity is disclosed for those compounds.

In 25 Zhur. Obs. Khim, 1719-1723 (1955), N,N'-bis-cyclohexyl hydrazine and N,N'-dibenzoylcyclohexyl hydrazine are disclosed. No biological activity is disclosed for those compounds.

In J. Chem. Soc., 4793-4800 (1964), certain dibenzoyl hydrazine derivatives are disclosed including tribenzoyl hydrazine and N,N'-dibenzoylcyclohexyl hydrazine. No biological activity is disclosed for those compounds.

In 36 J. Prakt. Chem., 197-201 (1967), certain dibenzoyl hydrazine derivatives including N'-ethyl-; N'-n-propyl-; N'-isobutyl-; N'-neopentyl-; N'-n-heptyl-; and N'-cyclohexylmethyl-N,N'-dibenzoyl hydrazines are disclosed. No biological activity is disclosed for those compounds.

In 26 J.O.C., 4336-4340 (1961) N'-t-butyl-N,N'-di-(t-butoxycarbonyl) hydrazide is disclosed. No biological activity is disclosed.

In 41 J.O.C., 3763-3765 (1976), N'-t-butyl-N-(phenylmethoxycarbonyl)-N'-(chlorocarbonyl)hydrazide is disclosed. No biological activity is disclosed.

In 94 J.A.C.S., 7406-7416 (1972) N'-t-butyl-N,N'-dimethoxycarbonyl hydrazide is disclosed. No biological activity is disclosed.

In 43 J.O.C., 808-815 (1978), N't-butyl-N-ethoxycarbonyl-N'-phenylaminocarbonyl-hydrazide and N'-t-butyl-N-ethoxycarbonyl-N'-methylaminocarbonyl hydrazide are disclosed. No biological activity is disclosed for those compounds.

In 39 J. Econ. Ent., 416-417 (1946), certain N-phenyl-N'-acylhydrazines are disclosed and evaluated for

their toxicity against codling moth larvae.

The N'-substituted-N-alkylcarbonyl-N'-acryl-hydrazines of the present invention differ from known compounds primarily by their N- and N'-substituents.

Compounds of the present invention are distinguished by their excellent insecticidal activity against insects of the orders Lepidoptera and Coleoptera.

In accordance with the present invention, there are provided insecticidal compounds, compositions containing them and methods of using such compounds and compositions wherein the compounds are those having the formula :

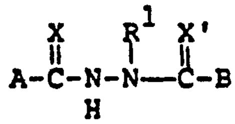

I

wherein

X and X' are the same or different O, S or NR ;

$R^1$ is unsubstituted ($C_3$-$C_{10}$) branched alkyl or ($C_1$-$C_4$) straight chain alkyl substituted with one or two of the same or different ($C_3$-$C_6$)cycloalkyl ;

A is unsubstituted or substituted ($C_1$-$C_{10}$)alkyl having one to four of the same or different halo, cyano, nitro, hydroxy, ($C_1$-$C_4$)alkoxy, carboxy, ($C_1$-$C_4$)alkoxy-carbonyl, ($C_1$-$C_4$)alkanoyloxy, $NH_2$, NHZ or NZZ' ;

unsubstituted or substituted ($C_3$-$C_8$)cyclo-alkyl or ($C_3$-$C_8$)cycloalkyl-($C_1$-$C_4$)alkyl having one to four of the same or different halo, cyano, nitro, hydroxy, ($C_1$-$C_4$)alkyl, halo($C_1$-$C_4$)alkyl, ($C_1$-$C_4$)-alkoxy, halo($C_1$-$C_4$)alkoxy, carboxy, ($C_1$-$C_4$)alkanoyl, ($C_1$-$C_4$)alkoxy-carbonyl, ($C_1$-$C_4$)alkanoyloxy, $NH_2$, NHZ OR NZZ';

unsubstituted or substituted ($C_2$-$C_8$)alkenyl or unsubstituted or substituted ($C_3$-$C_8$)-alkadienyl having one to four of the same or different halo, cyano, nitro, hydroxy, ($C_1$-$C_4$)alkyl, ($C_3$-$C_8$)cloalkyl, halo-($C_1$-$C_4$)alkyl, ($C_1$-$C_4$)alkoxy, halo($C_1$-$C_4$)-alkoxy, carboxy, ($C_1$-$C_4$)alkoxy-carbonyl, ($C_1$-$C_4$)alkanoyloxy, $NH_2$, NHZ or NZZ' ;

unsubstituted or substituted ($C_3$-$C_8$)cycloalkenyl or unsubstituted or substituted ($C_6$-$C_8$) cycloalkadienyl having one to four of the same or different halo, cyano, nitro, hydroxy, ($C_1$-$C_4$)alkyl, halo($C_1$-$C_4$)-alkyl, ($C_1$-$C_4$)alkoxy, halo($C_1$-$C_4$)alkoxy, carboxy, ($C_1$-$C_4$)alkoxy-carbonyl, ($C_1$-$C_4$)-alkanoyloxy, $NH_2$, NHZ or NZZ' ;

unsubstituted or substituted ($C_2$-$C_8$)alkynyl having one to four of the same or different halo, cyano, nitro, hydroxy, ($C_1$-$C_4$)alkyl, halo($C_1$-$C_4$)alkyl, ($C_1$-$C_4$)alkoxy, halo-($C_1$-$C_4$)alkoxy, carboxy, ($C_1$-$C_4$)-alkoxy-carbonyl, ($C_1$-$C_4$)alkanoyloxy, $NH_2$, NHZ or NZZ' ; or

phenalkyl having one to four carbon atoms in the alkyl group and the phenyl ring is unsubstituted or substituted with one to three of the same or different halo, cyano, nitro, hydroxy, ($C_1$-$C_4$)alkyl, halo($C_1$-$C_4$)-alkyl, cyano($C_1$-$C_4$)alkyl, ($C_1$-$C_4$)alkoxy, halo($C_1$-$C_4$)alkoxy, carboxy, ($C_1$-$C_4$)-alkoxy-carbonyl, ($C_1$-$C_4$)alkanoyloxy, ($C_2$-$C_6$)alkenyl, halo($C_2$-$C_6$)alkenyl, ($C_2$-$C_6$)alkynyl, $NH_2$, NHZ or NZZ' ;

B is unsubstituted or substituted naphthyl where the substituents can be from one to three of the same or different halo ; cyano ; nitro ; hydroxy ; ($C_1$-$C_4$)alkoxy ; ($C_1$-$C_4$)-alkyl ; carboxy ; ($C_1$-$C_4$)alkoxy-carbonyl ; ($C_1$-$C_4$)alkanoyloxy ; $NH_2$, NHZ or NZZ' ;

unsubstituted or substituted phenyl where the substituents can be from one to five of the same or different halo ; nitro ; cyano ; hydroxy ; ($C_1$-$C_6$)alkyl ; halo($C_1$-$C_6$)alkyl ; cyano($C_1$-$C_6$)alkyl ; ($C_1$-$C_6$)alkoxy; halo-($C_1$-$C_8$)alkoxy ; alkoxyalkyl having independently 1 to 6 carbon atoms in each alkyl group ; alkoxyalkoxy having independently 1 to 6 carbon atoms in each alkyl group ; ($-OCO_2R$) group ; ($C_2$-$C_6$)alkenyl optionally substituted with halo, cyano, ($C_1$-$C_4$)alkyl, ($C_1$-$C_4$)alkoxy, halo($C_1$-$C_4$)-alkoxy or ($C_1$-$C_4$)alkylthio ; $-RCO_2R'$ group ; $-COR$ group ; halo($C_1$-$C_6$)alkyl-carbonyl ; cyano($C_1$-$C_6$)alkyl-carbonyl ; nitro ($C_1$-$C_6$)-alkyl-carbonyl ; $-CO_2R$ group ; halo($C_1$-$C_6$)-alkoxy-carbonyl ; $-OCOR$ group ; $-NRR'$ group ; amino substituted with hydroxy, ($C_1$-$C_4$)-alkoxy or ($C_1$-$C_4$)alkylthio groups ; phenylamino; diphenylamino ; $-CONRR'$ group ; $-OCONRR'$ group ; $-NRCOR'$ group ; $-NRCO_2R'$ group ; $-N(COR)COR'$ group ; $-OCONRCOR'$ group ; sulfhydryl ; halothio ; ($C_1$-$C_6$)alkylthio ; halo($C_1$-$C_6$)alkylthio ; sulfinyl $-SOR$ group ; $-SO_2R$ group ; phenylsulfonyl ; $-OSO_2R$ group ; halo($C_1$-$C_6$)alkylsulfonyloxy ; $-SO_2NRR'$ group ; $-NRSOR'$ group ; $-NRSO_2R'$ group ; $-CSR$ group ; $-CS_2R$ group ; $-NRCSR'$ group ; $-SCOR$ group ; unsubstituted or substituted phenyl having one to three of the same or different halo, cyano, nitro, hydroxy($C_1$-$C_4$)alkyl, ($C_1$-$C_4$)alkoxy, carboxy, ($C_1$-$C_4$)alkoxy-carbonyl, ($C_1$-$C_4$)alkanoyloxy, $NH_2$ group, NHZ group or NZZ' group ; phenoxy where the phenyl ring is unsubstituted or substituted with one to three of the same or different halo, cyano, nitro, hydroxy,

($C_1$-$C_4$)alkyl, ($C_1$-$C_4$)alkoxy, carboxy, ($C_1$-$C_4$)alkoxy-carbonyl, ($C_1$-$C_4$)alkanoyloxy, $NH_2$ group, NHZ group or NZZ′ group ; phenylthio where the phenyl ring is unsubstituted or substituted with one to three of the same or different halo, cyano, nitro, hydroxy, ($C_1$-$C_4$)alky, ($C_1$-$C_4$)alkoxy, carboxy, ($C_1$-$C_4$)alkoxy-carbonyl, ($C_1$-$C_4$)-alkanoyloxy, $NH_2$ group, NHZ group or NZZ′ group ; or when two adjacent positions on the phenyl ring are substituted with alkoxy groups, these groups may be joined to form, together with the carbon atoms to which they are attached, a 5 or 6 membered dioxolano or dioxano heterocyclic ring;

where R, R′ and R" are hydrogen or ($C_1$-$C_6$)-alkyl ; Z and Z′ are ($C_1$-$C_4$)alkyl and "amino" means NRR′ ; and agronomically acceptable salts thereof.

The compositions of the invention also contain agronomically acceptable diluent or carrier.

The term "halo" should be understood as including chloro, fluoro, bromo and iodo. The term "alkyl" by itself or as a part of another substituent, unless otherwise stated, includes straight or branched chain groups such as methyl, ethyl, n-propyl, isopropyl, n-butyl, t-butyl, isobutyl and neopentyl and where indicated higher homologues and isomers such as n-octyl and isooctyl. The term "haloalkyl" by itself or as part of another substituent is an alkyl group of the stated number of carbon atoms having one or more halo atoms bonded thereto such as chloromethyl, 1- or 2-bromoethyl and trifluoromethyl. Analogously, "cyanoalkyl" by itself or as part of another group is an alkyl group of the stated number of carbon atoms having one or more cyano groups bonded thereto ; "haloalkoxy" by itself or as part of another group is an alkoxy group of the stated number of carbon atoms having one or more halo atoms bonded thereto such as difluoromethoxy, trifluoromethoxy, 2-fluoroethoxy and 2,2,2-trifluoroethoxy. "Alkenyl" and "alkynyl" by themselves or as part of another substituent comprise straight and branched chain groups of the stated number of carbon atoms. "Alkadienyl" is a straight or branched chain alkenyl group comprising two carboncarbon double bonds that can be conjugated such as 1,3-butadienyl, cumulated such as 1,2-propadienyl or isolated such as 1,4-pentadienyl.

Specially interesting compounds, within the scope of the present invention include :

N′-t-butyl-N-acetyl-N′-benzoylhydrazine
N′-t-butyl-N-propionyl-N′-benzoylhydrazine
N′-t-butyl-N-butyryl-N′-benzoylhydrazine
N′-t-butyl-N-valeryl-N′-benzoylhydrazine
N′-t-butyl-N-hexanoyl-N′-benzoylhydrazine
N′-t-butyl-N-heptanoyl-N′-benzoylhydrazine
N′-t-butyl-N-octanoyl-N′-benzoylhydrazine
N′-t-butyl-N-nonanoyl-N′-benzoylhydrazine
N′-t-butyl-N-decanoyl-N′-benzoylhydrazine
N′-t-butyl-N-undecanoyl-N′-benzoylhydrazine
N′-t-butyl-N-cyclopropylcarbonyl-N′-benzoylhydrazine
N′-t-butyl-N-cyclobutylcarbonyl-N′-benzoylhydrazine
N′-t-butyl-N-cyclohexylcarbonyl-N′-benzoylhydrazine
N′-t-butyl-N-cycloheptylcarbonyl-N′-benzoylhydrazine
N′-t-butyl-N-cyclooctylcarbonyl-N′-benzoylhydrazine
N′-t-butyl-N-acryloyl-N′-benzoylhydrazine
N′-t-butyl-N-allylcarbonyl-N′-benzoylhydrazine
N′-t-butyl-N-(1-propenylcarbonyl)-N′-benzoylhydrazine
N′-t-butyl-N-crotonoyl-N′-benzoylhydrazine
N′-t-butyl-N-isocrotonyl-N′-benzoylhydrazine
N′-t-butyl-N-cyclopropenylcarbonyl-N′-benzoylhydrazine
N′-t-butyl-N-cyclobutenylcarbonyl-N′-benzoylhydrazine
N′-t-butyl-N-cyclopentylcarbonyl-N′-benzoylhydrazine
N′-t-butyl-N-cyclohexenylcarbonyl-N′-benroylhydrazine
N′-t-butyl-N-cycloheptenylcarbonyl-N′-benzoylhydrazine
N′-t-butyl-N-cyclooctenylcarbonyl-N′-benzoylhydrazine
N′-t-butyl-N-propioloyl-N′-benzoylhydrazine
N′-t-butyl-N-(1-butynylcarbonyl)-N′-benzoylhydrazine
N′-t-butyl-N-(1-pentynylcarbonyl)-N′-benzoylhydrazine
N′-t-butyl-N-(1-hexynylcarbonyl)-N′-benzoylhydrazine
N′-t-butyl-N-(1-heptynylcarbonyl)-N′-benzoylhydrazine
N′-t-butyl-N-(1-octynylcarbonyl)-N′-benzoylhydrazine
N′-t-butyl-N-(2-butynylcarbonyl)-N′-benzoylhydrazine
N′-t-butyl-N-(2-pentynylcarbonyl)-N′-benzoylhydrazine
N′-t-butyl-N-(3-methyl-1-butynylcarbonyl)-N′-benzoylhydrazine

N′-t-butyl-N-(2-hexynylcarbonyl)-N′-benzoylhydrazine
N′-t-butyl-N-(3-hexynylcarbonyl)-N′-benzoylhydrazine
N′-t-butyl-N-(3,3-dimethyl-1-butynylcarbonyl)-N′-benzoylhydrazine
N′-t-butyl-N-(4-octynylcarbony-1)-N′-benzoylhydrazine
N′-t-butyl-N-benzylcarbonyl-N′-benzoylhydrazine
N′-t-butyl-N-phenethylcarbonyl-N′-benzoylhydrazine
N′-t-butyl-N-(3-phenylpropylcarbonyl)-N′-benzoylhydrazine
N′-t-butyl-N-(4-phenylbutylcarbonyl)-N′-benzoylhydrazine
N′-t-butyl-N-(4-(4-chlorophenyl)butylcarbonyl)-N′-benzoylhydrazine
N′-t-butyl-N-acetyl-N′-(4-chloroberzoyl)hydrazine
N′-t-butyl-N-propionyl-N′-(4-chlorobenzoyl)hydrazine
N′-t-butyl-N-butyryl-N′-(4-chlorobenzoyl)hydrazine
N′-t-butyl-N-valeryl-N′-(4-chlorobenzoyl)hydrazine
N′-t-butyl-N-hexanoyl-N′-(4-chlorobenzoyl)hydrazine
N′-t-butyl-N-heptanoyl-N′-(4-chlorobenzoyl)hydrazine
N′-t-butyl-N-octanoyl-N′-(4-chlorobenzoyl)hydrazine
N′-t-butyl-N-nonanoyl-N′-(4-chlorobenzoyl)hydrazine
N′-t-butyl-N-decanoyl-N′-(4-chlorobenzoyl)hydrazine
N′-t-butyl-N-undecanoyl-N′-(4-chlorobenzoyl)hydrazine
N′-t-butyl-N-cyclopropylcarbonyl-N′-(4-chlorobenzoyl)hydrazine
N′-t-butyl-N-cyclobutylcarbonyl-N′-(4-chlorobenzoyl)hydrazine
N′-t-butyl-N-cyclohexylcarbonyl-N′-(4-chlorobenzoyl)hydrazine
N′-t-butyl-N-cycloheptylcarbonyl-N′-(4-chlorobenzoyl)hydrazine
N′-t-butyl-N-cyclooctylcarbonyl-N′-(4-chlorobenzoyl)hydrazine
N′-t-butyl-N-acryloyl-N′-(4-chlorobenzoyl)hydrazine
N′-t-butyl-N-allylcarbonyl-N′-(4-chlorobenzoyl)hydrazine
N′-t-butyl-N-(1-propenylcarbonyl)-N′-(4-chlorobenzoyl)hydrazine
N′-t-butyl-N-crotonoyl-N′-(4-chlorobenzoyl)hydrazine
N′-t-butyl-N-isocrotonoyl-N′-(4-chlorobenzoyl)hydrazine
N′-t-butyl-N-cyclopropenylcarbonyl-N′-(4-chlorobenzoyl)hydrazine
N′-t-butyl-N-cyclobutenylcarbonyl-N′-(4-chlorobenzoyl)hydrazine
N′-t-butyl-N-cyclopentylcarbonyl-N′-(4-chlorobenzoyl)hydrazine
N′-t-butyl-N-cyclohexenylcarbonyl-N′-(4-chlorobenzoyl)hydrazine
N′-t-butyl-N-cycloheptenylcarbonyl-N′-(4-chlorobenzoyl)hydrazine
N′-t-butyl-N-cyclooctenylcarbonyl-N′-(4-chlorobenzoyl)hydrazine
N′-t-butyl-N-propioloyl-N′-(4-chlorobenzoyl)hydrazine
N′-t-butyl-N-(1-butynylcarbonyl)-N′-(4-chlorobenzoyl)hydrazine
N′-t-butyl-N-(1-pentynylcarbonyl)-N′-(4-chlorobenzoyl)hydrazine
N′-t-butyl-N-(1-hexynylcarbonyl)-N′-(4-chlorobenzoyl)hydrazine
N′-t-butyl-N-(1-heptynylcarbonyl)-N′-(4-chlorobenzoyl)hydrazine
N′-t-butyl-N-(1-octynylcarbonyl)-N′-(4-chlorobenzoyl)hydrazine
N′-t-butyl-N-(2-butynylcarbonyl)-N′-(4-chlorobenzoyl)hydrazine
N′-t-butyl-N-(2-pentynylcarbonyl)-N′-(4-chlorobenzoyl)hydrazine
N′-t-butyl-N-(3-methyl-1-butynylcarbonyl)-N′-(4-chlorobenzoyl)hydrazine
N′-t-butyl-N-(2-hexynylcarbonyl)-N′-(4-chlorobenzoyl)hydrazine
N′-t-butyl-N-(3-hexynylcarbonyl)-N′-(4-chlorobenzoyl)hydrazine
N′-t-butyl-N-(3,3-dimethyl-1-butynylcarbonyl)-N′-(4-chlorobenzoyl)hydrazine
N′-t-butyl-N-(4-octynylcarbonyl)-N′-(4-chlorobenzoyl)hydrazine
N′-t-butyl-N-benzylcarbonyl-N′-(4-chlorobenzoyl)hydrazine
N′-t-butyl-N-phenethylcarbonyl-N′-(4-chlorobenzoyl)hydrazine
N′-t-butyl-N-(3-phenylpropylcarbonyl)-N′-(4-chlorobenzoyl)hydrazine
N′-t-butyl-N-(4-phenylbutylcarbonyl)-N′-(4-chlorobenzoyl)hydrazine
N′-t-butyl-N-(4-(4-chlorophenyl)butylcarbonyl)-N′-(4-chlorobenzoyl)hydrazine
N′-t-butyl-N-acetyl-N′-(3-toluoyl)hydrazine
N′-t-butyl-N-propionyl-N′-(3-toluoyl)hydrazine
N′-t-butyl-N-butyryl-N′-(3-toluoyl)hydrazine
N′-t-butyl-N-valeryl-N′-(3-toluoyl)hydrazine
N′-t-butyl-N-hexanoyl-N′-(3-toluoyl)hydrazine

N'-t-butyl-N-heptanoyl-N'-(3-toluoyl)hydrazine
N'-t-butyl-N-octanoyl-N'-(3-toluoyl)hydrazine
N'-t-butyl-N-nonanoyl-N'-(3-toluoyl)hydrazine
N'-t-butyl-N-decanoyl-N'-(3-toluoyl)hydrazine
N'-t-butyl-N-undecanoyl-N'-(3-toluoyl)hydrazine
N'-t-butyl-N-cyclopropylcarbonyl-N'-(3-toluoyl)hydrazine
N'-t-butyl-N-cyclobutylcarbonyl-N'-(3-toluoyl)hydrazine
N'-t-butyl-N-cyclohexylcarbonyl-N'-(3-toluoyl)hydrazine
N'-t-butyl-N-cycloheptylcarbonyl-N'-(3-toluoyl)hydrazine
N'-t-butyl-N-cyclooctylcarbonyl-N'-(3-toluoyl)hydrazine
N'-t-butyl-N-acryloyl-N'-(3-toluoyl)hydrazine
N'-t-butyl-N-allylcarbonyl-N'-(3-toluoyl)hydrazine
N'-t-butyl-N-(1-propenylcarbonyl)-N'-(3-toluoyl)hydrazine
N'-t-butyl-N-crotonoyl-N'-(3-toluoyl)hydrazine
N'-t-butyl-N-isocrotonoyl-N'-(3-toluoyl)hydrazine
N'-t-butyl-N-cyclopropenylcarbonyl-N'-(3-toluoyl)hydrazine
N'-t-butyl-N-cyclobutenylcarbonyl-N'-(3-toluoyl)hydrazine
N'-t-butyl-N-cyclopentylcarbonyl-N'-(3-toluoyl)hydrazine
N'-t-butyl-N-cyclohexenylcarbonyl-N'-(3-toluoyl)hydrazine
N'-t-butyl-N-cycloheptenylcarbonyl-N'-(3-toluoyl)hydrazine
N'-t-butyl-N-cyclooctenylcarbonyl-N'-(3-toluoyl)hydrazine
N'-t-butyl-N-propioloyl-N'-(3-toluoyl)hydrazine
N'-t-butyl-N-(1-butynylcarbonyl)-N'-(3-toluoyl)hydrazine
N'-t-butyl-N-(1-pentynylcarbonyl)-N'-(3-toluoyl)hydrazine
N'-t-butyl-N-(1-hexynylcarbonyl)-N'-(3-toluoyl)hydrazine
N'-t-butyl-N-(1-heptynylcarbonyl)-N'-(3-toluoyl)hydrazine
N'-t-butyl-N-(1-octynylcarbonyl)-N'-(3-toluoyl)hydrazine
N'-t-butyl-N-(2-butynylcarbonyl)-N'-(3-toluoyl)hydrazine
N'-t-butyl-N-(2-pentynylcarbonyl)-N'-(3-toluoyl)hydrazine
N'-t-butyl-N-(3-methyl-1-butynylcarbonyl)-N'-(3-toluoyl)hydrazine
N'-t-butyl-N-(2-hexynylcarbonyl)-N'-(3-toluoyl)hydrazine
N'-t-butyl-N-(3-hexynylcarbonyl)-N'-(3-toluoyl)hydrazine
N'-t-butyl-N-(3,3-dimethyl-1-butynylcarbonyl)-N'-(3-toluoyl)hydrazine
N'-t-butyl-N-(4-octynylcarbonyl)-N'-(3-toluoyl)hydrazine
N'-t-butyl-N-benzylcarbonyl-N'-(3-toluoyl)hydrazine
N'-t-butyl-N-phenethylcarbonyl-N'-(3-toluoyl)hydrazine
N'-t-butyl-N-(3-phenylpropylcarbonyl)-N'-(3-toluoyl)hydrazine
N'-t-butyl-N-(4-phenylbutylcarbonyl)-N'-(3-toluoyl)hydrazine
N'-t-butyl-N-(4-(4-chlorophenyl)butylcarbonyl)-N'(3-toluoyl)hydrazine
N'-t-butyl-N-acetyl-N'-(2-methoxybenzoyl)hydrazine
N'-t-butyl-N-propionyl-N'-(2-methoxybenzoyl)hydrazine
N'-t-butyl-N-butyryl-N'-(2-methoxybenzoyl)hydrazine
N'-t-butyl-N-valeryl-N'-(2-methoxybenzoyl)hydrazine
N'-t-butyl-N-hexanoyl-N'-(2-methoxybenzoyl)hydrazine
N'-t-butyl-N-heptanoyl-N'-(2-methoxybenzoyl)hydrazine
N'-t-butyl-N-octanoyl-N'-(2-methoxybenzoyl)hydrazine
N'-t-butyl-N-nonanoyl-N'-(2-methoxybenzoyl)hydrazine
N'-t-butyl-N-decanoyl-N'-(2-methoxybenzoyl)hydrazine
N'-t-butyl-N-undecanoyl-N'-(2-methoxybenzoyl)hydrazine
N'-t-butyl-N-cyclopropylcarbonyl-N'-(2-methoxybenzoyl)hydrazine
N'-t-butyl-N-cyclobutylcarbonyl-N'-(2-methoxybenzoyl)hydrazine
N'-t-butyl-N-cyclohexylcarbonyl-N'-(2-methoxybenzoyl)hydrazine
N'-t-butyl-N-cycloheptylcarbonyl-N'-(2-methoxybenzoyl)hydrazine
N'-t-butyl-N-cyclooctylcarbonyl-N'-(2-methoxybenzoyl)hydrazine
N'-t-butyl-N-acryloyl-N'-(2-methoxybenzoyl)hydrazine
N'-t-butyl-N-allylcarbonyl-N'-(2-methoxybenzoyl)hydrazine
N'-t-butyl-N-(1-propenylcarbonyl)-N'-(2-methoxybenzoyl)hydrazine
N'-t-butyl-N-crotonoyl-N'-(2-methoxybenzoyl)hydrazine

N'-t-butyl-N-isocrotonoyl-N'-(2-methoxybenzoyl)hydrazine
N'-t-butyl-N-cyclopropenylcarbonyl-N'-(2-methoxybenzoyl)hydrazine
N'-t-butyl-N-cyclobutenylcarbonyl-N'-(2-methoxybenzoyl)hydrazine
N'-t-butyl-N-cyclopentylcarbonyl-N'-(2-methoxybenzoyl)hydrazine
N'-t-butyl-N-cyclohexenylcarbonyl-N'-(2-methoxybenzoyl)hydrazine
N'-t-butyl-N-cycloheptenylcarbonyl-N'-(2-methoxybenzoyl)hydrazine
N'-t-butyl-N-cyclooctenylcarbonyl-N'-(2-methoxybenzoyl)hydrazine
N'-t-butyl-N-propioloyl-N'-(2-methoxybenzoyl)hydrazine
N'-t-butyl-N-(1-butynylcarbonyl)-N'-(2-methoxybenzoyl)hydrazine
N'-t-butyl-N-(1-pentynylcarbonyl)-N'-(2-methoxybenzoyl)hydrazine
N'-t-butyl-N-(1-hexynylcarbonyl)-N'-(2-methoxybenzoyl)hydrazine
N'-t-butyl-N-(1-heptynylcarbonyl)-N'-(2-methoxybenzoyl)hydrazine
N'-t-butyl-N-(1-octynylcarbonyl)-N'-(2-methoxybenzoyl)hydrazine
N'-t-butyl-N-(2-butynylcarbonyl)-N'-(2-methoxybenzoyl)hydrazine
N'-t-butyl-N-(2-pentynylcarbonyl)-N'-(2-methoxybenzoyl)hydrazine
N'-t-butyl-N-(3-methyl-1-butynylcarbonyl)-N'-(2-methoxybenzoyl)hydrazine
N'-t-butyl-N-(2-hexynylcarbonyl)-N'-(2-methoxybenzoyl)hydrazine
N'-t-butyl-N-(3-hexynylcarbonyl)-N'-(2-methoxybenzoyl)hydrazine
N'-t-butyl-N-(3,3-dimethyl-1-butynylcarbonyl)-N'-(2-methoxybenzoyl)hydrazine
N'-t-butyl-N-(4-octynylcarbonyl)-N'-(2-methoxybenzoyl)hydrazine
N'-t-butyl-N-benzylcarbonyl-N'-(2-methoxybenzoyl)hydrazine
N'-t-butyl-N-phenethylcarbonyl-N'-(2-methoxybenzoyl)hydrazine
N'-t-butyl-N-(3-phenylpropylcarbonyl)-N'-(2-methoxybenzoyl)hydrazine
N'-t-butyl-N-(4-phenylbutylcarbonyl)-N'-(2-methoxybenzoyl)hydrazine
N'-t-butyl-N-(4-(4-chlorophenyl)butylcarbonyl)-N'-(2-methoxybenzoyl)hydrazine
N'-t-butyl-N-acetyl-N'-(3-fluorobenzoyl)hydrazine
N'-t-butyl-N-propionyl-N'-(3-fluorobenzoyl)hydrazine
N'-t-butyl-N-butyryl-N'-(3-fluorobenzoyl)hydrazine
N'-t-butyl-N-valeryl-N'-(3-fluorobenzoyl)hydrazine
N'-t-butyl-N-hexanoyl-N'-(3-fluorobenzoyl)hydrazine
N'-t-butyl-N-heptanoyl-N'-(3-fluorobenzoyl)hydrazine
N'-t-butyl-N-octanoyl-N'-(3-fluorobenzoyl)hydrazine
N'-t-butyl-N-nonanoyl-N'-(3-fluorobenzoyl)hydrazine
N'-t-butyl-N-decanoyl-N'-(3-fluorobenzoyl)hydrazine
N'-t-butyl-N-undecanoyl-N'-(3-fluorobenzoyl)hydrazine
N'-t-butyl-N-cyclopropylcarbonyl-N'-(3-fluorobenzoyl)hydrazine
N'-t-butyl-N-cyclobutylcarbonyl-N'-(3-fluorobenzoyl)hydrazine
N'-t-butyl-N-cyclohexylcarbonyl-N'-(3-fluorobenzoyl)hydrazine
N'-t-butyl-N-cycloheptylcarbonyl-N'-(3-fluorobenzoyl)hydrazine
N'-t-butyl-N-cyclooctylcarbonyl-N'-(3-fluorobenzoyl)hydrazine
N'-t-butyl-N-acryloyl-N'-(3-fluorobenzoyl)hydrazine
N'-t-butyl-N-allylcarbonyl-N'-(3-fluorobenzoyl)hydrazine
N'-t-butyl-N-(1-propenylcarbonyl)-N'-(3-fluorobenzoyl)hydrazine
N'-t-butyl-N-crotonoyl-N'-(3-fluorobenzoyl)hydrazine
N'-t-butyl-N-isocrotonoyl-N'-(3-fluorobenzoyl)hydrazine
N'-t-butyl-N-cyclopropenylcarbonyl-N'-(3-fluorobenzoyl)hydrazine
N'-t-butyl-N-cyclobutenylcarbonyl-N'-(3-fluorobenzoyl)hydrazine
N'-t-butyl-N-cyclopentylcarbonyl-N'-(3-fluorobenzoyl)hydrazine
N'-t-butyl-N-cyclohexenylcarbonyl-N'-(3-fluorobenzoyl)hydrazine
N'-t-butyl-N-cycloheptenylcarbonyl-N'-(3-fluorobenzoyl)hydrazine
N'-t-butyl-N-cyclooctenylcarbonyl-N'-(3-fluorobenzoyl)hydrazine
N'-t-butyl-N-propioloyl-N'-(3-fluorobenzoyl)hydrazine
N'-t-butyl-N-(1-butynylcarbonyl)-N'-(3-fluorobenzoyl)hydrazine
N'-t-butyl-N-(1-pentynylcarbonyl)-N'-(3-fluorobenzoyl)hydrazine
N'-t-butyl-N-(1-hexynylcarbonyl)-N'-(3-fluorobenzoyl)hydrazine
N'-t-butyl-N-(1-heptynylcarbonyl)-N'-(3-fluorobenzoyl)hydrazine
N'-t-butyl-N-(1-octynylcarbonyl)-N'-(3-fluorobenzoyl)hydrazine
N'-t-butyl-N-(2-butynylcarbonyl)-N'-(3-fluorobenzoyl)hydrazine

N'-t-butyl-N-(2-pentynylcarbonyl)-N'-(3-fluorobenzoyl)hydrazine
N'-t-butyl-N-(3-methyl-1-butynylcarbonyl)-N'-(3-fluorobenzoyl)hydrazine
N'-t-butyl-N-(2-hexynylcarbonyl)-N'-(3-fluorobenzoyl)hydrazine
N'-t-butyl-N-(3-hexynylcarbonyl)-N'-(3-fluorobenzoyl)hydrazine
N'-t-butyl-N-(3,3-dimethyl-1-butynylcarbonyl)-N'-(3-fluorobenzoyl)hydrazine
N'-t-butyl-N-(4-octynylcarbonyl)-N'-(3-fluorobenzoyl)hydrazine
N'-t-butyl-N-benzylcarbonyl-N'-(3-fluorobenzoyl)hydrazine
N'-t-butyl-N-phenethylcarbonyl-N'-(3-fluorobenzoyl)hydrazine
N'-t-butyl-N-(3-phenylpropylcarbonyl)-N'-(3-fluorobenzoyl)hydrazine
N'-t-butyl-N-(4-phenylbutylcarbonyl)-N'-(3-fluorobenzoyl)hydrazine
N'-t-butyl-N-(4-(4-chlorophenyl)butylcarbonyl)-N'-(3-fluorobenzoyl)hydrazine

Because of their good insecticidal activity, preferred compounds of the present invention for use in the insecticidal compositions and formulations include those where, any one or any combination of two or more of the substituents conforms to the following definitions :

X and X' are O or S ;
$R^1$ is unsubstituted ($C_3$-$C_8$) branched alkyl or ($C_1$-$C_4$) straight chain alkyl substituted with one or two of the same or different ($C_3$-$C_4$)cycloalkyl ;
A is unsubstituted or substituted ($C_1$-$C_6$)alkyl having one to three of the same or different halo, cyano, nitro, ($C_1$-$C_4$)alkoxy, carboxy, ($C_1$-$C_4$)alkoxy-carbonyl, ($C_1$-$C_4$)alkanoyloxy, $NH_2$, NHZ or NZZ' ;
($C_3$-$C_6$)cycloalkyl ;
($C_2$-$C_6$) unsubstituted or substituted alkenyl having one to three of the same or different halo, ($C_1$-$C_4$) alkyl, halo($C_1$-$C_4$)alkyl, ($C_1$-$C_4$)alkoxy or halo($C_1$-$C_4$)alkoxy ;
($C_3$-$C_6$)cycloalkenyl ; or
phenalkyl having one or two carbon atoms in the alkyl group and the phenyl ring is unsubstituted or substituted with one or two of the same or different halo, nitro or ($C_1$-$C_4$)alkyl ;
B is unsubstituted naphthyl ; or
unsubstituted or substituted phenyl where the substituents can be from one to three of the same or different halo ; nitro ;
cyano ; ($C_1$-$C_4$)alkyl ; halo($C_1$-$C_4$)alkyl ; cyano($C_1$-$C_4$)alkyl ; ($C_1$-$C_4$)alkoxyl ;
alkoxyalkyl having independently 1 to 4 carbon atoms in each alkyl group ; $-COD^4$ group ; carboxy ; ($C_1$-$C_4$)alkoxy-carbonyl ;
($C_1$-$C_4$)alkanoyloxy ; $-ND^4D^5$ group ;
($C_1$-$C_4$)alkyl-thio ; $-CSD^4$ group ; $-CS_2D^4$ group ; $-SCOD^4$ group ; unsubstituted or substituted phenyl having one to two of the same or different halo, nitro, ($C_1$-$C_4$)alkyl, ($C_1$-$C_4$)alkoxy, carboxy, ($C_1$-$C_4$)alkoxy-carbonyl, ($C_1$-$C_4$)alkanoyloxy, $NH_2$, NHZ or NZZ' ; phenoxy where the phenyl ring is unsubstituted or substituted with one or two of the same or different halo, nitro, ($C_1$-$C_4$)alkyl, ($C_1$-$C_4$)alkoxy, carboxy, ($C_1$-$C_4$)-alkoxy-carbonyl, ($C_1$-$C_4$)alkanoyloxy, $NH_2$, NHZ or NZZ' ; or when two adjacent positions on the phenyl ring are substituted with alkoxy groups, these groups may be joined to form, together with the carbon atoms to which they are attached, a 5- or 6-membered dioxolano or dioxano heterocyclic ring ;
where $D^4$ and $D^5$ are hydrogen or ($C_1$-$C_4$)alkyl ; Z and Z' are ($C_1$-$C_4$)alkyl ; and agronomically acceptable salts.

Insecticidal compounds of the present invention having very good activity for use in the insecticidal compositions and formulations of the present invention include those where, any one or any combination of two or more of the substituents conforms to the following definitions :

X and X' are O or S ;
$R^1$ is branched ($C_3$-$C_8$)alkyl ;
A is unsubstituted or substituted ($C_1$-$C_6$)alkyl having one to three of the same or different halo, cyano, nitro, carboxy or ($C_1$-$C_4$)alkoxy-carbonyl ;
cyclohexyl ;
unsubstituted or substituted ($C_2$-$C_6$)alkenyl having one to three of the same of different halo or ($C_1$-$C_4$)alkyl ;
cyclohexenyl ; or
benzyl where the phenyl ring is unsubstituted or substituted with one or two of the same or different halo, methyl or ethyl ;
B is unsubstituted naphthyl ; or
unsubstituted or substituted phenyl having one to three of the same or different halo ; nitro ; cyano ; ($C_1$-$C_4$)alkyl ; halo($C_1$-$C_4$)alkyl ; cyano($C_1$-$C_4$)alkyl ; ($C_1$-$C_4$)alkoxy ; $-COD^4$ group ; ($C_1$-$C_4$)alkoxy-carbonyl ; ($C_1$-$C_4$)alkanoyloxy ; or unsubstituted or substituted phenyl having one or two of the same or different halo,

nitro, $(C_1-C_4)$alkyl, $(C_1-C_4)$alkoxy, carboxy, $(C_1-C_4)$alkoxy-carbonyl, $(C_1-C_4)$alkanoyloxy, $NH_2$, NHZ or NZZ'; where $D^4$ is hydrogen or $(C_1-C_4)$alkyl ; Z and Z' are $(C_1-C_4)$alkyl ;
and
agronomically acceptable salts thereof.

Because of their excellent insecticidal activity, most preferred compounds of the present invention for use in the insecticidal compositions and formulations of the present invention include those where any one or combination of two or more of the substituents conform to the following definitions :

X and X' are O ;
$R^1$ is branched $(C_4-C_7)$alkyl ;
A is $(C_1-C_6)$ unsubstituted or substituted alkyl having one or two of the same or different halo ;
cyclohexyl ;
$(C_2-C_6)$ unsubstituted or substituted alkenyl having one to three of the same or different halo, methyl or ethyl ;
cyclohexenyl ; or
benzyl ;
B is unsubstituted or substituted phenyl where the substituents can be from one to three of the same or different halo, $(C_1-C_4)$alkyl, $(C_1-C_4)$alkoxy or $(C_1-C_4)$haloalkyl ; and
agronomically acceptable salts thereof.

Because of their outstanding insecticidal activity, most particularly preferred compounds of the present invention for use in the insecticidal compositions and formulations of the present invention include those where any one or combination of two or more of the substituents conform to the following definitions :

X and X' are O ;
$R^1$ is t-butyl, neopentyl (2,2-dimethylpropyl) or 1,2,2-trimethylpropyl ;
A is $(C_3-C_5)$ unsubstituted alkyl ;
cyclohexyl ;
$(C_2-C_4)$alkenyl unsubstituted or substituted with one to three of the same or different chloro, bromo or methyl ;
cyclohexenyl ; or
benzyl ;
B is unsubstituted or substituted phenyl where the substituents can be one or two of the same or different chloro, fluoro, bromo, iodo, methyl, ethyl, methoxy or trifluoromethyl ; and
agronomically acceptable salts thereof.

Those N'-substituted-N-alkylcarbonyl-N'-acyl hydrazines of the invention which possess acidic or basic functional groups may be further reacted to form novel salts with appropriate bases or acids. These salts also exhibit insecticidal activity. Typical salts are the agronomically acceptable metal salts, ammonium salts and acid addition salts. Among the metal salts are those in which the metal cation is an alkali metal cation such as sodium, potassium or lithium ; alkaline earth metal cation such as calcium, magnesium, barium or strontium ; or heavy metal cation such as zinc, manganese, cupric, cuprous, ferric, ferrous, titanium or aluminum. The ammonium salts include those in which the ammonium cation has the formula $NR^5R^6R^7R^8$ wherein each of $R^5$, $R^6$, $R^7$ and $R^8$ is independently hydrogen, hydroxy, $(C_1-C_4)$alkoxy, $(C_1-C_{20})$alkyl, $(C_3-C_8)$alkenyl, $(C_3-C_8)$alkynyl, $(C_2-C_8)$hydroxyalkyl, $(C_2-C_8)$alkoxyalkyl, $(C_2-C_6)$aminoalkyl, $(C_2-C_6)$haloalkyl, $NH_2$, NHZ or NZZ' ; substituted or unsubstituted phenyl, substituted or unsubstituted phenylalkyl, having up to four carbon atoms in the alkyl moiety, or any two of $R^5$, $R^6$, $R^7$ or $R^8$ can be taken to form together with the nitrogen atom, a 5- or 6-membered heterocyclic ring, optionally having up to one additional hetero atom (e.g., oxygen, nitrogen, or sulfur) in the ring, and preferably saturated, such as piperidino, morpholino, pyrrolidino, piperazino or the like, or any three of $R^5$, $R^6$, $R^7$ or $R^8$ can be taken to form together with the nitrogen atom, a 5- or 6-membered aromatic heterocyclic ring, such as piperazole or pyridine. When $R^5$, $R^6$, $R^7$ or $R^8$ substituent in the ammonium group is as substituted phenyl or substituted phenylalkyl, the substituents on the phenyl and phenalkyl will generally be selected from halo, $(C_1-C_8)$alkyl, $(C_1-C_4)$alkoxy, hydroxy, nitro, trifluoromethyl, cyano, amino and $(C_1-C_4)$alkylthio. Such substituted phenyl groups preferably have up to two such substituents. Representative ammonium cations include ammonium, dimethylammonium, 2-ethylhexyl-ammonium, bis(2-hydroxyethyl)ammonium, tris(2-hydroxyethyl)ammonium, dicyclohexylammonium, t-octylammonium, 2-hydroxyethylammonium, morpholinium, piperidinium, 2-phenethylammonium, 2-methylbenzylammonium, n-hexylammonium, triethylammonium, trimethylammonium, tri(n-butyl)-ammonium, methoxyethylammonium, diisopropylammonium, pyridinium, dialkylammonium, pyrazolium, propargylammonium, dimethylhydrazinium, octadecylammonium, 4-dichlorophenylammonium, 4-nitro-benzylammonium, benzyltrimethylammonium, 2-hydroxy-ethyldimethyloctadecylammonium, 2-hydroxyethyldiethyl-octylammonium, decyltrimethylammonium, hexyltriethylammonium, 4-methylbenzyltrimethylammonium, and the like. Among the acid addition salts are those in which

the anion is an agronomically acceptable anion such as hydrochloride, hydrobromide, sulfate, nitrate, perchlorate, acetate and oxalate.

The compounds of this invention or their precursors can be prepared according to the following processes. Process A can be used when preparing compounds according to Formula I where X and X′ are both oxygen.

Process A:

Step 1

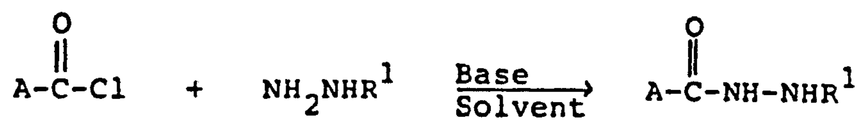


II III IV

Step 2

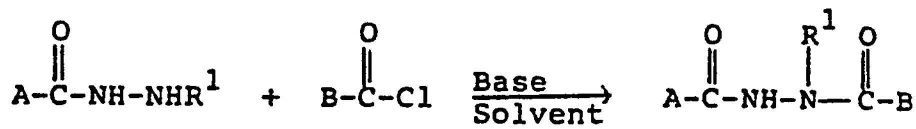


IV V I

where $R^1$, A and B are as defined above for Formula I and X and X′ are oxygen.

Alternatively, process B can be used when preparing compounds according to Formula I where X and X′ are oxygen, and $R^1$, A and B are as defined above for Formula I.

Process B :

Step 1

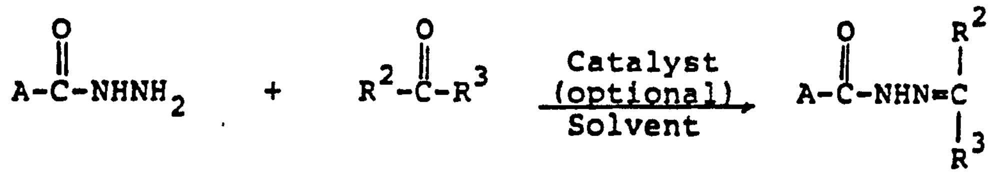


VI VII VIII

Step 2

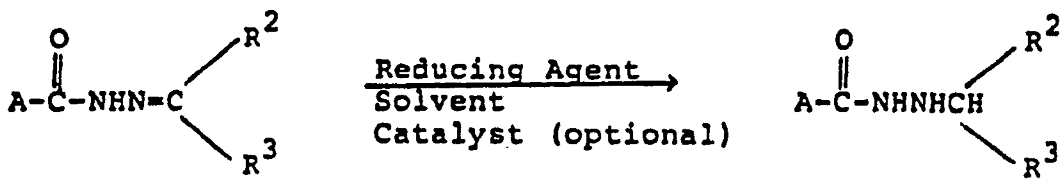


VIII IX (IV)

Step 3

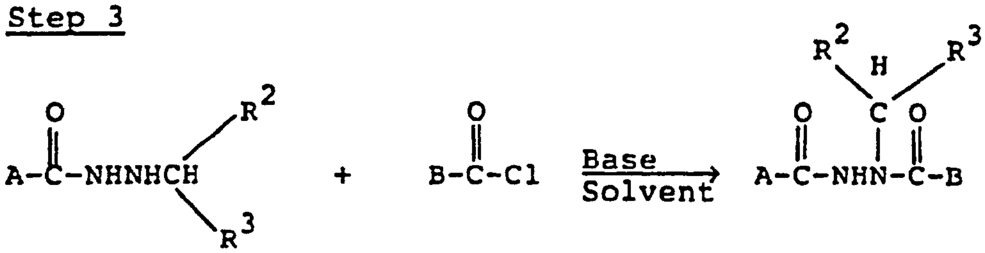


IX (IV) V X (I)

where X and X′ are oxygen, A and B are as defined above for Formula I, and $R^2$ and $R^3$ are the same or different hydrogen or ($C_2$-$C_9$) straight or branched chain alkyl provided that $R^2$ and $R^3$ are not both H or $R^2$ or $R^3$ is not a straight chain alkyl group when the other ($R^2$ or $R^3$) is hydrogen. As can be seen above, the intermediate product of Step 2, the compounds of Formula IX, corresponds to the compounds of Formula IV. In addition, the compound of Formula X corresponds to the compounds of Formula I where X and X′ are oxygen.

Process C can be used when preparing compounds according to Formula I where A, B and $R^1$ are as defined for Formula I and at least one of X and X′ is sulfur.

Process C :

Step 1

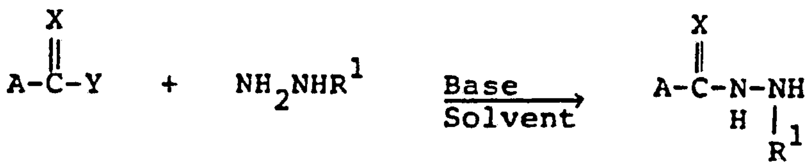


XI III XII

Step 2

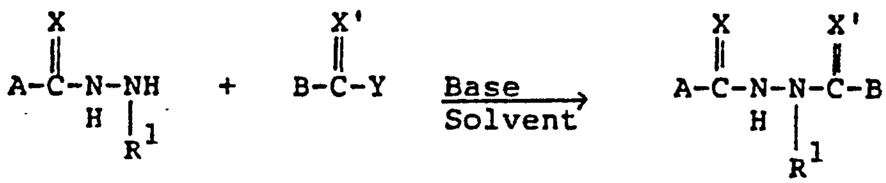


XII XIII I

wherein A, B and $R^1$ are as defined above for Formula I and at least one of X and X′ is sulfur, and Y is a good leaving group such as carboxyalkylthio (for example, carboxymethylthio $-SCH_2CO_2H$) ; alkylthio (for example, methylthio) ; or halo (for example, chloro).

Process D can be used when preparing compounds according to Formula I where X and X′ are oxygen and $R^1$, A and B are as defined above for Formula I.

Process D

Step 1

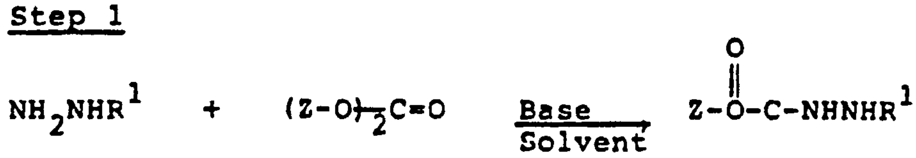


III XIV XV

Step 2

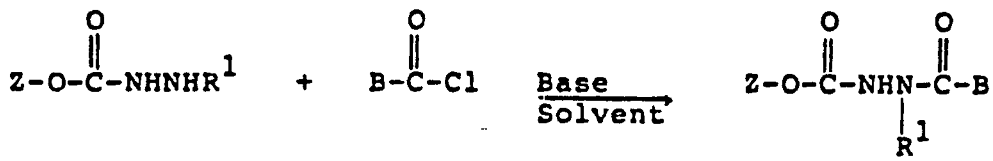


XV V XVI

Step 3

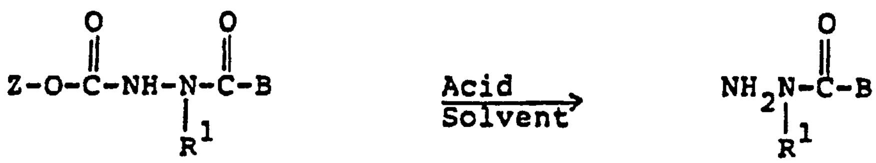


XVI XVII

Step 4

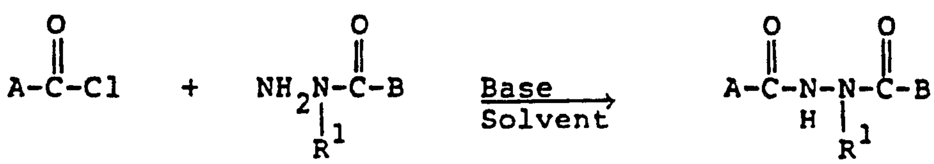


II XVII I

wherein A, B and $R^1$ are as defined above for Formula I and Z is t-butyl ; ethyl ; phenyl ; or benzyl.

Process E can be used when preparing compounds according to Formula I where X and X' are oxygen and $R^1$, A and B are as defined above for Formula I.

Process E :

Step 1

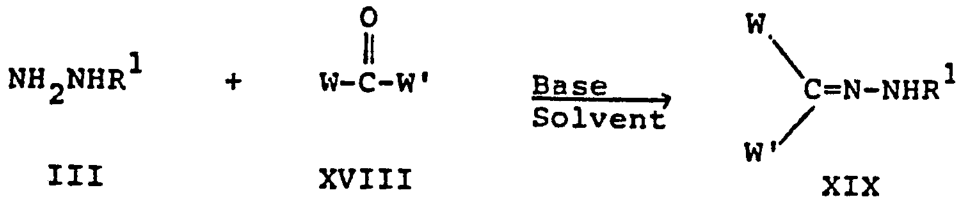


III XVIII XIX

Step 2

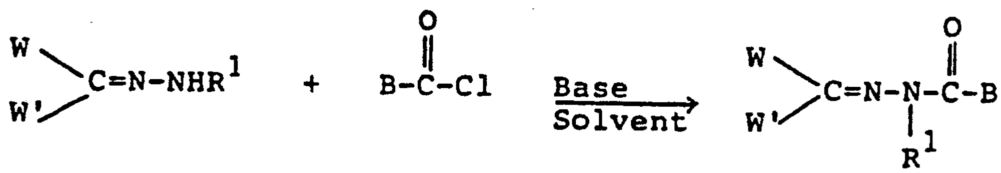


XIX V XX

Step 3

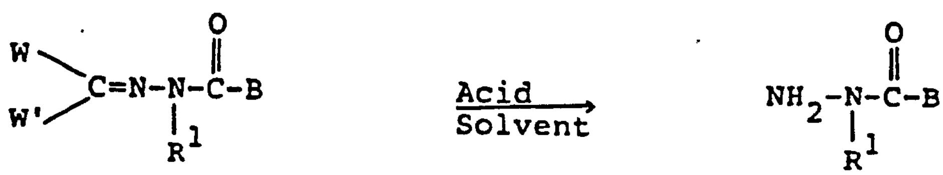


XX XVII

Step 4

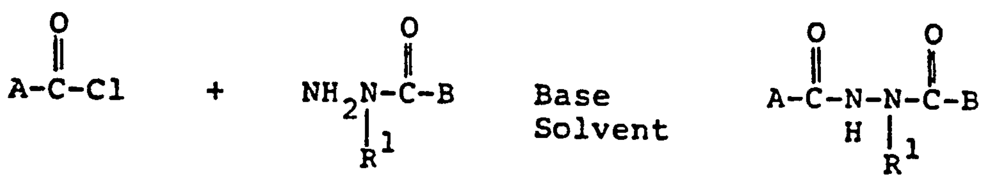


II XVII I

wherein $R^1$, A and B are as defined above for Formula I and W is hydrogen, methyl or ethyl ; and W' is methyl, ethyl, phenyl or isobutyl.

In process A, a compound of Formula II is reacted with a monosubstituted hydrazine of Formula III or a corresponding acid addition salt such as the hydrochloride salt or the like, in the presence of a base in an inert or substantially inert solvent or mixture of solvents to afford an intermediate product of Formula IV which can be isolated or further reacted with a compound of Formula V in the presence of a base in an inert or substantially inert solvent or mixture of solvents to afford the desired product of Formula I.

Examples of the compounds of Formula II which can be used in the above processes include cyclohexylcarbonyl chloride, n-butanoyl chloride, n-pentanoyl chloride, phenylacetyl chloride, 1-cyclohexenecarbonyl chloride, pivaloyl chloride, trichloroacetyl chloride, methacryloyl chloride and the like.

Examples of the compounds of Formula V which can be used in the above processes include benzoyl chloride, 4-chlorobenzoyl chloride, 4-methylbenzoyl chloride, 3,5-dichlorobenzoyl chloride, 2-bromobenzoyl chloride, 3-cyanobenzoyl chloride and the like.

The compounds of Formula II and/or Formula V are generally commercially available or can be prepared by known procedures.

Examples of the compounds of Formula III which can be used in the above processes include isopropylhydrazine, t-butylhydrazine, neopentyl-hydrazine, alpha-methylneopentylhydrazine, isobutyl-hydrazine, isopentylhydrazine, isooctylhydrazine, and the like. The compounds of Formula III are generally commercially available or can be prepared by known procedures.

Suitable solvents for use in the above processes include water ; alcohols such as methanol, ethanol, isopropanol and the like ; hydrocarbons such as toluene, xylene, hexane, heptane and the like ; glyme ; tetrahydrofuran ; acetonitrile, pyridine ; or haloalkanes such as methylene chloride or mixtures of these solvents.

Preferred solvents are water, toluene, methylene chloride or a mixture of these solvents.

Examples of bases for use in the above processes include tertiary amines such as triethylamine ; pyridine; potassium carbonate ; sodium carbonate ; sodium bicarbonate ; sodium hydroxide ; or potassium hydroxide. Preferred bases are sodium hydroxide, potassium hydroxide or triethylamine.

In Process B, a compound of Formula VI is reacted with a ketone or aldehyde of Formula VII in an inert or substantially inert solvent or mixture of solvents and optionally in the presence of a catalyst to afford an intermediate product of Formula VIII. The intermediate product of Formula VIII is then further reacted with a reducing agent in an inert or substantially inert solvent or mixture of solvents to afford a second intermediate product of Formula IX (IV) which can be isolated or further reacted with a compound of Formula V in the presence of a base in an inert or substantially inert solvent or mixture of solvents to afford the desired product of Formula X (I).

Examples of the compounds of Formula VI which can be used in the above Process B include cyclohexane carboxylic acid hydrazide, n-butanoic acid hydrazide, n-pentanoic acid hydrazide, phenylacethydrazide, 1-cyclohexene carboxylic acid hydrazide, pivalic acid hydrazide, trichloroacethydrazide, methacrylic acid hydrazide and the like. The compounds of Formula VI are generally commercially available or can be prepared by known procedures.

Examples of the compounds of Formula VII which can be used in the above Process B include 1,1,1-trimethylacetaldehyde, methylethylketone, diethylketone and the like. The compounds of Formula VII are generally commercially available or can be prepared by known procedures.

Optionally, a catalyst may be used in Step 1 of Process B. Suitable catalysts generally include organic acids such as acetic acid, trifluoroacetic acid, oxalic acid and the like ; mineral acids such as hydrochloric acid, sulfuric acid, nitric acid and the like ;arylsulfonic acids such as toluenesulfonic acid ; or pyridinium toluenesulfonate. Preferred catalysts are organic acids or arylsulfonic acids. Most preferred catalyst are acetic acid or trifluoroacetic acid.

Suitable solvents for use in the above Process B, Step 1, include alcohols such as methanol, ethanol, isopropanol and the like ; hydrocarbons such as toluene, benzene ; ethers such as tetrahydrofuran (THF), glyme and the like ; or dimethylformamide. Preferred solvents are alcohols and hydrocarbons. Most preferred solvents are alcohols such as methanol or ethanol.

Examples of suitable reducing agents for use in the above Process B, Step 2, include hydrides such as sodium borohydride and derivatives thereof such as sodium cyanoborohydride, lithium aluminum hydride and derivatives thereof and the like ; or diborane. Preferred reducing agents are sodium borohydride and derivatives thereof or lithium aluminum hydride and derivatives thereof. Most preferred as a reducing agent is sodium cyanoborohydride.

Optionally, in Process B, Step 2, a catalyst may be included. Examples of suitable catalysts include organic acids such as acetic acid, trifluoroacetic acid ; or mineral acids such as hydrochloric acid, sulfuric acid and the like. Preferred catalysts are organic acids or hydrochloric acid. Most preferred catalysts are acetic acid, trifluoroacetic acid or hydrochloric acid.

Suitable solvents for use in the above Process B, Step 2, include alcohols such as methanol, ethanol, isopropanol and the like ; ethers such as tetrahydrofuran (THF), diethylether, glyme and the like ; or halohydrocarbons such as methylene chloride, chloroform and the like. Preferred solvents are alcohols and most preferred are methanol or ethanol.

Step 3 of Process B corresponds to Step 2 of Process A. Consequently, those bases and solvents suitable for use in Step 2 of Process A are suitable for use in Step 3 of Process B including the preferred bases and solvents described above.

In Process C, a compound of Formula XI is reacted with a monosubstituted hydrazine of Formula III or a corresponding acid addition salt such as the hydrochloride salt or the like in the presence of a base in an inert or substantially inert solvent or mixture of solvents to afford an intermediate compound of Formula XII which can be isolated or further reacted with a compound of Formula XIII in the presence of a base in an inert or substantially inert solvent or mixture of solvents to afford the desired product of Formula I.

Examples of the compounds of Formula XI which can be used in the above Process C include methylthiothiobutyrate, methylthio-thiopentanoate, methylthio-thiocyclopentanecarboxylate, methylthio-alpha-

phenylthioacetate and the like.

Examples of the compounds of Formula XIII which can be used in the above Process C include 3-methyl-methylthio-thiobenzoate, 4-chloromethylthio-thiobenzoate, 4-methyl-methylthio-thiobenzoate, carboxymethylthio-thiobenzoate and the like.

The compounds of Formula XI and/or Formula XII are generally commercially available or can be prepared by known procedures.

Suitable solvents for use in the above Process C are generally polar high-boiling solvents such as dimethylformamide (DMF) ; glyme ; tetrahydrofuran (THF) ; and pyridine. The preferred solvent is pyridine.

Suitable bases for use in the above Process C include tertiary amines such as triethylamine ; and pyridine. The preferred base is pyridine.

In Process D, a monosubstituted hydrazine of Formula III or a corresponding acid addition salt, such as the hydrochloride salt or the like, is reacted with a compound of Formula XIV in the presence of a base in an inert or substantially inert solvent or mixture of solvents to afford an intermediate product of Formula XV. The intermediate product of Formula XV is then further reacted with a compound of Formula V in the presence of a base in an inert or substantially inert solvent or mixture of solvents to afford a second intermediate product of Formula XVI. The second intermediate product of Formula XVI is then further reacted with an acid in an inert or substantially inert solvent or mixture of solvents to afford a third intermediate product of Formula XVII. The third intermediate product of Formula XVII is then further reacted with a compound of Formula II in the presence of a base in an inert or substantially inert solvent or mixture of solvents to afford the desired product of Formula I.

Examples of the compounds of Formula XIV which can be used in the above Process D include di-t-butyl-carbonate, diethylcarbonate, diphenylcarbonate, dibenzylcarbonate and the like. The compounds of Formula XIV are generally commercially available or can be prepared by known procedures.

Suitable solvents for use in the above Process D, Steps 1, 2 and 4 include water ; tetrahydrofuran ; dioxane; toluene ; alcohols such as methanol, ethanol and isopropanol ; hexane ; acetonitrile ; pyridine ; and haloalkanes such as methylene chloride ; or mixtures of these solvents.

Preferred solvents are dioxane ; toluene ; tetrahydrofuran ; pyridine ; methylene chloride or water.

Most preferred solvents are dioxane ; water or toluene.

Examples of the bases for use in the above Process D Steps 1, 2 and 4 include tertiary amines such as triethylamine ; pyridine ; potassium carbonate, sodium carbonate ; sodium bicarbonate ; sodium hydroxide ; and potassium hydroxide.

Preferred bases are sodium hydroxide ; potassium hydroxide ; pyridine or triethylamine.

Suitable solvents for use in the above Process D, Step 3 include alcohols such as methanol, ethanol and isopropanol ; water ; tetrahydrofuran ; dioxane ; and acetonitrile.

Preferred solvents are methanol or ethanol.

Examples of acids for use in the above Process D, Step 3 include concentrated hydrochloric acid or concentrated sulfuric acid.

In Process E, a monosubstituted hydrazone of Formula III or a corresponding acid addition salt such as the hydrochloride salt or the like is reacted with a ketone or aldehyde of Formula XVIII in the presence of a base in an inert or substantially inert solvent or mixture of solvents to afford an intermediate product of Formula XIX. The intermediate product of Formula XIX is then further reacted with a compound of Formula V in the presence of a base in an inert or substantially inert solvent or mixture of solvents to afford a second intermediate product of Formula XX. The second intermediate product of Formula XX is then further reacted with an acid in the presence of an inert or substantially inert solvent or mixture of solvents to afford a third intermediate product of Formula XVII. The third intermediate product of Formula XVII is then further reacted with a compound of Formula II in the presence of a base in an inert or substantially inert solvent or mixture of solvents to afford the desired product of Formula I.

Examples of the compounds of Formula XVIII which can be used in the above Process E include acetone, benzaldehyde, methyl ethyl ketone, acetophenone and isobutyraldehyde.

Preferably, acetone is used. The compounds of Formula XVIII are generally commercially available or can be prepared by known procedures.

Suitable solvents for use in the above Process E, Steps 1, 2 and 4 include alcohols such as methanol, ethanol, isopropanol and n-propanol ; methylene chloride ; toluene ; tetrahydrofuran ; acetontrile ; water ; ether ; and pyridine.

Preferred solvents are alcohols such as methanol or ethanol ; toluene ; methylene chloride ; water ; tetrahydrofuran ; or pyridine.

Most preferred solvent are methanol, ethanol, toluene, water or pyridine.

Examples of bases for use in the above Process E, Steps 1, 2 and 4 include triethylamine, potassium hydroxide, sodium hydroxide, potassium carbonate and pyridine.

Preferred bases are sodium hydroxide or pyridine.

Suitable solvents for use in the above Process E, Step 3 include alcohols such as methanol, ethanol, isopropanol and n-propanol ; tetrahydrofuran ; toluene ; acetonitrile ; water ; and dioxane.

Preferred solvents are methanol, ethanol, tetrahydrofuran or water.

Examples of acids for use in the above Process E, Step 3 include dilute hydrochloric acid or dilute sulfuric acid.

The above processes A and B can be carried out at temperatures between about – 20°C and about 100°C. Preferably, these reactions are carried out between about – 5°C and about 50°C.

Process C can be carried out at temperatures between about 10°C and 200°C. Preferably, this reaction is carried out between about 70°C and about 100°C.

Preparation of the compounds of the present invention by processes A, B and C is preferably carried out at about atmospheric pressure although higher or lower pressures can be used if desired.

Substantially equimolar amounts of reactants are preferably used in processes A, B and C although higher or lower amounts can be used if desired.

Generally, about one equivalent of base is used per equivalent of starting material of Formula II, V, XI and/or XIII. Where the acid addition salt of the monosubstituted hydrazine of Formula III is used, one additional equivalent of base is used. For example, in Process A, when substituents A and B are the same and a monosubstituted hydrazine is used, about two equivalents of base are used since about two equivalents of a suitably substituted benzoyl chloride of Formula II or V are employed. In Process A, when substituents A and B are different and an acid addition salt of the monosubstituted hydrazine of Formula III is used, about two equivalents of base are used in step 1 and about one equivalent of base is used in step 2.

The above Processes D and E can be carried out at temperatures between about 0°C and about 100°C. Preferably, these reactions are carried out between about 0°C and about 50°C.

Preparation of the compounds of the present invention by Processes D and E is preferably carried out at about atmospheric pressure although higher or lower pressures can be used if desired.

Substantially equimolar amounts of reactants are preferably used in Processes D and E although higher or amounts can be used if desired.

Generally, about one equivalent of base is used per equivalent of starting material or intermediate product in each of Steps 1, 2 and 4 of Processes D and E. Where the acid addition salt of the monosubstituted hydrazine of Formula III is used, one additional equivalent of base is used.

Modifications to the above processes may be necessary to accommodate reactive functionalities of particular A and/or B substituents. Such modifications would be apparent and known to those skilled in the art.

The agronomically acceptable salts embraced by Formula I of the invention can be prepared by reacting a metal hydroxide, a metal hydride or an amine or ammonium salt, such as a halide, hydroxide or alkoxide with a compound of Formula I having one or more hydroxy or carboxy groups or reacting a quaternary ammonium salt, such as chloride, bromide, nitrate or the like with a metal salt of a compound of Formula I in a suitable solvent. When metal hydroxides are used as reagents, useful solvents include water ; ethers such as glyme and the like ; dioxane ; tetrahydrofuran ; alcohols such as methanol, ethanol, isopropanol and the like. When metal hydrides are used as reagents, useful solvents include nonhydroxylic solvents, for example, ethers such as dioxane, glyme, diethylether and the like ; tetrahydrofuran ; hydrocarbons such as toluene, xylene, hexane, pentane, heptane, octane ; dimethylformamide, and the like. When amines are used as reagents, useful solvents include alcohols, such as methanol or ethanol ; hydrocarbons, such as toluene, xylene, hexane and the like ; tetrahydrofuran ; glyme ; dioxane ; or water. When ammonium salts are used as reagents, useful solvents include water ; alcohols, such as methanol or ethanol ; glyme ; tetrahydrofuran ; or the like. When the ammonium salt is other than a hydroxide or alkoxide, an additional base, such as potassium or sodium hydroxide, hydride, or alkoxide is generally used. The particular choice of solvent will depend on the relative solubilities of the starting materials and the resultant salts, and slurries rather than solutions of certain reagents may be used to obtain the salts. Generally, equivalent amounts of the starting reagents are used and the salt-forming reaction is carried out at about 0°C to about 100°C, preferably at about room temperature.

The acid addition salts of the present invention can be prepared by reacting hydrochloric, hydrobromic, sulfuric, nitric, phosphoric, acetic, propionic, benzoic or other suitable acid with a compound of Formula I having a basic functional group in a suitable solvent. Useful solvents include water, alcohols, ethers, esters, ketones, haloalkanes and the like. The particular choice of solvent will depend on the relative solubilities of the starting materials and the resulting salts and slurries rather than solutions of certain reagents may be used to obtain the salts. Generally, equivalent molar amounts of starting materials are used and the salt-forming reaction is carried out at from about – 10°C to about 100°C, preferably at about room temperature. The following Examples and the individual substituents mentioned therein and each and every combination of, or group containing, two

or more of these substituents will further illustrate this invention but are not intended to limit it in any way. In table I, some N'-substituted-N-alkylcarbonyl-N'-acyl-hydrazines of the present invention that have been made are listed. Structures were confirmed by NMR and in some cases by IR and/or elemental analysis. Specific illustrative preparation of the compounds of Examples 1, 3, 9, 11, 14 and 15 are described after Table I.

## TABLE I

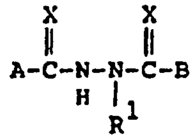

| Ex. No. | X | X' | $R^1$ | A | B | m.p. °C |
|---|---|---|---|---|---|---|
| 1 | O | O | $-C(CH_3)_3$ | 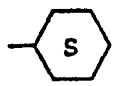 | $-C_6H_5$ | >250 |
| 2 | O | O | $-C(CH_3)_3$ | $-CH_2CH_2CH_3$ | $-C_6H_4Cl-4$ | 177-179 |
| 3 | O | O | $-C(CH_3)_3$ | $-CH_2CH_2CH_2CH_3$ | $-C_6H_5$ | 87-90 |
| 4 | O | O | $-C(CH_3)_3$ | $-CH_2CH_2CH_2CH_3$ | $-C_6H_4Cl-4$ | 179-180 |
| 5 | O | O | $-C(CH_3)_3$ | $-CH_2CH_2CH_2CH_3$ | $-C_6H_4CH_3-4$ | 127-128 |
| 6 | O | O | $-C(CH_3)_3$ | $-CH_2CH_2CH_3$ | $-C_6H_5$ | 114-115 |
| 7 | O | O | $-C(CH_3)_3$ | $-CH_2CH_2CH_2CH_3$ | $-C_6H_4F-4$ | 143-144 |
| 8 | O | O | $-C(CH_3)_3$ | $-CH_2CH_2CH_2CH_3$ | $-C_6H_4CF_3-4$ | 195-196 |
| 9 | O | O | $-C(CH_3)_3$ | $-CH_2C_6H_5$ | $-C_6H_5$ | 150-152 |
| 10 | O | O | $-C(CH_3)_3$ | $-C(CH_3)_3$ | $-C_6H_4Cl-4$ | 195-196 |
| 11 | O | O | $-C(CH_3)_3$ | 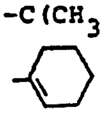 | $-C_6H_5$ | 185-187 |
| 12 | O | O | $-C(CH_3)_3$ | $-CH_2Cl$ | $-C_6H_5$ | 172-173 |
| 13 | O | O | $-C(CH_3)_3$ | $-CHCl_2$ | $-C_6H_5$ | 192-193 |
| 14 | O | O | $-C(CH_3)_3$ | $-CCl_3$ | $-C_6H_5$ | 139-141 |
| 15 | O | O | $-C(CH_3)_3$ | $-C(CH_3)_2CH_2Cl$ | $-C_6H_5$ | 233-234 |
| 16 | O | O | $-C(CH_3)_3$ | $-C(Cl)=CCl_2$ | $-C_6H_5$ | 184-186 |
| 17 | O | O | $-C(CH_3)_3$ | $-CH_2CH_2CH_2CH_2CH_3$ | $-C_6H_3Cl_2-3,4$ | 157-158 |
| 18 | O | O | $-C(CH_3)_3$ | $-CH_2CH_2CH_2CH_2CH_3$ | $-C_6H_4CH_3-3$ | 65-78 |
| 19 | O | O | $-C(CH_3)_3$ | $-C(CH_3)=CH_2$ | $-C_6H_5$ | 127-130 |
| 20 | O | O | $-C(CH_3)_3$ | 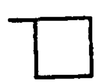 | $-C_6H_5$ | |

EXAMPLE NO. 1 - Preparation of N'-t-butyl-N-cyclohexyl-carbonyl-N'-benzoylhydrazine

To a stirred suspension of t-butylhydrazine hydrochloride (2.0 g, 0.016 M) in toluene (30 ml) was added 50% sodium hydroxide (1.3 g, 0.016 M). After 15 minutes, the mixture was cooled to 5°C and cyclohexanecarbonylchloride (2.4 g, 0.016 M) and 50% sodium hydroxide (1.3 g, 0.016 M) were added separately and simultaneously so as to maintain the reaction temperature below 10°C. After the addition, the reaction mixture was allowed to warm to room temperature and stirred for 1 hr. The mixture was diluted with hexane and the solid product isolated by filtration. This product (1.5 g, 0.008 M) was dissolved in a stirred mixture of toluene (30 ml) and cooled on ice. To this mixture was added benzoylchloride (1.1 g, 0.008 M) and 50% sodium hydroxide (0.6 g, 0.008 M) simultaneously. After addition, the mixture was stirred for 1/2 hr., diluted with hexane and the solid product, N'-t-butyl-N-cyclohexylcarbonyl-N'-benzoylhydrazine, isolated by filtration.

EXAMPLE NO. 3 - Preparation of N'-t-butyl-N-valeryl-N'-benzolhydrazine

To a stirred suspension of t-butylhydrazine hydrochloride (24.8 g, 0.20 mole) in toluene (150 ml) at 5°C was added one equivalent of NaOH, prepared by diluting 16 g of 50% aqueous (aq.) NaOH to 30 ml. After addition, valeryl chloride (24 g, 0.2 mole) and another one equivalent of NaOH solution (30 ml) were dropwise added separately and simultaneously. The reaction mixture was warmed to room temperature and stirred for 40 min. The two phase mixture was separated and the organic layer was washed with water and brine. The organic layer was dried over $Na_2SO_4$ and concentrated under vacuum to give a colorless oil.

To a stirred solution of 1-t-butyl-2-valeryl-hydrazine (4 g, 0.023 M) in toluene (40 ml) at 5°C was added benzoylchloride (3.4 g, 0.024 M) and 50% aq. sodium hydroxide (0.98 g, 0.024 M). After addition, the mixture was warmed to room temperature and stirred 2.5 hr. The mixture was diluted with ethyl acetate (50 ml) and washed with water (2 x 25 ml) and brine (1 x 25 ml). The organic layer was dried over magnesium sulfate and concentrated under vacuum to afford N'-t-butyl-N-valeryl-N'-benzoylhydrazine as a yellow oil.

EXAMPLE NO. 9 - Preparation of N'-t-butyl-N-phenyl-acetyl-N'-benzoylhydrazine

To a stirred suspension of t-butylhydrazine hydrochloride (2,0 g, 0.016 M) in toluene (30 ml) was added 50% sodium hydroxide (1.3 g, 0.016 M). After 15 minutes, the mixture was cooled to 5°C and phenylacetyl chloride (2.4 g, 0.016 M) and 50% sodium hydroxide (1.3 g, 0.016 M) were added separately and simultaneously so as to maintain the reaction temperature below 10°C. After the addition, the reaction mixture was allowed to warm to room temperature and stirred for 1 hr. The mixture was diluted with hexane and the solid product isolated by filtration. This product (3.2 g, 0.015 M) was dissolved in a stirred mixture of toluene (30 ml) and cooled on ice. To this mixture was added benzoylchloride (2.2 g, 0,016 M) and 50% sodium hydroxide (1.3 g, 0.016 M) simultaneously. After addition, the mixture was stirred for 1/2 hr., diluted with hexane and the solid product, N'-t- butyl-N-phenylacetyl-N'-benzoylhydrazine, isolated by filtration.

EXAMPLE NO. 11 - Preparation of N'-t-butyl-N-cyclohexenylcarbonyl-N'-benzoylhydrazine

To a stirred suspension of t-butylhydrazine hydrochloride (0.086 g, 0.007 M) in toluene (30 ml) was added 50% sodium hydroxide (0.55 g, 0.007 M). After 15 minutes, the mixture was cooled to 5°C and cyclohexenylcarbonyl chloride (1.0 g, 0.007 M) and 50% sodium hydroxide (0.55 g, 0.007 M) were added separately and simultaneously so as to maintain the reaction temperature below 10°C. After the addition, the reaction mixture was allowed to warm to room temperature and stirred for 1 hr. The mixture was diluted with hexane and the solid product isolated by filtration. This product (1.2 g, 0.006 M) was dissolved in a stirred mixture of toluene (30 ml) and cooled on ice. To this mixture was added benzoylchloride (1.0 g, 0.007 M) and 50% sodium hydroxide (1.3 g, 0.007 M) simultaneously. After addition, the mixture was stirred for 1/2 hr., diluted with hexane and the solid product, N'-t-butyl-N-cyclohexenyl-carbon 1-N'-benzoylhydrazine, isolated by filtration.

EXAMPLE NO. 14 - Preparation of N'-t-butyl-N-(beta-chloropivaloyl)-N'-benzoylhydrazine

To a stirred suspension of tert-butylhydrazine hydrochloride (200 g, 1.61 mole) in acetone (400 ml) was dropwise added triethylamine (200 g, 1.98 mole). After addition, the mixture was refluxed for 3 hours while periodically adding magnesium sulfate (100 g total). The mixture was cooled to room temperature and funnel filtered. The filtrate was dried over magnesium sulfate and concentrated under vacuum at 5°C until a slurry formed. The mixture was diluted with ethyl acetate (100 ml) and was filtered. The filtrate was distilled yielding a yellow oil (b.p. 121-127°C).

Into a stirred suspension of 1-t-butyl-2-acetone-hydrazone (43 g, 0.34 mole) in toluene (250 ml) at 5°C was added 10% aq. sodium hydroxide (200 ml, 0.50 mole). After addition, benzoylchloride (70 g, 0.50 mole) was added dropwise to this mixture. Stirring was continued for 2 hours at 5°C and 72 hours at room temperature. The mixture was diluted with ethyl acetate (300 ml) and washed with water (3 x 200 ml) and brine (200 ml). The organic layer was dried over magnesium sulfate and concentrated at 35°C under vacuum to afford a yellow oil.

A mixture of 1-t-butyl-1-benzoyl-2-acetone hydrazone (90 g, approximately 60% pure, approximately 0.3 mole), ethanol (500 ml, 200 proof) and 10% aq. hydrochloric acid (500 ml) was stirred overnight at room temperature. The mixture was concentrated under vacuum to a thick slurry. The slurry was suction filtered with a water wash (400 ml). The solids were air-dried overnight.

The solids were dissolved in a water (200 ml) and methanol (100 ml) solution on a steam bath. The mixture stood at room temperature overnight. The mixture was suction filtered and rinsed with cold water (100 ml) and the solids were air dried overnight. The solids were dissolved in 10% aq. hydrochloric acid (300 ml) and were washed with ethyl acetate (3 x 300 ml). The ethyl acetate washes were combined and extracted with 10% aq. hydrochloric acid (250 ml). The 10% aq. hydrochloric acid layers were combined and were neutralized with 50% aq. sodium hydroxide while being stirred. Stirring was continued for 1 hour at room temperature. The mixture was suction filtered and rinsed with water (100 ml) and the solids were air-dried yielding a white solid (m.p. 125-126°C).

To a stirred solution of 1-t-butyl-1-benzoyl-hydrazine (1 g, 0.005 M) in toluene (40 ml) cooled to 5°C was added 3-chloro-2,2-dimethylpropionylchloride (1.2 g, 0.007 M) and 50% aq. sodium hydroxide (0.45 g, 0.0056 M) so as to maintain the temperature below 10°C. The mixture was warmed to room temperature and stirred 1 hr. The mixture was diluted with hexane (40 ml) and water (10 ml). The white solid product N'-t-butyl-N-(beta-chloropivaloyl)-N'-benzoylhydrazine was isolated by suction filtration, washed with 50 ml hexane and 50 ml water and dried.

EXAMPLE NO. 15 - Preparation of N'-t-butyl-N-(1, 2, 2-trichlorovinyl)carbonyl-N'-benzoylhydrazine

To a stirred suspension of tert-butylhydrazine hydrochloride (200 g, 1.61 mole) in acetone (400 ml) was dropwise added triethylamine (200 g, 1.98 mole). After addition, the mixture was refluxed for 3 hours while periodically adding magnesium sulfate (100 g total). The mixture was cooled to room temperature and funnel filtered. The filtrate was dried over magnesium sulfate and concentrated under vacuum at 5°C until a slurry formed. The mixture was diluted with ethylacetate (100 ml) and was filtered. The filtrate was distilled yielding a lightly yellow tinted oil (b.p. 121-127°C).

To a stirred suspension of 1-t-butyl-2-acetone-hydrazone (43 g, 0.34 mole) in toluene (250 ml) at 5°C was added 10% aq. sodium hydroxide (200 ml, 0.50 mole). After addition, benzoylchloride (70g, 0.50 mole) was added dropwise to the mixture. Stirring was continued for 2 hours at 5°C and 72 hours at room temperature. The mixture was diluted with ethyl acetate (300 ml) and washed with water (3 x 200 ml) and brine (200 ml). The organic layer was dried over magnesium sulfate and concentrated at 35°C under vacuum yielding a yellow oil.

A mixture of 1-t-butyl-1-benzoyl-2-acetone hydrazone (90 g, approximately 60% pure, approximately 0.3 mole), ethanol (500 ml, 200 proof) and 10% aq. hydrochloric acid (500 ml) was stirred overnight at room temperature. The mixture was concentrated under vacuum to a thick slurry. The slurry was suction filtered and washed with water (400 ml). The solids were air-dried overnight.

To solids were dissolved in a water (200 ml) and methanol (100 ml) solution on a steam bath. The mixture stood at room temperature overnight. The mixture was suction filtered and rinsed with cold water (100 ml) and the solids were air dried overnight. The solids were dissolved in 10% aq. hydrochloric acid (300 ml) and were washed with ethyl acetate (3 x 300 ml). The ethyl acetate washes were combined and extracted with 10% aq. hydrochloric acid (250 ml). The 10% aq. hydrochloric acid layers were combined and were neutralized with 50% aq. sodium hydroxide while being stirred. Stirring was continued for 1 hour at room temperature. The mixture was suction filtered and rinsed with water (100 ml) and the solids were air-dried yielding a white solid (m.p. 125-126°).

To a stirred solution of 1-t-butyl-1-benzoyl-hydrazine (1.5 g, 0.008 M) in methylene chloride at 5°C was added, simultaneously and separately, trichloroacryloylchloride (1.7 g, 0.009 M) and triethylamine (0.9 g, 0.009 M). After addition, the reaction was warmed to room temperature and stirred for 3 h. The flask's contents were diluted with 50 ml methylene chloride and washed with water (3 x 75 ml) and brine solution (1 x 75 ml). The organic layer was dried over magnesium sulfate and the solvent removed under vacuum to afford N'-t-butyl-N-(1,2,2,-trichlorovinyl)carbonyl-N'-benzoylhydrazine as a white solid product.

By following substantially the procedures in processes A, B, C, D and E as exemplified above by the preparation of the compounds of Examples 1, 3, 9, 11, 14 and 15, the compounds of Formula I are prepared.

As previously noted, the compounds of the present invention exhibit excellent pesticidal activity and are selective against insects of the orders Lepidoptera and Coleoptera.

In general, for the control of insects in agriculture horticulture and forestry, a dosage corresponding to from about 10 grams to about 10 kilograms of the active substance per hectare may be used and from about 100 grams to about 5 kilograms per hectare of the active substance is preferred. The exact amount of dosage for a given situation can be routinely determined and depends on a variety of factors, for example, the substance used, the kind of pest, the formulation used, the state of the crop infested with the pest and the prevailing weather conditions. The term "insecticidal" as employed in the specification and claims of this application is to be construed as any means which adversely affects the existence or growth of the target insects. Such means can comprise a complete killing action, eradication, arresting in growth, inhibition, reducing in number or any combination thereof. The term "control" or "combat" as interchangeably employed in the specification and claims of this application is to be construed as meaning "insecticidal" or protecting plants from insect damage. By "insecticidally effective amount" is meant that dosage of active substance sufficient to exert insect "control".

The compounds of the present invention, for practical applications, can be utilized in the form of compositions or formulations. Examples of the preparation of compositions and formulations can be found in the American Chemical Society publication "Pesticidal Formulation Research", (1969), Advances in Chemistry Series No. 86, written by Wade Van Valkenburg ; and the Marcel Dekker, Inc. publication "Pesticide Formulations", (1973), edited by Wade Van Valkenburg. In these compositions and formulations, the active substance is mixed with inert agronomically acceptable (i.e., plant compatible and/or pesticidally inert) diluents or extenders such as solid carrier material or liquid carrier material, of the type usable in conventional compositions or formulations. By agronomically acceptable carrier is meant any substance which can be used to dissolve, disperse or diffuse the active ingredient in the composition without impairing the active ingredient's effectiveness and which by itself has no significant detrimental effect on the soil, equipment, desirable plants or agronomic environment. If desired, adjuvants such as surfactants, stabilizers, antifoam agents and antidrift agents may also be added.

Examples of compositions and formulations according to the invention are aqueous solutions and dispersions, oily solutions and oil dispersions, pastes, dusting powders, wettable powders, emulsifiable concentrates, flowables, granules, baits, invert emulsions, aerosol compositions and fumigating candles.

Wettable powders, pastes, flowables and emulsifiable concentrates are concentrated preparations which are diluted with water before or during use.

Baits are preparations generally comprising a food or other substance attractive to the target pest, that includes at least one lethal or non-lethal toxicant. Lethal toxicants kill the pest upon ingesting the bait while non-lethal toxicants change the behaviour, feeding habits and physiology of the pest for the purpose of control.

The invert emulsions are mainly used for aerial application, where large areas are treated with a comparatively small amount of preparation. The invert emulsion may be prepared in the spraying apparatus shortly before, or even during, the spraying operation by emulsifying water in an oil solution or an oil dispersion of the active substance.

Compositions and formulations are prepared in a known manner, for instance by extending the active compounds with conventional dispersible liquid diluent carriers and/or dispersible solid carriers optionally with the use of carrier vehicle assistants, e.g., conventional surface-active agents, including emulsifying agents and/or dispersing agents, whereby, for example, in the case where water is used as diluent, organic solvents may be added as auxiliary solvents. The following may be chiefly considered for use as conventional carrier vehicles for this purpose ; aerosol propellants which are gaseous at normal temperatures and pressures, such as halogenated hydrocarbons, e.g., dichlorodifluoromethane and trifluorochloromethane, as well as butane, propane, nitrogen and carbon dioxide ; inert dispersible liquid diluent carriers, including inert organic solvents, such as aromatic hydrocarbons (e.g., benzene, toluene, xylene, alkyl naphthalenes, etc.), halogenated, especially chlorinated, aromatic hydrocarbons (e.g., chlorobenzenes, etc.), cycloalkanes (e.g., cyclohexane, etc.), paraffins (e.g., petroleum or mineral oil fractions), chlorinated aliphatic hydrocarbons (e.g., methylene chloride, chloroethylenes, etc.), vegetable oils (e.g., soybean oil, cottonseed oil, corn oil, etc.), alcohols (e.g., methanol, ethanol, propanol, butanol, glycol, etc.) as well as ethers and esters thereof (e.g., glycol monomethyl ether, etc.), amines (e.g., ethanolamine, etc.), amides (e.g., dimethyl formamide, etc.), sulfoxides (e.g., dimethyl sulfoxide, etc.), acetonitrile, ketones (e.g., acetone, methyl ethyl ketone, methyl isobutyl ketone, cyclohexanone, isophorone, etc.), and/or water ; solid carriers including ground natural minerals, such as kaolins, clays, talcs, chalk, quartz, attapulgite, montmorillonite or diatomaceous earth, and ground synthetic minerals, such as highly-dispersed silicic acid, alumina and silicates ; solid carriers for granules include crushed and fractionated natural rocks such as calcite, marble, pumice, sepiolite and dolomite, as well as synthetic granules of inorganic and organic meals, and granules of organic material such as sawdust, coconut shells, corn cobs and tobacco

stalks. The following may be chiefly considered for use as conventional carrier vehicle assistants : emulsifying agents, such as cationic and/or nonionic and/or anionic emulsifying agents (e.g., polyethylene oxide esters of fatty acids, polethylene oxide ethers of fatty alcohols, alkyl sulfates, alkyl sulfonates, aryl sulfonates, albumin hydrolysates, etc., and especially alkyl arylpolyglycol ethers, magnesium stearate, sodium oleate, etc.) ; and/or dispersing agents, such as lignin, sulfite waste liquors, methyl cellulose, etc.

Adhesives such as carboxymethylcellulose and natural and synthetic polymers in the form of powders, granules or latices, such as gum arabic, polvinyl alcohol and polyvinyl acetate, can be used in the formulations.

If desired, it is possible to use colorants in compositions and formulations containing compounds of the present invention such as inorganic pigments, for example, iron oxide, titanium oxide and Prussian Blue, and organic dyestuffs, such as alizarin dyestuffs, azo dyestuffs and metal phthalocyanine dyestuffs, and trace nutrients such as salts of iron, manganese, boron, copper, cobalt, molybdenum and zinc.

The active compounds of the present invention may be employed alone or in the form of mixtures with one another and/or with such solid and/or liquid dispersible carrier vehicles and/or with other known compatible active agents, especially plant protection agents, such as other insecticides, arthropodicides, nematicides, fungicides, bactericides, rodenticides, herbicides, fertilizers, growth-regulating agents or synergists, if desired, or in the form of particular dosage preparations for specific application made therefrom, such as solutions, emulsions, suspensions, powders, pastes, and granules which are thus ready for use.

As concerns commercially marketed preparations, these generally contemplate carrier composition mixtures in which the active compound is present in an amount from 0.1% to 99% by weight, and preferably 1% to 75% by weight, of the mixture. Carrier composition mixtures suitable for direct application or field application generally contemplate those in which the active compound is used in an amount substantially between about 0.0001% and 5%, preferably between about 0.001% and 3%, by weight of the mixture. Thus the present invention contemplates overall formulations and compositions which comprise mixtures of a conventional dispersible carrier such as (1) a dispersible inert finely divided carrier solid, and/or (2) a dispersible carrier liquid such as an inert organic solvent and/or water, preferably including a surface-active effective amount of a carrier vehicle assistant (e.g., a surface-active agent, such as an emulsifying agent and/or a dispersing agent), and an amount of the active compound generally between about 0.0001% and about 99% by weight of the composition, preferably 0.001% to 90% by weight of the composition, and most preferably 0.01% to 75% by weight of the composition which is effective for the purpose in question.

The active compounds can be applied as sprays by methods commonly employed, such as conventional high-gallonage hydraulic sprays, low gallonage sprays, ultra-low-volume sprays, airblast spray, aerial sprays, and dusts. If low volume applications are desired, a solution of the compound is usually used. In ultra-low-volume applications, a liquid composition containing the active compound is usually applied as a spray (e.g., mist) by means of atomizing equipment in finely divided form (average particle size of from about 50 to about 100 microns or less) using airplane crop spraying techniques. Typically only a few liters per hectare are needed and often amounts up to about 15 to 1000 g/hectare, preferably about 40 to 600 g/hectare are sufficient. With ultra-low-volume, it is possible to use highly concentrated liquid compositions with said liquid carrier vehicles containing from about 20 to about 95% by weight of the active compound.

Furthermore, the present invention contemplates methods of selectively killing, combating or controlling pests, which comprises contacting insects with a corresponding combative or toxic amount (i.e., an insecticidally effective amount) of at least one active compound of the invention alone or together with a carrier vehicle (composition or formulation) as noted above. The term "contacting" as employed in the specification and claims of this application is to be construed as applying to at least one of (a) such insects and (b) the corresponding habitat thereof (i.e., the locus to be protected, for example, to a growing crop or an area where a crop is to be grown) the active compound of this invention alone or as a constituent of a composition or formulation. The instant formulations or compositions are applied in the usual manner, for instance by spraying, atomizing, vaporizing, scattering, dusting, watering, squirting, sprinkling, pouring, fumigating, dry dressing, moist dressing, wet dressing, slurry dressing, encrusting and the like.

It will be realized, of course, that the concentration of the particular active compound utilized in admixture with the carrier vehicle will depend upon such factors as the type of equipment employed, method of application, area to be treated, types of pests to be controlled and degree of infestation. Therefore, in special cases it is possible to go above or below the aforementioned concentration ranges.

Granular preparations are produced for example, by taking up the active substance in a solvent and by using the resulting solution, as the case may be in the presence of a binder, to impregnate a granular carrier material, such as porous granules (for example, pumice and attaclay), or chopped tobacco stems or the like.

A granular preparation (frequently termed a "pellet") may alternatively be produced by compressing the active substance together with powdered minerals in the presence of lubricants and binders and by disintegrating and straining the composite to the desired grain size.

Dusts are obtainable by intimately mixing the active substance with an inert solid carrier material in a concentration of from about 1 to about 50% by weight. Examples of suitable solid carrier materials are talc, kaolin, pipe clay, diatomaceous earth, dolomite, gypsum, chalk, bentonite, attapulgite and colloidal $SiO_2$ or mixtures of these and similar substances. Alternatively organic carrier materials such as, for example, ground walnut shells may be used.

Wettable powders and flowables are produced by mixing from about 10 to about 99 parts by weight of a solid inert carrier such, for example, as the aforementioned carrier materials with from about 1 to about 80 parts by weight of the active substance optionally dissolved in a volatile solvent such as acetone, from about 1 to about 5 parts by weight of a dispersing agent such, for example, as the lignosulfonates or alkylnaphthalene sulfonates known for this purpose and preferably also from about 0.5 to about 5 parts by weight of a wetting agent, such as fatty alcohol sulfates, or alkylarylsulfonates of fatty acid condensation products. In the case of flowables, a liquid inert carrier such as water is also included.

To produce emulsifiable concentrates the active compound is dissolved or finely divided in a suitable solvent which preferably is poorly miscible with water, an emulsifier being added to the resulting solution. Examples of suitable solvents are xylene, toluene, high-boiling aromatic petroleum distillates, for example solvent naphtha, distilled tar oil and mixtures of these liquids. Examples of suitable emulsifiers are alkylphenoxypolyglycol ethers, polyoxyethylene sorbitan esters of fatty acids or polyoxyethylene sorbitol esters of fatty acids. The concentration of the active compound in these emulsifiable concentrates is not restricted within narrow limits and may vary between about 2% and about 50% by weight depending upon toxicant solubility. A suitable liquid highly concentrated primary composition other than an emulsifiable concentrate is a solution of the active substance in a liquid which is readily miscible with water, for example, acetone, to which solution a dispersant and, as the case may be, a wetting agent are added. When such a primary composition is diluted with water shortly before or during the spraying operation an aqueous dispersion of the active substance is obtained.

An aerosol preparation according to the invention is obtained in the usual manner by incorporating the active substance or a solution thereof in a suitable solvent in a volatile liquid suitable for use as a propellant such, for example, as a mixture of chlorine and fluorine derivatives of methane and ethane.

Fumigating candles or fumigating powders, i.e., preparations which when burning are capable of emitting a pesticidal smoke, are obtained by taking up the active substance in a combustible mixture which may, for example, comprise a sugar or a wood, preferably in the ground form, as a fuel, a substance to sustain combustion such, for example, as ammonium nitrate or potassium chlorate, and furthermore a substance for retarding combustion, for example kaolin, bentonite and/or colloidal silicic acid.

A bait preparation comprises a food or other substance attractive to pests, a carrier, the toxicant and may optionally include other substances commonly used in preparations of this kind, such as, a preservative to inhibit bacterial and fungal growth, a waterproofing agent to prevent disintegration under wet conditions and dyes or colorants as described above.

In addition to the aforementioned ingredients, the preparations according to the invention may also contain other substances commonly used in preparations of this kind.

For example, a lubricant, such as calcium stearate or magnesium stearate, may be added to a wettable powder or to a mixture to be granulated. Furthermore, there may, for example, be added "adhesives" such as polyvinylalcohol cellulose derivatives or other colloidal materials, such as casein, to improve the adherence of this pesticide to the surface to be protected.

In its mechanical aspects therefore a process of the invention for improving the commercial value an.d/or profitability of vendible crops from plants whose growth is affected or likely to be affected by insects comprises (1) charging to a container, fumigation device or mechanical dissemination device an insecticidal composition of the invention as hereinbefore described (2) using the container, fumigator or mechanical dissemination device to apply the insecticidal composition, in the form of granules, dust, smoke, vapour or surfactant-containing liquid preparation to growing plants or to a growth medium where the plants are growing or are to be grown, or to the insects themselves, (3) controlling the dose of the active ingredient during this application step so that the rate of application of active insecticidal compound is sufficient to combat the insects but is insufficient to cause an unacceptably adverse effect on the crop plants growing or to be grown in the treated area.

Representative preparation of compositions and formulations including the compounds of the present invention are set forth below as Examples A through I by way of illustration but not limitation.

### Example A

### Granular

| Ingredient | %/wt. |
|---|---|
| Toxicant and toxicant impurities | 0.25 |
| Triton X-305 (binder) (Octylphenyl-30-ethylene oxide ethanol) | 0.25 |
| Agsorb 24/48 (diluent) (Montmorillonite clay) | 99.50 |

Preparation : The toxicant and Triton X-305 are dissolved into methylene chloride and the mixture is added to the Agsorb with continuous mixing. The methylene chloride is then allowed to evaporate.

### Example B

### Dust

| Ingredient | %/wt. |
|---|---|
| Toxicant and toxicant impurities | 1.0 |
| Talc | 99.0 |

Preparation : Toxicant is dissolved in excees acetone and the mixture is impregnated onto the talc. The acetone is then permitted to evaporate.

### Example C

### Wettable Powder

| Ingredient | %/wt. |
|---|---|
| Toxicant and toxicant impurities | 31.3 |
| Duponal WA Dry (wetter) (Sodium lauryl sulfate) | 2.0 |
| Reax 45A (dispersant) (Sodium lignin sulfonate) | 5.0 |
| Barden clay (diluent) | 31.7 |
| HiSil 233 (diluent) (Sodium silica) | 30.0 |

Preparation : The toxicant, optionally dissolved in a volatile solvent, is absorbed onto the Barden clay and HiSil carriers. The Duponal and Reax are then added and the entire dry mixture blended until homogenous. The composition is then micronized to a fine particle size.

### Example D

### Emulsifiable Concentrate

| Ingredient | %/wt. |
| --- | --- |
| Toxicant and toxicant impurities | 15.0 |
| Sponto 232T (emulsifier) (Anionic and nonionic blend of the following surfactants: calcium dodecyl benzene sulfonate; and ethoxylated alkylphenol) | 6.0 |
| Sponto 234T (emulsifier) (Anionic and nonionic blend of the following surfactants: calcium dodecyl benzene sulfonate; and ethoxylated alkylphenol) | 4.0 |
| Cyclohexanone (solvent) | 22.5 |
| Tenneco 500-100 (solvent) (Aromatic solvent mixture principally comprising xylene, cumene and ethyl benzene having a boiling point range of 290-345°F) | 52.5 |

Preparation : All ingredients are mixed together with continuous agitation until a homogenous clear solution is obtained.

### Example E

### Aerosol

| Ingredient | %/wt. |
| --- | --- |
| Toxicant and toxicant impurities | 0.5 |
| Freon 12 | 99.5 |

Preparation : The components are mixed and packaged under pressure in a suitable container equipped with a release spray valve.

### Example F

### Fumigating Candle or Fumigating Powder

| Ingredient | %/wt. |
| --- | --- |
| Toxicant and toxicant impurities | 1.0 |
| Wood dust | 96.0 |
| Starch | 3.0 |

Preparation : Toxicant, wood dust, and starch are blended together and then molded into a candle using a small amount of water to activate the starch.

Example G

Bait

Method A

| Ingredient | %/wt. |
|---|---|
| Toxicant and toxicant impurities | 1.00 |
| Wheat Bran (carrier and attractant) | 89.95 |
| Corn Syrup (attractant) | 7.00 |
| Corn Oil (attractant) | 2.00 |
| Kathon 4200 (preservative) (3-isothiazolone) | 0.05 |

Preparation : The corn oil and corn syrup are added to the wheat bran with adequate mixing. The toxicant and Kathon are premixed with excess acetone and this solution is added to the wheat bran base with continued mixing. The acetone is then permitted to evaporate.

Method B

| Ingredient | %/wt. |
|---|---|
| Toxicant and toxicant impurites | 0.06 |
| Granulated Sugar (carrier and attractant) | 99.94 |

Example H

Pellet

Same as Example G, Method A, with this addition : the bait composition is formed into 1/4" diameter by 3/8" long pellets using a suitable die and press apparatus.

Example I

Flowable

| Ingredient | %/wt. |
|---|---|
| Toxicant and toxicant impurities | 31.3 |
| Duponal WA Dry (wetter) (Sodium lauryl sulfate) | 2.0 |
| Reax 45A (dispersant) (Sodium lignin sulfonate) | 5.0 |
| HiSil 233 (diluent) (Sodium silica) | 30.0 |
| Kelzan (thickener) | 0.5 |
| (Xanthan gum) | 31.2 |

Preparation : The toxicant is absorbed onto the HiSil carrier. The Duponal and Reax are then added and the entire dry mixture blended until homogeneous. The composition is then micronized to a fine particle size.

The resulting powder is suspended in water and the Kelzan added.

Compositions and formulations according to the present invention may also include known pesticidal compounds. This expands the spectrum of activity of preparations and may give rise to synergism.

The following known insecticidal, fungicidal and acaricidal compounds are suitable for use in such a combined preparation.

Insecticides such as :

chlorinated hydrocarbons, for example, 2,2-bis(p-chlorophenyl)-1,1,1-trichloroethane and hexachloroepoxyoctahydrodimethanonnaphtalene ;

Carbamates, for example, N-methyl-1-naphthylcarbamates ;

Dinitrophenols, for example, 2-methyl-4,6-dinitrophenyl and 2-(2-butyl)-4,6-dinitrophenyl-3,3-dimethylacrylate ;

Organic phosphorus compounds, such as dimethyl-2-methoxy-3-carbonyl-1-methylvinyl phosphate, O,O-diethyl-O-p-nitrophenylphosphorothioate ; N-monomethylamide of O,O-dimethyldithio-phosphorylacetic acid ;

Diphenylsulfides, for example, p-chlorobenzyl or p-chlorophenyl sulfide and 2,4,4',5-tetrachlorodiphenylsulfide ;

Diphenylsulfonates, for example, p-chlorophenylbenzene-sulfonate ;

Methylcarbinols, for example, 4,4-dichloro-1-trichloro-methylbenzhydrol ;

Quinoxaline compounds, such as methylquinoxaline dithiocarbonate ;

Amidines such as N'-(4-chloro-2-methylphenyl) N,N-dimethylformamidine ;

Pyrethroids such as Allethrin ;

Biological such as Bacillus thuringiensis preparations ;

Organic tin compounds such as tricyclohexyltin hydroxide ;

Synergists such as piperonyl butoxide.

Fungicides such as :

Organic mercury compounds, for example, phenylmercuryacetate and methylmercurycyanoguanide ;

Organic tin compounds, for example, triphenyltin hydroxide and triphenyltin acetate ;

Alkylenebisdithiocarbamates, for example, zinc ethylene- bisthiocarbamate and manganese ethylenebisdithio-carbamate ; and

2,4-dinitro-6-(2-octyl-phenylcrotonate), 1-bis(dimethyl-amino)phosphoryl-3-phenyl-5-amino-1,2,4-triazole, 6-methylquinoxaline-2,3-dithiocarbonate, 1,4-dithio-anthraquinone-2,3-dicarbonitrile, N-trichloromethylthiophthalimide, N-trichloromethylthiotetrahydrophthalimide, N-(1,1,2,2-tetrachloroethylthio)-tetrahydrophthalimide, N-dichlorofluoromethylthio-N-phenyl-N'-dimethylsulfonyldiamide and tetrachloroisophthalonitrile.

It has been found by biological evaluation that compounds according to the present invention have pesticidal activity and are capable of controlling larvae and adult forms of pests, especially insects from the orders Lepidoptera and Coleoptera. One skilled in the art will know how to determine the activity of a given compound against a given insect and the dosage required.

As previously noted, the compounds of the present invention are particularly suitable for controlling plant-destructive insects in crops of cultivated plants, such as, but not limited to, cotton, vegetables, corn and other cereals, and the like ; forestry, such as, but not limited to, birch, spruce, pine, fir, and the like ; and ornamental plants, flowers and trees. Compounds of the present invention are also particularly suitable for controlling insects destructive to stored commodities such as seeds and the like ; fruit crops, such as, but not limited to, fruit and/or citrus trees, raspberry bushes, and the like ; and turf, such as, but not limited to, lawns, sod and the like.

In evaluating the pesticidal activity of the compounds of this invention, the following test procedures were employed.

A test solution containing 600 parts per million (ppm) was made by dissolving the test compound in a solvent (acetone : methanol, 1 : 1), adding water to give an acetone : methanol : water system of 5 : 5 : 90 and then a surfactant. A 1 : 1 mixture of an alkylarylpolyetheralcohol (sold under the trademark Triton X-155) and a modified phthalic glycerol alkyl resin (sold under the trademark Triton B-1956) was utilized at the equivalent of 1 ounce per 100 gal. of test solution as a surfactant.

Initial evaluations were made on one or more of the following pests :

| Code Symbol | Common Name | Latin Name |
|---|---|---|
| SAW | Southern Armyworm | Spodoptera eridania |
| MBB | Mexican Bean Beetle | Epilachna varivestis |

For the foliar bean beetle and armyworm tests, individual bean (Phaseolus limensis var. Woods' Prolific) leaves are placed on moistened pieces of filter paper in Petri dishes. The leaves are then sprayed with the test solution using a rotating turntable and allowed to dry. The dishes are infested with 10 third instar larvae of Southern armyworm or Mexican bean beetle. The dishes are then covered.

The percent mortality for the bean beetle and armyworm evaluations are determined 96 hours after treatment. Evaluations are based on a scale of 0-100% in which 0 equals no activity and 100 equals total kill.

The rotating turntable consists of a fixed, continuously operating spray nozzle under which targets are rotated at a fixed speed and distance. When the target is a Petri dish (such as for the armyworm), the distance from the nozzle is 15 inches. The nozzle is located 8 inches from the rotating shaft. The targets on individual platforms revolve around the shaft at 1 revolution per 20 seconds but only a brief portion of this time occurs in the spray path. Targets pass only once under the nozzle and then are removed to drying hoods.

The nozzle used is a 1/4 JCO Spraying Systems (Wheaton, Illinois) air atomizing nozzle equipped with a No. 2850 fluid cap and No. 70 air cap. At the 10 psig air pressure used and with liquid siphon feed 0.5 GPH (gallons per hour) are delivered in a round spray pattern with a 21° spray angle. Targets are misted with spray droplets to the point that the droplets coalesce to form a uniform thin film insufficient to drown test organisms.

All treatments are maintained at 75-80°F under continuous fluorescent light in a well-ventilated room.

For soil treatment (systemic) trials, a portion of the 600 ppm test solution is diluted to 150 ppm. Ten (10) ml of the 150 ppm test solution is pipetted into soil (approximately 200 g of standard greenhouse soil) in a 3-inch pot containing a lima bean seedling. This results in a soil concentration of approximately 8 ppm. Treated plants are maintained under existing greenhouse conditions for one week. Two bean leaves are removed and placed individually on moist filter paper in Petri dishes. One leaf is infested with 10 third instar larvae of Mexican bean beetle. The other leaf is infested with 10 third instar larvae of Southern armyworm. The dishes are then covered and held for 3 days at which time the percent control (mortality) is determined. A second observation may be made 6 days after infesting the dishes if the experimenter feels the effect may not be complete or moribund insects appear to evidence signs of some recovery. Where necessary, untreated bean leaves are introduced into dishes held for a second observation to preclude insect starvation.

The results of the initial insecticidal evaluations are given in Table II.

Armyworm and bean beetle spray (foliar) results are 96 hour observations. Soil treatment results are 72 hour observations. At the discretion of the experimenter, particular evaluations were held for 144 hour observations. If, after 144 hours, there was a change in the percent control, it is shown in parentheses.

TABLE II

Biological Evaluations

| Example No. | Foliar Application Test Species SAW | Foliar Application Test Species MBB | Soil Application Test Species MBB | Soil Application Test Species SAW |
|---|---|---|---|---|
| 1 | 100 | 0 | 20 | 100 |
| 2 | 100 | 60 | 20 | 40(60) |
| 3 | 70 | 100 | 80(20) | 40(100) |
| 4 | 100 | 40 | 40 | 0(80) |
| 5 | 0 | 10 | 0(40) | 0(20) |
| 6 | 90 | 60 | 80(60) | 0(20) |
| 7 | 20 | 10 | 0(40) | 40(60) |
| 8 | 0 | 0 | 0(20) | 0 |
| 9 | 70 | 100 | 20(40) | 0 |
| 10 | 80 | 70 | 40(100) | 40(80) |
| 11 | 100 | 0 | 0 | 100 |
| 12 | 30 | 0 | 0 | 0 |
| 13 | 0 | 90 | 80(100) | 0 |
| 14 | 0 | 80 | 0(80) | 0 |
| 15 | 0 | 70 | 0 | 0 |
| 16 | 100 | 20 | 0 | 0 |
| 17 | 70 | 40 | 0 | 0 |
| 18 | 100 | 0 | - | -[1] |
| 19 | 0 | 0 | 0 | 0 |
| 20 | 60 | 30 | 0(20) | 0 |

[1] No data reported.

Further examples of compounds which were prepared generally according to the above Procedures A to E were as follows :

TABLE III

| Ex. No. | X | X' | $R^1$ | A | B | m.p. °C |
|---|---|---|---|---|---|---|
| 21 | 0 | 0 | $-C(CH_3)_3$ | 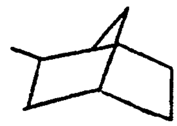 | $-C_6H_4CH_3$-3 | 190-192 |
| 22 | 0 | 0 | $-C(CH_3)_3$ | 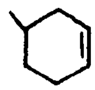 | $-C_6H_4CH_3$-3 | 160-162.5 |
| 23 | 0 | 0 | $-C(CH_3)_3$ | 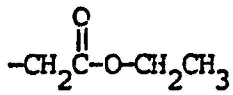 | $-C_6H_4Cl_2$-3,4 | 110-112 |
| 24 | 0 | 0 | $-C(CH_3)_3$ | 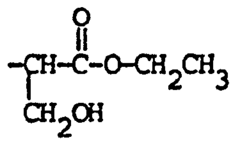 | $-C_6H_4Cl_2$-3,4 | low melting solid |
| 25 | 0 | 0 | $-C(CH_3)_3$ | 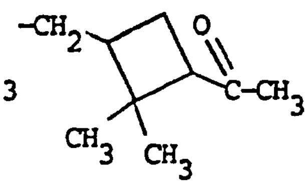 | $-C_6H_4CH_3$-3 | oil |
| 26 | 0 | 0 | $-C(CH_3)_3$ | $-CH{=}CHCH_2CH_3$ | $-C_6H_4CH_3$-3 | 144-146 |
| 27 | 0 | 0 | $-C(CH_3)_3$ | $-CH_2CH_2CH{=}CH_2$ | $-C_6H_4CH_3$-3 | 110-115 |
| 28 | 0 | 0 | $-C(CH_3)_3$ | 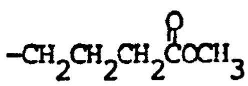 | $-C_6H_4CH_3$-3 | oil |
| 29 | 0 | 0 | $-C(CH_3)_3$ | $-CH(CH_3)_2$ | $-C_6H_3(CH_2)_2$-3,5 | 136-138 |
| 30 | 0 | 0 | $-C(CH_3)_3$ | 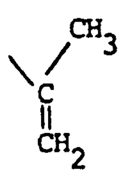 | $-C_6H_3(CH_2)_2$-3,5 | 155-163 |
| 31 | 0 | 0 | $-C(CH_3)_3$ | $-CH(CH_3)_2$ | $-C_6H_4Br$-2 | 160-162 |

| Ex. No. | X | X' | $R^1$ | A | B | m.p. °C |
|---|---|---|---|---|---|---|
| 32 | 0 | 0 | $-C(CH_3)_3$ | $-CH(CH_3)_2$ | $-C_6H_5$ | 129-131 |
| 33 | 0 | 0 | $-C(CH_3)_3$ | 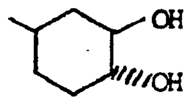 | $-C_6H_4CH_3$-3 | glass |
| 34 | 0 | 0 | $-C(CH_3)_3$ | 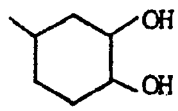 | $-C_6H_4CH_3$-3 | glass |
| 35 | 0 | 0 | $-C(CH_3)_3$ | 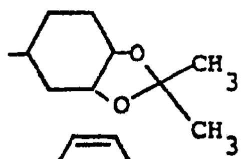 | $-C_6H_4CH_3$-3 | oil |
| 36 | 0 | 0 | $-C(CH_3)_3$ | 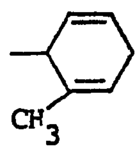 | $-C_6H_4CH_3$-3 | 207-210 |

Biological evaluation of these further Examples by the above described procedures gave the following results :

TABLE IV

| | Foliar Application | |
|---|---|---|
| | Test Species | |
| Example No. | SAW | MBB |
| 21 | 100 | 0 |
| 22 | 100 | 0 |
| 23 | 0 | 20 |
| 24 | 0 | 30 |
| 25 | 90 | 40 |
| 26 | 100 | 30 |
| 27 | 100 | 100 |
| 28 | 20 | 40 |
| 29 | 100 | 50 |
| 30 | 100 | 30 |
| 31 | 0 | 80 |
| 32 | 0 | 40 |
| 33 | 50 | 30 |
| 34 | 100 | 30 |
| 35 | 100 | 10 |
| 36 | 100 | 100 |

The following words are trademarks which may or may not be registered in some or all of the designated states :
Triton, Agsorb, Duponal, Reax, HiSil, Sponto, Tenneco, Kathon and Kelzan.

## Claims

**Claims for the Contracting States : BE, CH, DE, FR, GB, GR, IT, LI, LU, NL, SE**

1. An insecticidal compound having the formula :

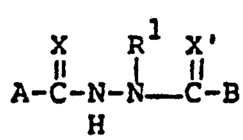

I

Wherein

X and X' are the same or different 0, S or NR ;

$R^1$ is unsubstituted ($C_3$-$C_{10}$) branched alkyl or ($C_1$-$C_4$) straight chain alkyl substituted with one or two of the same or different ($C_3$-$C_6$)cycloalkyl ;

A is unsubstituted or substituted ($C_1$-$C_{10}$)alkyl having one to four of the same or different halo, cyano, nitro,

hydroxy, $(C_1-C_4)$alkoxy, carboxy, $(C_1-C_4)$alkoxy-carbonyl, $(C_1-C_4)$alkanoyloxy, $NH_2$, NHZ or NZZ' ;

unsubstituted or substituted $(C_3-C_8)$cycloalkyl or $(C_3-C_8)$cycloalkyl-$(C_1-C_4)$alkyl having one to four of the same or different halo, cyano, nitro, hydroxy, $(C_1-C_4)$alkyl, halo$(C_1-C_4)$alkyl, $(C_1-C_4)$-alkoxy, halo-$(C_1-C_4)$alkoxy, carboxy, $(C_1-C_4)$alkanoyl, $(C_1-C_4)$alkoxy-carbonyl, $(C_1-C_4)$alkanoyloxy, $NH_2$, NHZ or NZZ' ;

unsubstituted or substituted $(C_2-C_8)$alkenyl or unsubstituted or substituted $(C_3-C_8)$-alkadienyl having one to four of the same or different halo, cyano, nitro, hydroxy, $(C_1-C_4)$alkyl, $(C_3-C_6)$cycloalkyl, halo-$(C_1-C_4)$alkyl, $(C_1-C_4)$alkoxy, halo$(C_1-C_4)$-alkoxy, carboxy, $(C_1-C_4)$altoxy-carbonyl, $(C_1-C_4)$alkanoyloxy, $NH_2$, NHZ or NZZ' ;

unsuhstituted or substituted $(C_3-C_8)$cycloalkenyl or unsubstituted or substituted $(C_6-C_8)$cycloalkadienyl having one to four of the same or different halo, cyano, nitro, hydroxy, $(C_1-C_4)$alkyl, halo$(C_1-C_4)$-alkyl, $(C_1-C_4)$alkoxy, halo$(C_1-C_4)$alkoxy, carboxy, $(C_1-C_4)$alkoxy-carbonyl, $(C_1-C_4)$-alkanoyloxy, $NH_2$, NHZ or NZZ' ;

unsubstituted or substituted $(C_2-C_8)$alkynyl having one to four of the same or different halo, cyano, nitro, hydroxy, $(C_1-C_4)$alkyl, halo$(C_1-C_4)$alkyl, $(C_1-C_4)$alkoxy, halo-$(C_1-C_4)$alkoxy, carboxy, $(C_1-C_4)$-alkoxy-carbonyl, $(C_1-C_4)$alkanoyloxy, $NH_2$, NHZ or NZZ' ; or

phenalkyl having one to four carbon atoms in the alkyl group and the phenyl ring is unsubstituted or substituted with one to three of the same or different halo, cyano, nitro, hydroxy, $(C_1-C_4)$alkyl, halo$(C_1-C_4)$alkyl, cyano$(C_1-C_4)$alkyl, $(C_1-C_4)$alkoxy, halo$(C_1-C_4)$alkoxy, carboxy, $(C_1-C_4)$alkoxy-carbonyl, $(C_1-C_4)$alkanoyloxy, $(C_2-C_6)$alkenyl, halo$(C_2-C_6)$alkenyl, $(C_2-C_6)$alkynyl, $NH_2$, NHZ or NZZ' ;

B is unsubstituted or substituted naphthyl where the substituents can be from one to three of the same or different halo ; cyano ; nitro ; hydroxy ; $(C_1-C_4)$alkoxy ; $(C_1-C_4)$-alkyl ; carboxy ; $(C_1-C_4)$alkoxy-carbonyl ; $(C_1-C_4)$alkanoyloxy ; $NH_2$, NHZ or NZZ' ;

unsubstituted or substituted phenyl where the substituents car be from one to five of the same or different halo ; nitro ; cyano ; hydroxy ; $(C_1-C_6)$alkyl ; halo$(C_1-C_6)$alkyl ; cyano$(C_1-C_6)$alkyl ; $(C_1-C_6)$alkoxy ; halo$(C_1-C_6)$alkoxy ; alkoxyalkyl having independently 1 to 6 carbon atoms in each alkyl group ; alkoxyalkoxy having independently 1 to 6 carbon atoms in each alkyl group ; $-OCO_2R$ group ; $(C_2-C_6)$alkenyl optionally substituted with halo, cyano, $(C_1-C_4)$alkyl, $(C_1-C_4)$alkoxy, halo$(C_1-C_4)$alkoxy or $(C_1-C_4)$alkylthio ; $-RCO_2R'$ group; $-COR$ group ; halo$(C_1-C_6)$alkyl-carbonyl ; cyano$(C_1-C_6)$alkyl-carbonyl ; nitro$(C_1-C_6)$alkyl-carbonyl ; $-CO_2R$ group ; halo$(C_1-C_6)$alkoxy-carbonyl ; $-OCOR$ group ; $-NRR'$ group ; amino substituted with hydroxy, $(C_1-C_4)$alkoxy or $(C_1-C_4)$alkylthio groups ; phenylamino ; diphenylamino ; $-CONRR'$ group ; $-OCONRR'$ group ; $-NRCOR'$ group ; $-NRCO_2R'$ group ; $-N(COR)COR'$ group ; $-OCONRCOR'$ group; sulfhydryl ; halothio ; $(C_1-C_6)$alkylthio ; halo$(C_1-C_6)$alkylthio ; $-SOR$ group ; $-SO_2R$ group ; phenylsulfonyl; $-OSO_2R$ group ; halo$(C_1-C_6)$alkylsulfonyloxy ; $-SO_2NRR'$ group ; $-NRSOR'$ group ; $-NRSO_2R'$ group ; $-CSR$ group ; $-CS_2R$ group ; $-NRCSR'$ group ; $-SCOR$ group ; unsubstituted or substituted phenyl having one to three of the same or different halo, cyano, nitro, hydroxy $(C_1-C_4)$alkyl, $(C_1-C_4)$alkoxy, carboxy, $(C_1-C_4)$alkoxy-carbonyl, $(C_1-C_4)$alkanoyloxy, $NH_2$ group ; NHZ group ; or NZZ' group ; phenoxy where the phenyl ring is unsubstituted or substituted with one to three of the same or different halo, cyano, nitro, hydroxy, $(C_1-C_4)$alkyl, $(C_1-C_4)$alkoxy, carboxy, $(C_1-C_4)$alkoxy-carbonyl, $(C_1-C_4)$alkanoyloxy, $NH_2$ group ; NHZ group ; or NZZ' group ; phenylthio where the phenyl ring is unsubstituted or substituted with one to three of the same or different halo, cyano, nitro, hydroxy, $(C_1-C_4)$alkyl, $(C_1-C_4)$alkoxy, carboxy, $(C_1-C_4)$alkoxy-carbonyl, $(C_1-C_4)$alkanoyloxy, $NH_2$ group ; NHZ group ; or NZZ' group ; or when two adjacent positions on the phenyl ring are substituted with alkoxy groups, these groups may be joined to form, together with the carbon atoms to which they are both attached, a 5 or 6 membered dioxolano or dioxano heterocyclic ring;

where R, R' and R" are hydrogen or $(C_1-C_6)$alkyl ; Z and Z' are $(C_1-C_4)$alkyl ;

"amino" means NRR' ; and

agronomically acceptable salts thereof.

2. A compound according to claim 1 wherein

X and X' are O or S ; and/or

$R^1$ is unsubstituted $(C_3-C_8)$ branched alkyl or $(C_1-C_4)$ straight chain alkyl substituted with one or two of the same or different $(C_3-C_4)$cycloalkyl ; and/or

A is unsubstituted or substituted $(C_1-C_6)$alkyl having one to three of the same or different halo, cyano, nitro, $(C_1-C_4)$alkoxy, carboxy, $(C_1-C_4)$alkoxy-carbonyl, $(C_1-C_4)$alkanoyloxy, $NH_2$, NHZ or NZZ' ;

$(C_3-C_6)$cycloalkyl ;

$(C_2-C_6)$ unsubstituted or substituted alkenyl having one to three of the same or different halo, $(C_1-C_4)$ alkyl, halo$(C_1-C_4)$-alkyl, $(C_1-C_4)$alkoxy, or halo$(C_1-C_4)$alkoxy ;

$(C_3-C_6)$cycloalkenyl ; or

phenalkyl having one or two carbon atoms in the alkyl group and the phenyl ring is unsubstituted or substituted with one or two of the same or different halo, nitro or $(C_1-C_4)$alkyl ; and/or

B is unsubstituted naphthyl ; or
unsubstituted or substituted phenyl where the substituents can be from one to three of the same of different halo ; nitro ; cyano ; $(C_1\text{-}C_4)$alkyl ; halo$(C_1\text{-}C_4)$alkyl ; cyano$(C_1\text{-}C_4)$alkyl ; $(C_1\text{-}C_4)$alkoxy ; alkoxyalkyl having independently 1 to 4 carbon atoms in each alkyl group ; $-COD^4$ group ; carboxy ; $(C_1\text{-}C_4)$alkoxy-carbonyl; $(C_1\text{-}C_4)$alkanoyloxy ; $NH_2$ ; NHZ ; NZZ' ; $(C_1\text{-}C_4)$alkylthio ; $-CSD^4$ group ; $-CS_2D^4$ group ; $-SCOD^4$ group; unsubstituted or substituted phenyl having one to two of the same or different halo, nitro, $(C_1\text{-}C_4)$ alkyl, $(C_1\text{-}C_4)$alkoxy, carboxy, $(C_1\text{-}C_4)$alkoxy-carbonyl, $(C_1\text{-}C_4)$alkanoyloxy, $NH_2$, NHZ or NZZ' ; phenoxy where the phenyl ring is unsubstituted or substituted with one or two of the same or different halo, nitro, $(C_1\text{-}C_4)$alkyl, $(C_1\text{-}C_4)$alkoxy, carboxy, $(C_1\text{-}C_4)$alkoxy-carbonyl, $(C_1\text{-}C_4)$alkanoyloxy, $NH_2$, NHZ or NZZ' ; or when two adjacent positions on the phenyl ring are substituted with alkoxy groups, these groups may be joined to form, together with the carbon atoms to which they are attached, a 5- or 6-membered dioxolano or dioxano heterocyclic ring ;
where $D^4$ is hydrogen or $(C_1\text{-}C_4)$alkyl ; Z and Z' are $(C_1\text{-}C_4)$alkyl ; and
agronomically acceptable salts thereof.
3. A compound according to claim 2 wherein
X and X' are O or S ; and/or
$R^1$ is branched $(C_3\text{-}C_8)$alkyl ; and/or
A is unsubstituted or substituted $(C_1\text{-}C_6)$alkyl having one to three of the same or different halo, cyano, nitro, carboxy or $(C_1\text{-}C_4)$alkoxy-carbonyl ;
cyclohexyl ;
cyclohexenyl ;
unsubstituted or substituted $(C_2\text{-}C_6)$alkenyl having one to three of the same or different halo or $(C_1\text{-}C_4)$alkyl ; or
benzyl where the phenyl ring is unsubstituted or substituted with one or two of the same or different halo, methyl or ethyl ; and/or
B is unsubstituted naphthyl ;
unsubstituted or substituted phenyl having one to three of the same or different halo ; nitro ; cyano ; $(C_1\text{-}C_4)$alkyl ; halo$(C_1\text{-}C_4)$alkyl ; cyano$(C_1\text{-}C_4)$alkyl ; $(C_1\text{-}C_4)$alkoxy ; $-COD^4$ group ; $(C_1\text{-}C_4)$alkoxy-carbonyl ; $(C_1\text{-}C_4)$alkanoyloxy ; or unsubstituted or substituted phenyl having one or two of the same or different halo, nitro, $(C_1\text{-}C_4)$alkyl, $(C_1\text{-}C_4)$alkoxy, carboxy, $(C_1\text{-}C_4)$alkoxy-carbonyl, $(C_1\text{-}C_4)$alkanoyloxy, $NH_2$, NHZ or NZZ';
where $D^4$ is hydrogen or $(C_1\text{-}C_4)$alkyl ; Z and Z' are $(C_1\text{-}C_4)$alkyl ; and
agronomically acceptable salts thereof.
4. A compound according to claim 3 wherein
X and X' are O ; and/or
$R^1$ is branched $(C_4\text{-}C_7)$alkyl ; and/or
A is unsubstituted or substituted $(C_1\text{-}C_6)$alkyl having one to two of the same or different halo ;
cyclohexyl ;
unsubstituted or substituted $(C_2\text{-}C_3)$alkenyl having one to three of the same or different halo, methyl or ethyl ;
cyclohexenyl ; or
benzyl ; and/or
B is unsubstituted or substituted phenyl where the substituents can be from one to three of the same or different halo, $(C_1\text{-}C_4)$alkyl, $(C_1\text{-}C_4)$alkoxy, or halo$(C_1\text{-}C_4)$alkyl ; and
agronomically acceptable salts thereof.
5. A compound according to claim 4 wherein
X and X' are O ; and/or
$R^1$ is t-butyl, neopentyl (2,2-dimethylpropyl) or 1,2,2-trimethylpropyl ; and/or
A is $(C_3\text{-}C_5)$ unsubstituted alkyl ;
cyclohexyl ;
$(C_2\text{-}C_4)$ unsubstituted or substituted straight chain alkenyl having one to three of the same or different chloro, bromo or methyl ;
cyclohexenyl ; or
benzyl ; and/or
B is unsubstituted or substituted phenyl where the substituents can be one or two of the same or different chloro, fluoro, bromo, iodo, methyl, ethyl, methoxy or trifluoromethyl ; and
agronomically acceptable salts thereof.
6. A compound according to claim 1 wherein
X and X' are O ;

$R^1$ is t-butyl ; and

B is phenyl and A is cyclohexyl, n-butyl, n-propyl, benzyl, 1-cyclohexenyl, dichloromethyl or trichloromethyl; or

B is 4-chlorophenyl and A is n-propyl or t-butyl ; or

B is 3-methylphenyl and A is norbornyl, cyclohex-3-enyl, 1-butenyl, 3-butenyl, cis-3,4-dihydroxycyclohexyl, 2,2-dimethyl-3a,4,5,6,7,7a-hexahydrobenzo[d]-dioxalano-5-yl, 3-acetyl-2,2-dimethyl-cyclobutyl-methyl or 2-methylcyclohexyl di-2,5-enyl ; or

B is 3,5-dimethylphenyl and A is isopropyl or 1-methylethenyl.

7. A compound as claimed in Claim 2 wherein B is unsubstituted phenyl, 4-chlorophenyl, 3-substituted phenyl, 2-substituted phenyl or disubstituted phenyl.

8. An insecticidal composition comprising an insecticidal compound as claimed in any preceding claim together with agronomically acceptable diluent or carrier.

9. A composition according to claim 8 wherein said insecticidal compound is present at from 0.0001 to 99% by weight of the composition.

10. A composition according to claim 8 wherein said insecticidal compound is present at from 0.001 to 99% by weight of the composition.

11. A composition according to claim 8 wherein said insecticidal compound is present at from 0.01 to 99% by weight of the composition.

12. An insecticidal composition according to any of claims 8 to 11 (a) additionally containing an emulsifying agent, said composition being in the form of an emulsifiable concentrate ; or (b) additionally containing a dispersing agent, said composition being in the form of a wettable powder ; or (c) containing a liquid agronomically acceptable carrier and a dispersing agent, said composition being in the form of a flowable ; or (d) in the form of a dust ; or (e) additionally containing a binding agent, said composition being in the form of a granule ; or (f) additionally containing an attractant agent, said composition being in the form of a bait.

13. A method of controlling insects which comprises contacting said insects with an insecticidally effective amount of a compound according to any one of claims 1 to 7, optionally in a composition according to any of claims 8 to 12.

14. A method according to claim 13 wherein the insecticidal compound is applied to growing plants or an area where plants are to be grown at a dosage rate of 10 grams to 10 kilograms per hectare.

15. A method according to claim 14 wherein the application rate is 100 grams to 5 kilograms per hectare.

16. A method according to claim 13, 14 or 15 for killing insects from the order Lepidoptera or Coleoptera.

**Claims for the Contacting State : ES**

1. The use of an insecticidal compound having the formula :

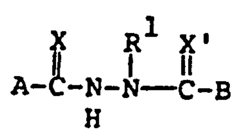

I

wherein

X and X' are the same or different O, S or NR ;

$R^1$ is unsubstituted ($C_3$-$C_{10}$) branched alkyl or ($C_1$-$C_4$) straight chain alkyl substituted with one or two of the same or different ($C_3$-$C_6$)cycloalkyl ;

A is unsubstituted or substituted ($C_1$-$C_{10}$)alkyl having one to four of the same or different halo, cyano, nitro, hydroxy, ($C_1$-$C_4$)alkoxy, carboxy, ($C_1$-$C_4$)alkoxy-carbonyl, ($C_1$-$C_4$)-alkanoyloxy, $NH_2$, NHZ or NZZ' ;

unsubstituted or substituted ($C_3$-$C_8$)cycloalkyl or ($C_3$-$C_8$)cycloalkyl-($C_1$-$C_4$)alkyl having one to four of the same or different halo, cyano, nitro, hydroxy, ($C_1$-$C_4$)alkyl, halo($C_1$-$C_4$)alkyl, ($C_1$-$C_4$)-alkoxy, halo($C_1$-$C_4$)alkoxy, carboxy, ($C_1$-$C_4$)alkanoyl, ($C_1$-$C_4$)alkoxy-carbonyl, ($C_1$-$C_4$)alkanoyloxy, $NH_2$, NHZ or NZZ' ;

unsubstituted or substituted ($C_2$-$C_8$)alkenyl or unsubstituted or substituted ($C_3$-$C_8$)-alkadienyl having one to four of the same or different halo, cyano, nitro, hydroxy, ($C_1$-$C_4$)alkyl, ($C_3$-$C_8$)cycloalkyl, halo-($C_1$-$C_4$)alkyl, ($C_1$-$C_4$)alkoxy, halo($C_1$-$C_4$)-alkoxy, carboxy, ($C_1$-$C_4$)alkoxy-carbonyl, ($C_1$-$C_4$)alkanoyloxy, $NH_2$, NHZ or NZZ' ;

unsubstituted or substituted ($C_3$-$C_8$)cycloalkenyl or unsubstituted or substituted ($C_6$-$C_8$)cycloalkadienyl having one to four of the same or different halo, cyano, nitro, hydroxy, ($C_1$-$C_4$)alkyl, halo($C_1$-$C_4$)-alkyl, ($C_1$-$C_4$)alkoxy, halo($C_1$-$C_4$)alkoxy, carboxy, ($C_1$-$C_4$)alkoxy-carbonyl, ($C_1$-$C_4$)-alkanoyloxy, $NH_2$, NHZ or

NZZ' ;
unsubstituted or substituted ($C_2$-$C_8$)alkynyl having one to four of the same or different halo, cyano, nitro, hydroxy, ($C_1$-$C_4$)alkyl, halo($C_1$-$C_4$)alkyl, ($C_1$-$C_4$)alkoxy, halo-($C_1$-$C_4$)alkoxy, carboxy, ($C_1$-$C_4$)-alkoxy-carbonyl, ($C_1$-$C_4$)alkanoyloxy, $NH_2$, NHZ or NZZ' ; or
phenalkyl having one to four carbon atoms in the alkyl group and the phenyl ring is unsubstituted or substituted with one to three of the same or different halo, cyano, nitro, hydroxy, ($C_1$-$C_4$)alkyl, halo($C_1$-$C_4$)-alkyl, cyano($C_1$-$C_4$)alkyl, ($C_1$-$C_4$)alkoxy, halo($C_1$-$C_4$)alkoxy, carboxy, ($C_1$-$C_4$)-alkoxy-carbonyl, ($C_1$-$C_4$)alkanoyloxy, ($C_2$-$C_6$)alkenyl, halo($C_2$-$C_6$)alkenyl, ($C_2$-$C_6$)alkynyl, $NH_2$, NHZ or NZZ' ;
B is unsubstituted or substituted naphthyl where the substituents can be from one to three of the same or different halo ; cyano ; nitro ; hydroxy ; ($C_1$-$C_4$)alkoxy ; ($C_1$-$C_4$)-alkyl ; carboxy ; ($C_1$-$C_4$)alkoxy-carbonyl ; ($C_1$-$C_4$)alkanoyloxy ; $NH_2$, NHZ or NZZ' ;
unsubstituted or substituted phenyl where the substituents can be from one to five of the same or different halo ; nitro ; cyano ; hydroxy ; ($C_1$-$C_6$)alkyl ; halo($C_1$-$C_6$)alkyl ; cyano($C_1$-$C_6$)alkyl ; ($C_1$-$C_6$)alkoxy ; halo-($C_1$-$C_6$)alkoxy ; alkoxyalkyl having independently 1 to 6 carbon atoms in each alkyl group ; alkoxyalkoxy having independently 1 to 6 carbon atoms in each alkyl group ; $-OCO_2R$ group ; ($C_2$-$C_6$)alkenyl optionally substituted with halo, cyano, ($C_1$-$C_4$)alkyl, ($C_1$-$C_4$)alkoxy, halo($C_1$-$C_4$)-alkoxy or ($C_1$-$C_4$)alkylthio ; $-RCO_2R'$ group ; $-COR$ group ; halo($C_1$-$C_6$)alkyl-carbonyl ; cyano($C_1$-$C_6$)alkyl-carbonyl ; nitro($C_1$-$C_6$)-alkyl-carbonyl ; $-CO_2R$ group ; halo($C_1$-$C_6$)-alkoxy-carbonyl ; $-OCOR$ group ; $-NRR'$ group ; amino substituted with hydroxy, ($C_1$-$C_4$)-alkoxy or ($C_1$-$C_4$)alkylthio groups ; phenyl-amino ; diphenylamino ; $-CONRR'$ group; $-OCONRR'$ group ; $-NRCOR'$ group ; $-NRCO_2R'$ group ; $-N(COR)COR'$ group ; $-OCONRCOR'$ group; sulfhydryl ; halothio ; ($C_1$-$C_6$)alkylthio ; halo($C_1$-$C_6$)alkylthio ; $-SOR$ group ; $-SO_2R$ group ; phenylsulfonyl; $-OSO_2R$ group ; halo-($C_1$-$C_6$)alkylsulfonyloxy ; $-SO_2NRR'$ group ; $-NRSOR'$ group ; $-NRSO_2R'$ group ; $-CSR$ group ; $-CS_2R$ group ; $-NRCSR'$ group ; $-SCOR$ group ; unsubstituted or substituted phenyl having one to three of the same or different halo, cyano, nitro, hydroxy ($C_1$-$C_4$)alkyl, ($C_1$-$C_4$)alkoxy, carboxy, ($C_1$-$C_4$)-alkoxy-carbonyl, ($C_1$-$C_4$)alkanoyloxy, $NH_2$ group ; NHZ group ; or NZZ' group ; phenoxy where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy, $NH_2$ group ; NHZ group ; or NZZ' group ; phenylthio where the phenyl ring is unsubstituted or substituted with one to three of the same or different halo, cyano, nitro, hydroxy, ($C_1$-$C_4$)alkyl, ($C_1$-$C_4$)-alkoxy, carboxy, ($C_1$-$C_4$)alkoxy-carbonyl, ($C_1$-$C_4$)alkanoyloxy, $NH_2$ group ; NHZ group ; or NZZ' group ; or when two adjacent positions on the phenyl ring are substituted with alkoxy groups, these groups may be joined to form, together with the carbon atoms to which they are both attached, a 5 or 6 membered dioxolano or dioxano heterocyclic ring; where R, R' and R" are hydrogen or ($C_1$-$C_6$)alkyl ; Z and Z' are ($C_1$-$C_4$)alkyl ; "amino" means NRR' ; or an agronomically acceptable salt thereof, together with agronomically acceptable diluent or carrier, in making an insecticidal composition.

2. The use according to claim 1 of a compound or salt wherein,
X and X' are O or S ; and/or
$R^1$ is unsubstituted ($C_3$-$C_8$) branched alkyl or ($C_1$-$C_4$) straight chain alkyl substituted with one or two of the same or different ($C_3$-$C_4$)cycloalkyl ; and/or
A is unsubstituted or substituted ($C_1$-$C_6$)alkyl having one to three of the same or different halo, cyano, nitro, ($C_1$-$C_4$)-alkoxy, carboxy, ($C_1$-$C_4$)alkoxy-carbonyl, ($C_1$-$C_4$)alkanoyloxy, $NH_2$, NHZ or NZZ' ;
($C_3$-$C_6$)cycloalkyl ;
($C_2$-$C_6$) unsubstituted or substituted alkenyl having one to three of the same or different halo, ($C_1$-$C_4$) alkyl, halo($C_1$-$C_4$)-alkyl, ($C_1$-$C_4$)alkoxy, or halo($C_1$-$C_4$)alkoxy ;
($C_3$-$C_6$)cycloalkenyl ; or
phenalkyl having one or two carbon atoms in the alkyl group and the phenyl ring is unsubstituted or substituted with one or two of the same or different halo, nitro or ($C_1$-$C_4$)alkyl ; and/or
B is unsubstituted naphthyl ; or
unsubstituted or substituted phenyl where the substituents can be from one to three of the same of different halo ; nitro ; cyano ; ($C_1$-$C_4$)alkyl ; halo($C_1$-$C_4$)alkyl ; cyano($C_1$-$C_4$)alkyl ; ($C_1$-$C_4$)alkoxy ; alkoxyalkyl having independently 1 to 4 carbon atoms in each alkyl group ; $-COD^4$ group ; carboxy ; ($C_1$-$C_4$)alkoxy-carbonyl; ($C_1$-$C_4$)-alkanoyloxy ; $NH_2$ ; NHZ ; NZZ' ; ($C_1$-$C_4$)alkylthio ; $-CSD^4$ group ; $-CS_2D^4$ group ; $-SCOD^4$ group ; unsubstituted or substituted phenyl having one to two of the same or different halo, nitro, ($C_1$-$C_4$) alkyl, ($C_1$-$C_4$)alkoxy, carboxy, ($C_1$-$C_4$)alkoxy-carbonyl, ($C_1$-$C_4$)-alkanoyloxy, $NH_2$, NHZ or NZZ' ; phenoxy where the phenyl ring is unsubstituted or substituted with one or two of the same or different halo, nitro, ($C_1$-$C_4$)alkyl, ($C_1$-$C_4$)alkoxy, carboxy, ($C_1$-$C_4$)-alkoxy-carbonyl, ($C_1$-$C_4$)alkanoyloxy, $NH_2$, NHZ or NZZ' ; or when two adjacent positions on the phenyl ring are substituted with alkoxy groups, these groups may be joined to form, together with the carbon atoms to which they are attached, a 5- or 6-membered dioxolano

or dioxano heterocyclic ring ;
where $D^4$ is hydrogen or $(C_1$-$C_4)$alkyl ; Z and Z′ are $(C_1$-$C_4)$alkyl

3. The use, according to claim 2, of a compound or salt wherein
X and X′ are O or S ; and/or
$R^1$ is branched $(C_3$-$C_8)$alkyl ; and/or
A is unsubstituted or substituted $(C_1$-$C_6)$alkyl having one to three of the same or different halo, cyano, nitro, carboxy or $(C_1$-$C_4)$alkoxy-carbonyl ;
cyclohexyl ;
cyclohexenyl ;
unsubstituted or substituted $(C_2$-$C_6)$alkenyl having one to three of the same or different halo or $(C_1$-$C_4)$alkyl ; or
benzyl where the phenyl ring is unsubstituted or substituted with one or two of the same or different halo, methyl or ethyl ; and/or
B is unsubstituted naphthyl ;
unsubstituted or substituted phenyl having one to three of the same or different halo ; nitro ; cyano ; $(C_1$-$C_4)$ alkyl ; halo$(C_1$-$C_4)$-alkyl ; cyano$(C_1$-$C_4)$alkyl ; $(C_1$-$C_4)$alkoxy ; –$COD^4$ group ; $(C_1$-$C_4)$alkoxy-carbonyl ; $(C_1$-$C_4)$alkanoyloxy ; or unsubstituted or substituted phenyl having one or two of the same or different halo, nitro, $(C_1$-$C_4)$-alkyl, $(C_1$-$C_4)$alkoxy, carboxy, $(C_1$-$C_4)$-alkoxy-carbonyl, $(C_1$-$C_4)$alkanoyloxy, $NH_2$, NHZ or NZZ′ ; where $D^4$ is hydrogen or $(C_1$-$C_4)$alkyl ; Z and Z′ are $(C_1$-$C_4)$alkyl.

4. The use, according to claim 3, of a compound or salt wherein
X and X′ are O ; and/or
$R^1$ is branched $(C_4$-$C_7)$alkyl ; and/or
A is unsubstituted or substituted $(C_1$-$C_6)$alkyl having one to two of the same or different halo ;
cyclohexyl ;
unsubstituted or substituted $(C_2$-$C_6)$alkenyl having one to three of the same of different halo, methyl or ethyl ;
cyclohexenyl ; or
benzyl ; and/or
B is unsubstituted or substituted phenyl where the substituents can be from one to three of the same or different halo, $(C_1$-$C_4)$alkyl, $(C_1$-$C_4)$alkoxy, or halo-$(C_1$-$C_4)$alkyl.

5. The use, according to claim 4, of a compound or salt wherein
X and X′ are O ; and/or
$R^1$ is t-butyl, neopentyl (2,2-dimethylpropyl) or 1,2,2-trimethylpropyl ; and/or
A is $(C_3$-$C_5)$ unsubstituted alkyl ;
cyclohexyl ;
$(C_2$-$C_4)$ unsubstituted or substituted straight chain alkenyl having one to three of the same or different chloro, bromo or methyl ;
cyclohexenyl ; or
benzyl ; and/or
B is unsubstituted or substituted phenyl where the substituents can be one or two of the same or different chloro, fluoro, bromo, iodo, methyl, ethyl, methoxy or trifluoromethyl.

6. The use, according to claim 1, of a compound or salt wherein
X and X′ are O ;
$R^1$ is t-butyl ; and
B is phenyl and A is cyclohexyl, n-butyl, n-propyl,-benzyl, 1-cyclohexenyl, dichloromethyl or trichloromethyl ; or
B is 4-chlorophenyl and A is n-propyl or t-butyl ; or
B is 3-methylphenyl and A is norbornyl, cyclohex-3-enyl, 1-butenyl, 3-butenyl, cis-3,4-dihydroxycyclohexyl, 2,2-dimethyl-3a,4,5,6,7,7a-hexahydrobenzo[d]-dioxalano-5-yl, 3-acetyl-2,2-dimethyl-cyclobutylmethyl or 2-methylcyclohexyl di-2,5-enyl ; or
B is 3,5-dimethylphenyl and A is isopropyl or 1-methylethenyl.

7. The use according to claim 2 of the compound or salt wherein B is unsubstituted phenyl, 4-chlorophenyl, 3-substituted phenyl, 2-substituted phenyl or disubstituted phenyl.

8. The use according to any preceding claim of the compound or salt in an amount of from 0.0001 to 99% by weight of the composition.

9. The use according to claim 8 of the compound or salt in an amount of from 0.001 to 99% by weight of the composition.

10. The use according to claim 8 of the compound or salt in an amount of from 0.01 to 99% by weight of

the composition.

11. The use according to any preceding claim of the compound or salt (a) together with emulsifying agent, in making an emulsifiable concentrate ; or (b) together with a dispersing agent in making a wettable powder ; or (c) together with a liquid agronomically acceptable carrier and a dispersing agent in making a flowable ; or (d) in the form of a dust ; or (e) together with a binding agent in making a granular formulation ; or (f) together with an attractant agent in making a bait.

12. The use of a compound or salt as defined in any one of claims 1 to 7, optionally in a composition also containing agronomically acceptable diluent or carrier, for controlling insects by contacting said insects with an insecticidally effective amount of the compound or salt.

13. A mechanical process for improving the commercial value and/or profitability of vendible crops from plants whose growth is affected or likely to be affected by insects comprising (1) charging to a container, fumigation device or mechanical dissemination device an insecticidal compound or salt as defined in any of claims 1 to 7, optionally in a mixture with agronomically acceptable diluent or carrier, (2) using the container, fumigator or mechanical dissemination device to apply the insecticidal compound or salt, in the form of granules, dust, smoke, vapour or surfactant-containing liquid preparation to growing plants or to a growth medium where the plants are growing or are to be grown, or to the insects themselves, (3) controlling the dose of the active ingredient during this application step so that the rate of application of active insecticidal compound is sufficient to combat the insects but is insufficient to cause an unacceptably adverse effect on the crop plants growing or to be grown in the treated area.

14. The use or process according to claim 12 or 13 wherein the insecticidal compound or salt is applied to growing plants or an area where plants are to be grown at a dosage rate of 10 grams to 10 kilograms per hectare.

15. The use or process according to claim 14 wherein the application rate is 100 grams to 5 kilograms per hectare.

16. The use or process according to claim 13 or 14 for killing insects from the order Lepidoptera or Coleoptera.

**Claims for the Contracting State : AT**

1. An insecticidal composition comprising, as an active insecticidal ingredient, a compound having the formula :

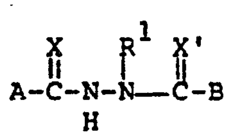

I

wherein

X and X′ are the same or different O, S or NR ;

$R^1$ is unsubstituted ($C_3$-$C_{10}$) branched alkyl or ($C_1$-$C_4$) straight chain alkyl substituted with one or two of the same or different ($C_3$-$C_8$)cycloalkyl ;

A is unsubstituted or substituted ($C_1$-$C_{10}$)alkyl having one to four of the same or different halo, cyano, nitro, hydroxy, ($C_1$-$C_4$)alkoxy, carboxy, ($C_1$-$C_4$)alkoxy-carbonyl, ($C_1$-$C_4$)-alkanoyloxy, $NH_2$, NHZ or NZZ′ ;

unsubstituted or substituted ($C_3$-$C_8$)cycloalkyl or ($C_3$-$C_8$)cycloalkyl-($C_1$-$C_4$)alkyl having one to four of the same or different halo, cyano, nitro, hydroxy, ($C_1$-$C_4$)alkyl, halo($C_1$-$C_4$)alkyl, ($C_1$-$C_4$)-alkoxy, halo($C_1$-$C_4$)alkoxy, carboxy, ($C_1$-$C_4$)alkanoyl, ($C_1$-$C_4$)alkoxy-carbonyl, ($C_1$-$C_4$)altianoyloxy, $NH_2$, NHZ or NZZ′ ;

unsubstituted or substituted ($C_2$-$C_8$)alkenyl or unsubstituted or substituted ($C_3$-$C_8$)-alkadienyl having one to four of the same or different halo, cyano, nitro, hydroxy, ($C_1$-$C_4$)alkyl, ($C_3$-$C_6$)cycloalkyl, halo-($C_1$-$C_4$)alkyl, ($C_1$-$C_4$)alkoxy, halo($C_1$-$C_4$)-alkoxy, carboxy, ($C_1$-$C_4$)alkoxy-carbonyl, ($C_1$-$C_4$)alkanoyloxy, $NH_2$, NHZ or NZZ′ ;

unsubstituted or substituted ($C_3$-$C_8$)cycloalkenyl or unsubstituted or substituted cycloalkadienyl having one to four of the same or different halo, cyano, nitro, hydroxy, ($C_1$-$C_4$)alkyl, halo($C_1$-$C_4$)-alkyl, ($C_1$-$C_4$)alkoxy, halo($C_1$-$C_4$)alkoxy, carboxy, ($C_1$-$C_4$)alkoxy-carbonyl, ($C_1$-$C_4$)-alkanoyloxy, $NH_2$, NHZ or NZZ′ ;

unsubstituted or substituted ($C_2$-$C_8$)alkynyl having one to four of the same or different halo, cyano, nitro, hydroxy, ($C_1$-$C_4$)alkyl, halo($C_1$-$C_4$)alkyl, ($C_1$-$C_4$)alkoxy, halo-($C_1$-$C_4$)alkoxy, carboxy, ($C_1$-$C_4$)-alkoxy-carbonyl, ($C_1$-$C_4$)alkanoyloxy, $NH_2$, NHZ or NZZ′ ; or

phenalkyl having one to four carbon atoms in the alkyl group and the phenyl ring is unsubstituted or substituted with one to three of the same or different halo, cyano, nitro, hydroxy, ($C_1$-$C_4$)alkyl, halo($C_1$-

$C_4$)-alkyl, cyano($C_1$-$C_4$)alkyl, ($C_1$-$C_4$)alkoxy, halo($C_1$-$C_4$)alkoxy, carboxy, ($C_1$-$C_4$)-alkoxy-carbonyl, ($C_1$-$C_4$)alkanoyloxy, ($C_2$-$C_6$)alkenyl, halo($C_2$-$C_6$)alkenyl, ($C_2$-$C_6$)alkynyl, $NH_2$, NHZ or NZZ' ;

B is unsubstituted or substituted naphthyl where the substituents can be from one to three of the same or different halo ; cyano ; nitro ; hydroxy ; ($C_1$-$C_4$)alkoxy ; ($C_1$-$C_4$)-alkyl ; carboxy ; ($C_1$-$C_4$)alkoxy-carbonyl ; ($C_1$-$C_4$)alkanoyloxy ; $NH_2$, NHZ or NZZ' ;

unsubstituted or substituted phenyl where the substituents can be from one to five of the same or different halo ; nitro ; cyano ; hydroxy ; ($C_1$-$C_6$)alkyl ; halo($C_1$-$C_6$)alkyl ; cyano($C_1$-$C_6$)alkyl ; ($C_1$-$C_6$)alkoxy ; halo-($C_1$-$C_6$)alkoxy ; alkoxyalkyl having independently 1 to 6 carbon atoms in each alkyl group ; alkoxyalkoxy having independently 1 to 6 carbon atoms in each alkyl group ; $-OCO_2R$ group ; ($C_2$-$C_6$)alkenyl optionally substituted with halo, cyano, ($C_1$-$C_4$)alkyl, ($C_1$-$C_4$)alkoxy, halo($C_1$-$C_4$)-alkoxy or ($C_1$-$C_4$)alkylthio ; $-RCO_2R'$ group ; $-COR$ group ; halo($C_1$-$C_6$)alkyl-carbonyl ; cyano($C_1$-$C_6$)alkyl-carbonyl ; nitro($C_1$-$C_6$)-alkyl-carbonyl ; $-CO_2R$ group ; halo($C_1$-$C_6$)-alkoxy-carbonyl ; $-OCOR$ group ; $-NRR'$ group ; amino substituted with hydroxy, ($C_1$-$C_4$)-alkoxy or ($C_1$-$C_4$)alkylthio groups ; phenylamino ; diphenylamino ; $-CONRR'$ group; $-OCONRR'$ group ; $-NRCOR'$ group ; $-NRCO_2R'$ group ; $-N(COR)COR'$ group ; $-OCONRCOR'$ group; sulfhydryl ; halothio ; ($C_1$-$C_6$)alkylthio ; halo($C_1$-$C_6$)alkyltio ; $-SOR$ group ; $-SO_2R$ group ; phenylsulfonyl; $-OSO_2R$ group ; halo-($C_1$-$C_6$)alkilsulonyloxy ; $-SO_2NRR'$ group ; $-NRSOR'$ group ; $-NRSO_2R'$ group ; $-CSR$ group ; $-CS_2R$ group ; $-NRCSR'$ group ; $-SCOR$ group ; unsubstituted or substituted phenyl having one to three of the same or different halo, cyano, nitro, hydroxy ($C_1$-$C_4$)alkyl, ($C_1$-$C_4$)alkoxy, carboxy, ($C_1$-$C_4$)-alkoxy-carbonyl, ($C_1$-$C_4$)alkanoyloxy, $NH_2$ group ; NHZ group ; or NZZ' group ; phenoxy where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy, $NH_2$ group ; NHZ group ; or NZZ' group ; phenylthio where the phenyl ring is unsubstituted or substituted with one to three of the same or different halo, cyano, nitro, hydroxy, ($C_1$-$C_4$)alkyl, ($C_1$-$C_4$)-alkoxy, carboxy, ($C_1$-$C_4$)alkoxy-carbonyl, ($C_1$-$C_4$)alkanoyloxy, $NH_2$ group ; NHZ group ; or NZZ' group ; or when two adjacent positions on the phenyl ring are substituted with alkoxy groups, these groups may be joined to form, together with the carbon atoms to which they are both attached, a 5 or 6 membered dioxolano or dioxano heterocyclic ring; where R, R' and R" are hydrogen or ($C_1$-$C_6$)alkyl ; Z and Z' are ($C_1$-$C_4$)alkyl ; "amino" means NRR' ; or an agronomically acceptable salt thereof, together with agronomically acceptable diluent or carrier.

2. A composition according to claim 1 wherein

X and X' are O or S ; and/or

$R^1$ is unsubstituted ($C_3$-$C_8$) branched alkyl or ($C_1$-$C_4$) straight chain alkyl substituted with one or two of the same or different ($C_3$-$C_4$)cycloalkyl ; and/or

A is unsubstituted or substituted ($C_1$-$C_6$)alkyl having one to three of the same or different halo, cyano, nitro, ($C_1$-$C_4$)-alkoxy, carboxy, ($C_1$-$C_4$)alkoxy-carbonyl, ($C_1$-$C_4$)alkanoyloxy, $NH_2$, NHZ or NZZ' ;

($C_3$-$C_6$)cycloalkyl ;

($C_2$-$C_6$) unsubstituted or substituted alkenyl having one to three of the same or different halo, ($C_1$-$C_4$-)alkyl, halo($C_1$-$C_4$)-alkyl, ($C_1$-$C_4$)alkoxy, or halo($C_1$-$C_4$)alkoxy ;

($C_3$-$C_6$)cycloalkenyl ; or

phenalkyl having one or two carbon atoms in the alkyl group and the phenyl ring is unsubstituted or substituted with one or two of the same or different halo, nitro or ($C_1$-$C_4$)alkyl ; and/or

B is unsubstituted naphthyl ; or

unsubstituted or substituted phenyl where the substituents can be from one to three of the same of different halo ; nitro ; cyano ; ($C_1$-$C_4$)alkyl ; halo($C_1$-$C_4$)alkyl ; cyano($C_1$-$C_4$)alkyl ; ($C_1$-$C_4$)alkoxy ; alkoxyalkyl having independently 1 to 4 carbon atoms in each alkyl group ; $-COD^4$ group ; carboxy ; ($C_1$-$C_4$)alkoxy-carbonyl; ($C_1$-$C_4$)-alkanoyloxy ; $NH_2$ ; NHZ ; NZZ' ; ($C_1$-$C_4$)alkylthio ; $-CSD^4$ group ; $-CS_2D^4$ group ; $-SCOD^4$ group; unsubstituted or substituted phenyl having one to two of the same or different halo, nitro, ($C_1$-$C_4$)alkyl, ($C_1$-$C_4$)alkoxy, carboxy, ($C_1$-$C_4$)-alkoxy-carbonyl, ($C_1$-$C_4$)alkanoyloxy, $NH_2$, NHZ or NZZ' ; phenoxy where the phenyl ring is unsubstituted or substituted with one or two of the same or different halo, nitro, ($C_1$-$C_4$)alkyl, ($C_1$-$C_4$)alkoxy, carboxy, ($C_1$-$C_4$)alkoxy-carbonyl, ($C_1$-$C_4$)alkanoyloxy, $NH_2$, NHZ or NZZ' ; or when two adjacent positions on the phenyl ring are substituted with alkoxy groups, these groups may be joined to form, together with the carbon atoms to which they are attached, a 5- or 6-membered dioxolano or dioxano heterocyclic ring ;

where $D^4$ is hydrogen or ($C_1$-$C_4$)alkyl ; Z and Z' are ($C_1$-$C_4$)alkyl.

3 A composition according to claim 2 wherein

X and X' are O or S ; and/or

$R^1$ is branched ($C_3$-$C_8$)alkyl ; and/or

A is unsubstituted or substituted ($C_1$-$C_6$)alkyl having one to three of the same or different halo, cyano, nitro, carboxy or ($C_1$-$C_4$)alkoxy-carbonyl ;

cyclohexyl ;
cyclohexenyl ;
unsubstituted or substituted ($C_2$-$C_6$)alkenyl having one to three of the same or different halo or ($C_1$-$C_4$)alkyl ; or benzyl where the phenyl ring is unsubstituted or substituted with one or two of the same or different halo, methyl or ethyl ; and/or
B is unsubstituted naphthyl ;
unsubstituted or substituted phenyl having one to three of the same or different halo ; nitro ; cyano ; ($C_1$-$C_4$)alkyl ; halo($C_1$-$C_4$)-alkyl ; cyano($C_1$-$C_4$)alkyl ; ($C_1$-$C_4$)alkoxy ; $-COD^4$ group ; ($C_1$-$C_4$)alkoxy-carbonyl ; ($C_1$-$C_4$)alkanoyloxy ; or unsubstituted or substituted phenyl having one or two of the same or different halo, nitro, ($C_1$-$C_4$)-alkyl, ($C_1$-$C_4$)alkoxy, carboxy, ($C_1$-$C_4$)-alkoxy-carbonyl, ($C_1$-$C_4$)alkanoyloxy, $NH_2$, NHZ or NZZ′ ; where $D^4$ is hydrogen or ($C_1$-$C_4$)alkyl ; Z and Z′ are ($C_1$-$C_4$)alkyl

4. A composition according to claim 3 wherein
X and X′ are O ; and/or
$R^1$ is branched ($C_4$-$C_7$)alkyl ; and/or
A is unsubstituted or substituted ($C_1$-$C_6$)alkyl having one to two of the same or different halo ;
cyclohexyl ;
unsubstituted or substituted ($C_2$-$C_6$)alkenyl having one to three of the same of different halo, methyl or ethyl ;
cyclohexenyl ; or
benzyl ; and/or
B is unsubstituted or substituted phenyl where the substituents can be from one to three of the same or different halo, ($C_1$-$C_4$)alkyl, ($C_1$-$C_4$)alkoxy, or halo-($C_1$-$C_4$)alkyl

5. A composition according to claim 4 wherein
X and X′ are O ; and/or
$R^1$ is t-butyl, neopentyl (2,2-dimethylpropyl) or 1,2,2-trimethylpropyl ; and/or
A is ($C_3$-$C_5$) unsubstituted alkyl ;
cyclohexyl ;
($C_2$-$C_4$) unsubstituted or substituted straight chain alkenyl having one to three of the same or different chloro, bromo or methyl ;
cyclohexenyl ; or
benzyl ; and/or
B is unsubstituted or substituted phenyl where the substituents can be one or two of the same or different chloro, fluoro, bromo, iodo, methyl, ethyl, methoxy or trifluoromethyl.

6. A composition according to claim 1 wherein
X and X′ are O ;
$R^1$ is t-butyl ; and
B is phenyl and A is cyclohexyl, n-butyl, n-propyl, benzyl, 1-cyclohexenyl, dichloromethyl or trichloromethyl; or
B is 4-chlorophenyl and A is n-propyl or t-butyl ; or
B is 3-methylphenyl and A is norbornyl, cyclohex-3-enyl, 1-butenyl, 3-butenyl, cis-3,4-dihydroxycyclohexyl, 2,2-dimethyl-3a,4,5,6,7,7a-hexahydrobenzo[d]-dioxalano-5-yl, 3-acetyl-2,2-dimethyl-cyclobutylmethyl or 2-methylcyclohexyl di-2,5-enyl ; or
B is 3,5-dimethylphenyl and A is isopropyl or 1-methylethenyl.

7. A composition as claimed in claim 2 wherein B is unsubstituted phenyl, 4-chlorophenyl, 3-substituted phenyl, 2-substituted phenyl or disubstituted phenyl.

8. A composition according to any preceding claim wherein said insecticidal ingredient is present at from 0.0001 to 99% by weight of the composition.

9. A composition according to claim 8 wherein said insecticidal ingredient is present at from 0.001 to 99% by weight of the composition.

10. A composition according to claim 8 wherein said insecticidal ingredient is present at from 0.01 to 99% by weight of the composition.

11. An insecticidal composition according to any preceding claim (a) additionally containing an emulsifying agent, said composition being in the form of an emulsifiable concentrate ; or (b) additionally containing a dispersing agent, said composition being in the form of a wettable powder ; or (c) containing a liquid agronomically acceptable carrier and a dispersing agent, said composition being in the form of a flowable ; or (d) in the form of a dust ; or (e) additionally containing a binding agent, said composition being in the form of a granule ; or (f) additionally containing an attractant agent, said composition being in the form of a bait.

12. A method of controlling insects which comprises contacting said insects with an insecticidally effective

amount of insecticidal ingredient as defined in any one of claims 1 to 6, optionally in a composition according to any preceding claim.

13. A method according to claim 12 wherein the insecticidal ingredient is applied to growing plants or an area where plants are to be grown at a dosage rate of 10 grams to 10 kilograms per hectare.

14. A method according to claim 13 wherein the application rate is 100 grams to 5 kilograms per hectare.

15. A method according to claim 12 or 13 for killing insects from the order Lepidoptera or Coleoptera.

## Ansprüche

**Patentansprüche für die Vertragsstaaten : BE, CH, DE, FR, GB, GR, IT, LI, LU, NL, SE.**

1. Insektizide Verbindung mit der Formel

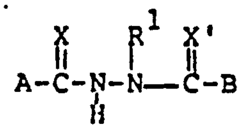

(I),

wobei

X und X' gleich oder unterschiedlich die Bedeutung O, S oder NR haben ;

$R^1$ bedeutet :

unsubstituiertes verzweigtes $(C_3-C_{10})$-Alkyl oder geradkettiges $(C_1-C_4)$-Alkyl, gleich oder unterschiedlich substituiert mit eins oder zwei $(C_3-C_6)$-Cycloalkyl ;

A bedeutet :

unsubstituiertes oder substituiertes $(C_1-C_{10})$-Alkyl mit gleich oder unterschiedlich eins bis vier aus Halo, Cyano, Nitro, Hydroxy, $(C_1-C_4)$-Alkoxy, Carboxy, $(C_1-C_4)$-Alkoxycarbonyl, $(C_1-C_4)$-Alkanoyloxy, $NH_2$, NHZ oder NZZ' ;

unsubstituiertes oder substituiertes $(C_3-C_8)$-Cycloalkyl oder $(C_3-C_8)$-Cycloalkyl-$(C_1-C_4)$-alkyl mit gleich oder unterschiedlich eins bis vier aus Halo, Cyano, Nitro, Hydroxy, $(C_1-C_4)$-Alkyl, Halo-$(C_1-C_4)$-alkyl, $(C_1-C_4)$-Alkoxy, Halo-$(C_1-C_4)$-alkoxy, Carboxy, $(C_1-C_4)$-Alkanoyl, $(C_1-C_4)$-Alkoxycarbonyl, $(C_1-C_4)$-Alkanoyloxy, $NH_2$, NHZ oder NZZ' ;

unsubstituiertes oder substituiertes $(C_2-C_8)$-Alkenyl oder unsubstituiertes oder substituiertes $(C_3-C_8)$-Alkadienyl mit gleich oder unterschiedlich eins bis vier aus Halo, Cyano, Nitro, Hydroxy, $(C_1-C_4)$-Alkyl, $(C_3-C_6)$-Cycloalkyl, Halo-$(C_1-C_4)$-alkyl, $(C_1-C_4)$-Alkoxy, Halo-$(C_1-C_4)$-Alkoxy, Carboxy, $(C_1-C_4)$-Alkoxycarbonyl, $(C_1-C_4)$-Alkanoyloxy, $NH_2$, NHZ oder NZZ' ;

unsubstituiertes oder substituiertes $(C_3-C_8)$-Cycloalkenyl oder unsubstituiertes oder substituiertes $(C_6-C_8)$-Cycloalkadienyl mit gleich oder unterschiedlich eins bis vier aus Halo, Cyano, Nitro, Hydroxy, $(C_1-C_4)$-Alkyl, Halo-$(C_1-C_4)$-alkyl, $(C_1-C_4)$-Alkoxy, Halo-$(C_1-C_4)$-alkoxy, Carboxy, $(C_1-C_4)$-Alkoxycarbonyl, $(C_1-C_4)$-Alkanoyloxy, $NH_2$, NHZ oder NZZ' ;

unsubstituiertes oder substituiertes $(C_2-C_8)$-Alkynyl mit gleich oder unterschiedlich eins bis vier aus Halo, Cyano, Nitro, Hydroxy, $(C_1-C_4)$-Alkyl, Halo-$(C_1-C_4)$-Alkyl, $(C_1-C_4)$-Alkoxy, Halo-$(C_1-C_4)$-alkoxy, Carboxy, $(C_1-C_4)$-Alkoxycarbonyl, $(C_1-C_4)$-Alkanyloxy, $NH_2$, NHZ oder NZZ' oder ;

Phenalkyl mit eins bis vier Kohlenstoffatomen in der Alkylgruppe, wobei der Phenylring unsubstituiert ist oder gleich oder unterschiedlich substituiert ist mit eins bis drei aus Halo, Cyano, Nitro, Hydroxy, $(C_1-C_4)$-Alkyl, Halo-$(C_1-C_4)$-alkyl, Cyano-$(C_1-C_4)$-alkyl, $(C_1-C_4)$-Alkoxy, Halo-$(C_1-C_4)$-alkoxy, Carboxy, $(C_1-C_4)$-Alkoxycarbonyl, $(C_1-C_4)$-Alkanoyloxy, $(C_2-C_6)$-Alkenyl, Halo-$(C_2-C_6)$-alkenyl, $(C_2-C_6)$-Alkynyl, $NH_2$, NHZ oder NZZ' ;

B bedeutet :

unsubstituiertes oder substituiertes Naphthyl, wobei die Substituenten gleich oder unterschiedlich eins bis drei aus Halo, Cyano, Nitro, Hydroxy, $(C_1-C_4)$-Alkoxy, $(C_1-C_4)$-Alkyl, Carboxy, $(C_1-C_4)$-Alkoxycarbonyl, $(C_1-C_4)$-Alkanoyloxy, $NH_2$, NHZ oder NZZ' sein können ;

unsubstituiertes oder substituiertes Phenyl, wobei die Substituenten gleich oder unterschiedlich sein können eins bis fünf aus Halo ; Nitro ; Cyano ; Hydroxy ; $(C_1-C_6)$-Alkyl ; Halo-$(C_1-C_6)$-alkyl ; Cyano-$(C_1-C_6)$-Alkyl ; $(C_1-C_6)$-Alkoxy ; Halo-$(C_1-C_6)$-alkoxy ; Alkoxyalkyl mit unabhängig eins bis sechs Kohlenstoffatomen in jeder Alkylgruppe ; Alkoxyalkoxy mit unabhängig eins bis sechs Kohlenstoffatomen in

jeder Alkylgruppe ; $-OCO_2R$-Gruppe ; $(C_2$-$C_6)$-Alkenyl, wahlweise substituiert mit Halo, Cyano, $(C_1$-$C_4)$-Alkyl, $(C_1$-$C_4)$-Alkoxy, Halo-$(C_1$-$C_4)$-alkoxy oder $(C_1$-$C_4)$-Alkylthio ; $-RCO_2R'$-Gruppe ; $-COR-$ Gruppe ; Halo-$(C_1$-$C_6)$-alkylcarbonyl ; Cyano-$(C_1$-$C_6)$-alkylcarbonyl ; Nitro-$(C_1$-$C_6)$-akylcarbonyl ; $-CO_2R-$Gruppe ; Halo-$(C_1$-$C_6)$-Alkoxycarbonyl ; $-OCOR-$ Gruppe, $-NRR'-$ Gruppe ; Amino substituiert mit Hydroxy, $(C_1$-$C_4)$-Alkoxy oder $(C_1$-$C_4)$-Alkylthio-Gruppen ; Phenylamino ; Diphenylamino ; $-CONRR'-$ Gruppe ; $-OCONRR'-$ Gruppe ; $-NRCOR'-$ Gruppe ; $-NRCO_2R'$Gruppe ; $-N(COR)COR'-$ Gruppe ; $-OCONRCOR'-$ Gruppe ; Sulfhydryl ; halothio ; $(C_1$-$C_6)$-Alkylthio ; Halo-$(C_1$-$C_6)$-alkylthio ; $-SOR-$ Gruppe ; $-SO_2R-$ Gruppe ; Phenylsulfonyl ; $-OSO_2R-$ Gruppe ; Halo-$(C_1$-$C_6)$-Alkylsulfonyloxy ; $-SO_2NRR'-$ Gruppe ; $-NRSOR'-$ Gruppe ; $-NRSO_2R'-$ Gruppe ; $-CSR-$ Gruppe ; $-CS_2R-$ Gruppe ; $-NRCSR'-$ Gruppe ; $-SCOR-$ Gruppe ; unsubstituiertes oder substituiertes Phenyl mit gleich oder unterschiedlich eins bis drei aus Halo, Cyano, Nitro, Hydroxy, $(C_1$-$C_4)$-Alkyl, $(C_1$-$C_4)$-Alkoxy, Carboxy, $(C_1$-$C_4)$-Alkoxycarbonyl, $(C_1$-$C_4)$-Alkanoyloxy, $NH_2$-Gruppe ; NHZ-Gruppe oder NZZ'-Gruppe ; Phenoxy, wobei der Phenylring unsubstiuiert ist oder gleich oder unterschiedlich substituiert ist mit eins bis drei aus Halo, Cyano, Nitro, Hydroxy, $(C_1$-$C_4)$-Alkyl, $(C_1$-$C_4)$-Alkoxy, Carboxy, $(C_1$-$C_4)$-Alkoxycarbonyl, $(C_1$-$C_4)$-Alkanoyloxy, $NH_2$-Gruppe ; NHZ-Gruppe ; oder NZZ'-Gruppe ; Phenylthio, wobei der Phenylring unsubstituiert ist oder gleich oder unterschiedlich substituiert ist mit eins bis drei aus Halo, Cyano, Nitro, Hydroxy $(C_1$-$C_4)$-Alkyl, $(C_1$-$C_4)$-Alkoxy, Carboxy, $(C_1$-$C_4)$-Alkoxycarbonyl, $(C_1$-$C_4)$-Alkanoyloxy, oder $NH_2$-Gruppe ; NHZ-Gruppe ; oder NZZ'-Gruppe ; oder wenn zwei benachbarte Positionen am Phenylring mit Alkoxygruppen substituiert sind, diese Gruppen verbunden sein können, um zusammen mit den Kohlenstoffatomen, an denen sie beide hängen, einen 5-oder 6-gliedrigen heterocyclischen Dioxolano- oder Dioxanoring zu bilden ;

wobei R, R' und R" Wasserstoff oder $(C_1$ -$C_6)$-Alkyl bedeuten ; Z und Z' $(C_1$ -$C_4)$ -Alkyl bedeuten ; wobei "Amino" NRR' bedeutet ; und

Landwirtschaftlich vertretbare Salze davon.

2. Verbindung nach Anspruch 1, wobei

X und X' die Bedeutung O oder S haben ; und/oder

$R^1$ bedeutet :

unsubstituiertes verzweigtes $(C_3$-$C_8)$-Alkyl oder geradkettiges $(C_1$-$C_4)$-Alkyl, gleich oder unterschiedlich substituiert mit eins oder zwei $(C_3$-$C_4)$-Cycloalkyl ; und/oder

A bedeutet :

unsubstituiertes oder unsubstituiertes $(C_1$-$C_6)$-Alkyl mit gleich oder unterschiedlich eins bis drei aus Halo, Cyano, Nitro, $(C_1$-$C_4)$-Alkoxy, Carboxy, $(C_1$-$C_4)$-Alkoxycarbonyl, $(C_1$-$C_4)$-Alkanoyloxy, $NH_2$, NHZ oder NZZ' ;

$(C_3$-$C_6)$-Cycloalkyl ;

unsubstituiertes oder substituiertes $(C_2$-$C_6)$-Alkenyl mit gleich oder unterschiedlich eins bis drei aus Halo, $(C_1$-$C_4)$-Alkyl, Halo-$(C_1$-$C_4)$-Alkyl, $(C_1$-$C_4)$-Alkoxy oder Halo-$(C_1$-$C_4)$-alkoxy ;

$(C_3$-$C_6)$-Cycloalkenyl ; oder

Phenalkyl mit einem oder zwei Kohlenstoffatomen in der Alkylgruppe, wobei der Phenylring unsubstituiert ist oder gleich oder unterschiedlich substituiert ist mit eins oder zwei aus Halo, Nitro oder $(C_1$-$C_4)$-Alkyl ; und/oder

B unsubstituiertes Naphthyl bedeutet ; oder

unsubstituiertes oder substituiertes Phenyl, wobei die Substituenten gleich oder unterschiedlich sein können eins bis drei aus Halo ; Nitro ; Cyano ; $(C_1$-$C_4)$-Alkyl ; Halo-$(C_1$-$C_4)$-alkyl ; Cyano-$(C_1$-$C_4)$-alkyl ; $(C_1$-$C_4)$-Alkoxy ; Alkoxyalkyl mit unabhängig eins bis vier Kohlenstoffatomen in jeder Alkylgruppe ; $-COD^4-$ Gruppe ; Carboxy ; $(C_1$-$C_4)$-Alkoxycarbonyl ; $(C_1$-$C_4)$-Alkanoyloxy ; $NH_2$ ; NHZ ; NZZ' ; $(C_1$-$C_4)$-Alkylthio ; $-CSD^4-$ Gruppe ; $-CS_2D^4-$ Gruppe ; $-SCOD^4-$ Gruppe ; unsubstituiertes Phenyl mit gleich oder unterschiedlich eins bis zwei aus Halo, Nitro $(C_1$-$C_4)$-Alkyl, $(C_1$-$C_4)$-Alkoxy, Carboxy, $(C_1$-$C_4)$-Alkoxycarbonyl, $(C_1$-$C_4)$-Alkanoyloxy, $NH_2$, NHZ oder NZZ' sein können ; Phenoxy, wobei der Phenylring unsubstituiert ist oder gleich oder unterschiedlich substituiert ist mit eins oder zwei aus Halo, Nitro, $(C_1$-$C_4)$-Alkyl, $(C_1$-$C_4)$-Alkoxy, Carboxy, $(C_1$-$C_4)$-Alkoxycarbonyl, $(C_1$-$C_4)$-Alkanoyloxy, $NH_2$, NHZ oder NZZ' ; oder wenn zwei benachbarte Positionen am Phenylring mit Alkoxy-Gruppen substituiert sind, diese Gruppen verbunden sein können, um zusammen mit den Kohlenstoffatomen, an denen sie hängen, ein 5- oder 6-gliedrigen heterocyclischen Dioxolan- oder Dioxanring zu bilden ;

wobei $D^4$ Wasserstoff oder $(C_1$-$C_4)$-Alkyl bedeutet ; Z und Z' $(C_1$-$C_4)$-Alkyl bedeuten ; und

landwirtschaftlich vertretbare Salze davon.

3. Verbindung nach Anspruch 2, wobei

X und X' die Bedeutung O oder S haben ; und/oder

$R^1$ verzweigtes $(C_3$-$C_8)$-Alkyl bedeutet ; und/oder

A bedeutet:

unsubstituiertes oder substituiertes ($C_1$-$C_6$)-Alkyl mit gleich unterschiedlich eins bis drei aus Halo, Nitro, Cyano, Carboxy oder ($C_1$-$C_4$)-Alkoxycarbonyl ;

Cyclohexyl ;

Cyclohexenyl ;

unsubstituiertes oder substituiertes ($C_2$-$C_6$)-Alkenyl mit gleich oder unterschiedlich eins bis drei aus Halo oder ($C_1$-$C_4$)-Alkyl ; oder

Benzyl, wobei der Phenylring unsubstituiert ist oder gleich oder unterschiedlich substituiert ist mit eins oder zwei aus Halo, Methyl oder Ethyl ; und/oder

B bedeutet:

unsubstituiertes Naphthyl ;

unsubstituiertes oder substituiertes Phenyl mit gleich oder unterschiedlich eins bis zwei Halo ; Nitro ; Cyano ; ($C_1$-$C_4$)-Alkyl ; Halo-($C_1$-$C_4$)-Alkyl ; Cyano-($C_1$-$C_4$)-alkyl ; ($C_1$-$C_4$)-Alkoxy ; $-COD^4-$ Gruppe ; ($C_1$-$C_4$)Alkoxycarbonyl ; ($C_1$-$C_4$)-Alkanoyloxy ; oder unsubstituiertes oder substituiertes Phenyl mit gleich oder unterschiedlich eins oder zwei aus Halo, Nitro, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, $NH_2$, NHZ oder NZZ' ; wobei $D^4$ die Bedeutung Wasserstoff oder ($C_1$-$C_4$)-Alkyl hat ; Z und Z' die Bedeutung ($C_1$-$C_4$)-Alkyl haben ; und

landwirtschaftlich vertretbare Salze davon.

4. Verbindung nach Anspruch 3, wobei

X und X' die Bedeutung O haben ; und/oder

$R^1$ verzweigtes ($C_4$-$C_7$)-Alkyl bedeutet ; und/oder

A bedeutet:

unsubstituiertes oder substituiertes ($C_1$-$C_6$)-Alkyl mit gleich oder unterschiedlich eins bis zwei Halo ;

Cyclohexyl ;

unsubstituiertes oder substituiertes ($C_2$-$C_6$)-Alkenyl mit gleich oder unterschiedlich eins bis drei aus Halo, Methyl oder Ethyl ;

Cyclohexenyl ; oder

Benzyl ; und/oder

B bedeutet:

unsubstituiertes oder substituiertes Phenyl, wobei die Substituenten gleich oder unterschiedlich eins oder drei aus Halo, ($C_1$-$C_4$)-Alkyl, ($C_1$-$C_4$)-Alkoxy oder Halo-($C_1$-$C_4$)-alkyl sein können ; und

landwirtschaftlich vertretbare Salze davon

5. Verbindung nach Anspruch 4, wobei

X und X' die Bedeutung O haben ; und/oder

$R^1$ die Bedeutung t-Butyl, Neopentyl (2,2-Dimethylpropyl) oder 1,2,2-Trimethylpropyl hat ; und/oder

A bedeutet:

unsubstituiertes ($C_3$-$C_5$)-Alkyl ;

Cyclohexyl ;

($C_2$-$C_4$) unsubstituiertes oder substituiertes geradkettiges Alkenyl mit gleich oder unterschiedlich ein bis zwei aus Chlor, Brom oder Methyl ;

Cyclohexenyl ; oder

Benzyl ; und/oder

B bedeutet:

unsubstituiertes oder substituiertes Phenyl, wobei die Substituenten gleich oder unterschiedlich eins oder zwei aus Chlor, Fluor, Brom, Iod, Methyl, Ethyl, Methoxy oder Trifluormethyl sein können ; und landwirtschaftlich vertretbare Salze davon.

6. Verbindung nach Anspruch 1, wobei

X und X' die Bedeutung O haben ;

$R^1$ die Bedeutung t-Butyl hat ; und

B die Bedeutung Phenyl hat und A die Bedeutung Cyclohexyl, n-Butyl, n-Propyl, Benzyl, 1-Cyclo-hexenyl, Dichlormethyl oder Trichlormethyl hat ; oder

B die Bedeutung 4-Chlorphenyl und A die Bedeutung nPropyl oder t-Butyl hat ; oder

B die Bedeutung 3-Methylphenyl hat und A die Bedeutung Norbonyl, Cyclohex-3-enyl, 1-Butenyl, 3-Butenyl, Cis-3,4-Dihydroxycyclohexyl, 2,2-Dimethyl-3a,4,5,6,7,7a-hexahydrobenzo[d]-dioxalano-5-yl, 3Acetyl-2,2-dimethylcyclobutyl-methyl oder 2-Methylcyclohexyl-di-2,5-enyl hat ; oder

B die Bedeutung 3,5-Dimethylphenyl hat und A die Bedeutung Isopropyl oder 1-Methylethenyl hat

7. Verbindung nach Anspruch 2, wobei B die Bedeutung unsubstituiertes Phenyl, 4-Chlorphenyl, 3-substituiertes Phenyl, 2-substituiertes Phenyl oder disubstituiertes Phenyl hat.

8. Insektizide Zusammensetzung, enthaltend eine insektizide Verbindung wie in einem der vorhergehenden Ansprüche beansprucht zusammen mit landwirtschaftlich vertretbarem Verdünnungs- oder Trägerstoff

9. Zusammensetzung nach Anspruch 8, wobei die insektizide Verbindung mit 0,0001 bis 99 Gew.-% der Zusammensetzung anwesend ist

10. Zusammensetzung nach Anspruch 8, wobei die insektizide Verbindung mit 0,001 bis 99 Gew.-% der Zusammensetzung ist.

11. Zusammensetzung nach Anspruch 8, wobei die insektizide Verbindung mit 0,01 bis 99 Gew-% der Zusammensetzung vorhanden ist

12. Insektizide Zusammensetzung nach einem der Ansprüche 8 bis 11,

(a) zusätzlich enthaltend ein Emulgiermittel, wobei die Zusammensetzung in Form eines emulgierbaren Konzentrats vorliegt ; oder

(b) zusätzlich enthaltend ein Dispergiermittel, wobei die Zusammensetzung in Form eines benetzbaren Pulvers vorliegt ; oder

(c) enthaltend einen flüssigen landwirtschaftlich vertretbaren Trägerstoff und ein Dispergiermittel, wobei die Zusammensetzung in Form eines Fließbaren vorliegt ; oder

(d) in Form eines Staubes ; oder

(e) zusätzlich enthaltend ein Bindemittel, wobei die Zusammensetzung in Form eines Granulats vorliegt ; oder

(f) zusätzlich enthaltend einen Lockstoff, wobei die Zusammensetzung in Form eines Köders vorliegt

13. Verfahren zum Kontrollieren von Insekten, bei dem man die Insekten mit einer insektizid wirksamen Menge einer Verbindung gemäß einem der Ansprüche 1 bis 7 in Kontakt bringt, wahlweise in einer Zusammensetzung nach einem der Ansprüche 8 bis 12.

14. Verfahren nach Anspruch 13, wobei man die insektizide Verbindung wachsenden Pflanzen oder einem Gebiet zuführt, wo die Pflanzen gezogen werden, bei einer Dosisrate von 10 g bis 10 kg pro Hektar.

15. Verfahren nach Anspruch 14, wobei die Applikationsrate 100 g bis 5 kg pro Hektar beträgt

16. Verfahren nach Anspruch 13, 14 oder 15 zum Töten von Insekten der Ordnung Lepidoptera oder Coleoptera.

**Patentansprüche für den Vertragsstaat : AT**

1. Insektizide Zusammensetzung, enthaltend als insektiziden Wirkstoff eine Verbindung der Formel :

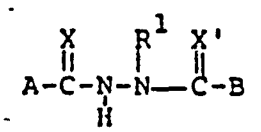

(I),

wobei

X und X′ gleich oder unterschiedlich die Bedeutung O, S oder NR haben ;

$R^1$ bedeutet :

unsubstituiertes verzweigtes $(C_3-C_{10})$-Alkyl oder geradkettiges $(C_1-C_4)$-Alkyl gleich oder unterschiedlich substituiert ist mit eins oder zwei $(C_3-C_6)$-Cycloalkyl ;

A bedeutet :

unsubstituiertes oder substituiertes $(C_1-C_{10})$-Alkyl mit gleich oder unterschiedlich eins bis vier aus Halo, Cyano, Nitro, Hydroxy, $(C_1-C_4)$-Alkoxy, Carboxy, $(C_1-C_4)$-Alkoxycarbonyl, $(C_1-C_4)$-Alkanoyloxy, $NH_2$, NHZ oder NZZ′ ;

unsubstituiertes oder substituiertes $(C_3-C_8)$-Cycloalkyl oder $(C_3-C_8)$-Cycloalkyl-$(C_1-C_4)$-alkyl mit gleich oder unterschiedlich eins bis vier aus Halo, Cyano, Nitro, Hydroxy, $(C_1-C_4)$-Alkyl, Halo-$(C_1-C_4)$-alkyl, $(C_1-C_4)$-Alkoxy, Halo-$(C_1-C_4)$-alkoxy, Carboxy, $(C_1-C_4)$-Alkanoyl, $(C_1-C_4)$-Alkoxycarbonyl, $(C_1-C_4)$-Alkanoyloxy, $NH_2$, NHZ oder NZZ′ ;

unsubstituiertes oder substituiertes $(C_2-C_6)$-Alkenyl oder unsubstituiertes oder substituiertes $(C_3-C_8)$-Alkadienyl mit gleich oder unterschiedlich eins bis vier aus Halo, Cyano, Nitro, Hydroxy, $(C_1-C_4)$-Alkyl, $(C_3-C_6)$-Cycloalkyl, Halo-$(C_1-C_4)$-alkyl, $(C_1-C_4)$-Alkoxy, Halo-$(C_1-C_4)$-Alkoxy, Carboxy, $(C_1-C_4)$-Alkoxycarbonyl, $(C_1-C_4)$-Alkanoyloxy, $NH_2$, NHZ oder NZZ′ ;

unsubstituiertes oder substituiertes $(C_3-C_8)$-Cycloalkenyl oder unsubstituiertes oder substituiertes $(C_6-C_8)$-Cycloalkadienyl mit gleich oder unterschiedlich eins bis vier aus Halo, Cyano, Nitro, Hydroxy, $(C_1-C_4)$-Alkyl, Halo-$(C_1-C_4)$-alkyl, $(C_1-C_4)$-Alkoxy, Halo-$(C_1-C_4)$-alkoxy, Carboxy, $(C_1-C_4)$-Alkoxycarbonyl,

($C_1$-$C_4$)-Alkanoyloxy, $NH_2$, NHZ oder NZZ' ;
unsubstituiertes oder substituiertes ($C_2$-$C_8$)-Alkynyl mit gleich oder unterschiedlich eins bis vier aus Halo, Cyano, Nitro, Hydroxy, ($C_1$-$C_4$)-Alkyl, Halo-($C_1$-$C_4$)-Alkyl, ($C_1$-$C_4$)-Alkoxy, Halo-($C_1$-$C_4$)-alkoxy, Carboxy, ($C_1$-$C_4$)-Alkoxycarbonyl, ($C_1$-$C_4$)-Alkanyloxy, $NH_2$, NHZ oder NZZ' oder ;
Phenalkyl mit eins bis vier Kohlenstoffatomen in der Alkylgruppe, wobei der Phenylring unsubstituiert ist oder gleich oder unterschiedlich substituiert ist mit eins bis drei aus Halo, Cyano, Nitro, Hydroxy, ($C_1$-$C_4$)-Alkyl, Halo-($C_1$-$C_4$)-alkyl, Cyano-($C_1$-$C_4$)-alkyl, ($C_1$-$C_4$)-Alkoxy, Halo-($C_1$-$C_4$)-alkoxy, Carboxy, ($C_1$-$C_4$)-Alkoxycarbonyl, ($C_1$-$C_4$)-Alkanoyloxy, ($C_2$-$C_6$)-Alkenyl, Halo-($C_2$-$C_6$)-alkenyl, ($C_2$-$C_6$)-Alkynyl, $NH_2$, NHZ oder NZZ' ;

B bedeutet :

unsubstituiertes oder substituiertes Naphthyl, wobei die Substituenten gleich oder unterschiedlich eins bis drei aus Halo, Cyano, Nitro, Hydroxy, ($C_1$-$C_4$)-Alkoxy, ($C_1$-$C_4$)-Alkyl, Carboxy, ($C_1$-$C_4$)-Alkoxycarbonyl, ($C_1$-$C_4$)-Alkanoyloxy, $NH_2$, NHZ oder NZZ' sein können ;
unsubstituiertes oder substituiertes Phenyl, wobei die Substituenten gleich oder unterschiedlich sein können eins bis fünf aus Halo ; Nitro ; Cyano ; Hydroxy ; ($C_1$-$C_6$)-Alkyl ; Halo-($C_1$-$C_6$)-alkyl ; Cyano($C_1$-$C_6$)-Alkyl ; ($C_1$-$C_6$)-Alkoxy ; Halo-($C_1$-$C_6$)-alkoxy ; Alkoxyalkyl mit unabhängig eins bis sechs Kohlenstoffatomen in jeder Alkylgruppe ; Alkoxyalkoxy mit unabhängig eins bis sechs Kohlenstoffatomen in jeder Alkylgruppe ; $-OCO_2R-$ Gruppe ; ($C_2$-$C_6$)-Alkenyl, wahlweise substituiert mit Halo, Cyano, ($C_1$-$C_4$)-Alkyl, ($C_1$-$C_4$)-Alkoxy, Halo-($C_1$-$C_4$)-alkoxy oder ($C_1$-$C_4$)-Alkylthio ; $-RCO_2R'-$ Gruppe ; $-COR-$ Gruppe ; Halo-($C_1$-$C_6$)-alkylcarbonyl ; Cyano-($C_1$-$C_6$)-alkylcarbonyl ; Nitro-($C_1$-$C_6$)-akylcarbonyl ; $-CO_2R-$ Gruppe ; Halo-($C_1$-$C_6$)-Alkoxycarbonyl ; $-OCOR-$ Gruppe, $-NRR'-$ Gruppe ; Amino substituiert mit Hydroxy, ($C_1$-$C_4$)-Alkoxy oder ($C_1$-$C_4$)-Alkylthio-Gruppen ; Phenylamino ; Diphenylamino ; $-CONRR'-$ Gruppe ; $-OCONRR'-$ Gruppe ; $-NRCOR'-$ Gruppe ; $-NRCO_2R'$Gruppe ; $-N(COR)COR'-$ Gruppe ; $-OCONRCOR'-$ Gruppe ; Sulfhydryl ; Halothio ; ($C_1$-$C_6$)-Alkylthio ; Halo-($C_1$-$C_6$)-alkylthio ; $-SOR-$ Gruppe ; $-SO_2R-$ Gruppe ; Phenylsulfonyl ; $-OSO_2R-$ Gruppe ; Halo-($C_1$-$C_6$)-Alkylsulfonyloxy ; $-SO_2NRR'-$ Gruppe ; $-NRSOR'-$ Gruppe ; $-NRSO_2R'-$ Gruppe ; $-CSR-$ Gruppe ; $-CS_2R-$ Gruppe ; $-NRCSR'-$ Gruppe ; $-SCOR-$ Gruppe ; unsubstituiertes oder substituiertes Phenyl mit gleich oder unterschiedlich eins bis drei aus Halo, Cyano, Nitro, Hydroxy, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, $NH_2$-Gruppe ; NHZ-Gruppe oder NZZ'-Gruppe ; Phenoxy, wobei der Phenylring unsubstiuiert ist oder gleich oder unterschiedlich substituiert ist mit eins bis drei aus Halo, Cyano, Nitro, Hydroxy, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, $NH_2$-Gruppe ; NHZ-Gruppe ; oder NZZ'-Gruppe ; Phenylthio, wobei der Phenylring unsubstituiert ist oder gleich oder unterschiedlich substituiert ist mit eins bis drei aus Halo, Cyano, Nitro, Hydroxy ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, oder $NH_2$-Gruppe ; NHZ-Gruppe ; oder NZZ'-Gruppe ; oder wenn zwei benachbarte Positionen am Phenylring mit Alkoxygruppen substituiert sind, diese Gruppen verbunden sein können, um zusammen mit den Kohlenstoffatomen, an denen sie beide hängen, einen 5- oder 6-gliedrigen hetrocyclischen Dioxolanoder Dioxanring zu bilden ;

wobei R, R' und R" Wasserstoff oder ($C_1$-$C_6$)-Alkyl bedeuten ; Z und Z' ($C_1$ -$C_4$) -Alkyl bedeuten ; wobei "Amino" NRR' bedeutet ; oder ein
landwirtschaftlich vertretbares Salz davon zusammen mit landwirtschaftlich vertretbarem Verdünnungs- oder Trägerstoff.

2. Zusammensetzung nach Anspruch I, wobei
X und X' die Bedeutung O oder S haben ; und/oder
$R^1$ bedeutet :
unsubstituiertes verzweigtes ($C_3$-$C_8$)-Alkyl oder geradkettiges ($C_1$-$C_4$)-Alkyl, gleich oder unterschiedlich substituiert mit eins oder zwei ($C_3$-$C_4$)-Cycloalkyl ; und/oder
A bedeutet :

unsubstituiertes oder unsubstituiertes ($C_1$-$C_6$)-Alkyl mit gleich oder unterschiedlich eins bis drei aus Halo, Cyano, Nitro, ($C_1$-$C_4$)-Alkoxy, Carboxy, ($C_1$-$C_4$)-Alkoxycarbonyl, ($C_1$-$C_4$)-Alkanoyloxy, $NH_2$, NHZ oder NZZ' ;
($C_3$-$C_6$)-Cycloalkyl ;
unsubstituiertes oder substituiertes ($C_2$-$C_6$)-Alkenyl mit gleich oder unterschiedlich eins bis drei aus Halo, ($C_1$-$C_4$)-Alkyl, Halo-($C_1$-$C_4$)-Alkyl, ($C_1$-$C_4$)-Alkoxy oder Halo-($C_1$-$C_4$)-alkoxy ;
($C_3$-$C_6$)-Cycloalkenyl ; oder
Phenalkyl mit einem oder zwei Kohlenstoffatomen in der Alkylgruppe, wobei der Phenylring unsubstituiert ist oder mit gleich oder unterschiedlich substituiert ist mit eins oder zwei aus Halo, Nitro oder ($C_1$-$C_4$)-Alkyl ; und/oder

B unsubstituiertes Naphthyl bedeutet ; oder

unsubstituiertes oder substituiertes Phenyl, wobei die Substituenten gleich oder unterschiedlich eins bis drei aus Halo ; Nitro ; Cyano ; ($C_1$-$C_4$)-Alkyl ; Halo-($C_1$-$C_4$)-alkyl ; Cyano-($C_1$-$C_4$)-alkyl ; ($C_1$-$C_4$)-Alkoxy ; Alkoxyalkyl mit unabhängig eins bis vier Kohlenstoffatomen in jeder Alkylgruppe ; $-COD^4-$ Gruppe ; Carboxy ; ($C_1$-$C_4$)-Alkoxycarbonyl ; ($C_1$-$C_4$)-Alkanoyloxy ; $NH_2$ ; NHZ ; NZZ′ ; ($C_1$-$C_4$)-Alkylthio ; $-CSD^4-$ Gruppe ; $-CS_2D^4-$ Gruppe ; $-SCOD^4-$ Gruppe ; unsubstituiertes Phenyl mit gleich oder unterschiedlich eins bis zwei aus Halo, Nitro ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, $NH_2$, NHZ oder NZZ′ sein können ; Phenoxy, wobei der Phenylring unsubstituiert ist oder gleich oder unterschiedlich substituiert ist mit eins oder zwei aus Halo, Nitro, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, $NH_2$, NHZ oder NZZ′ ; oder wenn zwei benachbarte Positionen am Phenylring mit Alkoxy-Gruppen substituiert sind, diese Gruppen verbunden sein können, um zusammen mit den Kohlenstoffatomen, an denen sie hängen, ein 5- oder 6-gliedrigen heterocyclischen Dioxolan- oder Dioxanring zu bilden ;

wobei $D^4$ Wasserstoff oder ($C_1$-$C_4$)-Alkyl bedeutet ; Z und Z′ ($C_1$-$C_4$)-Alkyl bedeuten.

3. Zusammensetzung nach Anspruch 2, wobei

X und X′ die Bedeutung O oder S haben ; und/oder

$R^1$ verzweigtes ($C_3$-$C_8$)-Alkyl bedeutet ; und/oder

A bedeutet :

unsubstituiertes oder substituiertes ($C_1$-$C_6$)-Alkyl mit gleich unterschiedlich eins bis drei aus Halo, Nitro, Cyano, Carboxy oder ($C_1$-$C_4$)-Alkoxycarbonyl ;

Cyclohexyl ;

Cyclohexenyl ;

unsubstituiertes oder substituiertes ($C_2$-$C_6$)-Alkenyl mit gleich oder unterschiedlich eins bis drei aus Halo oder ($C_1$-$C_4$)-Alkyl ; oder

Benzyl, wobei der Phenylring unsubstituiert ist oder gleich oder unterschiedlich substituiert ist mit eins oder zwei aus Halo, Methyl oder Ethyl ; und/oder

B bedeutet :

unsubstituiertes Naphthyl ;

unsubstituiertes oder substituiertes Phenyl mit gleich oder unterschiedlich eins bis zwei Halo ; Nitro ; Cyano ; ($C_1$-$C_4$)-Alkyl ; Halo-($C_1$-$C_4$)-Alkyl ; Cyano-($C_1$-$C_4$)-alkyl ; ($C_1$-$C_4$)-Alkoxy ; $-COD^4-$ Gruppe ; ($C_1$-$C_4$)Alkoxycarbonyl ; ($C_1$-$C_4$)-Alkanoyloxy ; oder unsubstituiertes oder substituiertes Phenyl mit gleich oder unterschiedlich eins oder zwei aus Halo, Nitro, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, $NH_2$, NHZ oder NZZ′ ; wobei $D^4$ die Bedeutung Wasserstoff oder ($C_1$-$C_4$)-Alkyl hat ; Z und Z′ die Bedeutung ($C_1$-$C_4$)-Alkyl haben.

4. Zusammensetzung nach Anspruch 3, wobei

X und X′ die Bedeutung O haben ; und/oder

$R^1$ verzweigtes ($C_4$-$C_7$)-Alkyl bedeutet ; und/oder

A bedeutet :

unsubstituiertes oder substituiertes ($C_1$-$C_6$)-Alkyl mit gleich oder unterschiedlich eins bis zwei Halo ;

Cyclohexyl ;

unsubstituiertes oder substituiertes ($C_2$-$C_6$)-Alkenyl mit gleich oder unterschiedlich eins bis drei aus Halo, Methyl oder Ethyl ;

Cyclohexenyl ; oder

Benzyl ; und/oder

B bedeutet : unsubstituiertes oder substituiertes Phenyl, wobei die Substituenten gleich oder unterschiedlich eins oder drei aus Halo, ($C_1$-$C_4$)-Alkyl, ($C_1$-$C_4$)-Alkoxy oder Halo-($C_1$-$C_4$)-alkyl sein können.

5. Zusammensetzung nach Anspruch 4, wobei

X und X′ die Bedeutung O haben ; und/oder

$R^1$ die Bedeutung t-Butyl, Neopentyl (2,2-Dimethylpropyl) oder 1,2,2-Trimethylpropyl hat ; und/oder

A bedeutet :

unsubstituiertes ($C_3$-$C_5$)-Alkyl ;

Cyclohexyl ;

($C_2$-$C_4$) unsubstituiertes oder substituiertes geradkettiges Alkenyl mit gleich oder unterschiedlich ein bis zwei aus Chlor, Brom oder Methyl ;

Cyclohexenyl ; oder

Benzyl ; und/oder

B bedeutet :

unsubstituiertes oder substituiertes Phenyl, wobei die Substituenten gleich oder unterschiedlich eins oder

zwei aus Chlor, Fluor, Brom, Iod, Methyl, Ethyl, Methoxy oder Trifluormethyl sein können.

6. Verbindung nach Anspruch 1, wobei

X und X' die Bedeutung O haben ;

$R^1$ die Bedeutung t-Butyl hat ; und

B die Bedeutung Phenyl hat und A die Bedeutung Cyclohexyl, n-Butyl, n-Propyl, Benzyl, 1-Cyclohexenyl, Dichlormethyl oder Trichlormethyl hat ; oder

B die Bedeutung 4-Chlorphenyl und A die Bedeutung nPropyl oder t-Butyl hat ; oder

B die Bedeutung 3-Methylphenyl hat und A die Bedeutung Norbonyl, Cyclohex-3-enyl, 1-Butenyl, 3-Butenyl, Cis-3,4-Dihydroxycyclohexyl, 2,2-Dimethyl-3a,4,5,6,7,7a-hexahydrobenzo[d]-dioxalano-5-yl, 3Acetyl-2,2-dimethylcyclobutyl-methyl oder 2-Methylcyclohexyl-di-2,5-enyl hat ; oder

B die Bedeutung 3,5-Dimethylphenyl hat und A die Bedeutung Isopropyl oder 1-Methylethenyl hat.

7. Zusammensetzung gemäß Anspruch 2, wobei B die Bedeutung unsubstituiertes Phenyl, 4-Chlorphenyl, 3-substituiertes Phenyl, 2-substituiertes Phenyl oder disubstituiertes Phenyl hat.

8. Zusammensetzung nach einem der vorhergehenden Ansprüche, wobei die insektizide Verbindung mit 0,0001 bis 99 Gew.-% der Zusammensetzung anwesend ist.

9. Zusammensetzung nach Anspruch 8, wobei die insektizide Verbindung mit 0,001 bis 99 Gew.-% der Zusammensetzung ist.

10. Zusammensetzung nach Anspruch 8, wobei die insektizide Verbindung mit 0,01 bis 99 Gew.-% der Zusammensetzung vorhanden ist.

11. Insektizide Zusammensetzung nach einem beliebigen vorhergehenden Anspruch,

(a) zusätzlich enthaltend ein Emulgiermittel, wobei die Zusammensetzung in Form eines emulgierbaren Konzentrats vorliegt ; oder

(b) zusätzlich enthaltend ein Dispergiermittel, wobei die Zusammensetzung in Form eines benetzbaren Pulvers vorliegt ; oder

(c) enthaltend einen flüssigen landwirtschaftlich vertretbaren Trägerstoff und ein Dispergiermittel, wobei die Zusammensetzung in fließbarer Form vorliegt ; oder

(d) in Form eines Staubes ; oder

(e) zusätzlich enthaltend ein Bindemittel, wobei die Zusammensetzung in Form eines Granulats vorliegt ; oder

(f) zusätzlich enthaltend einen Lockstoff, wobei die Zusammensetzung in Form eines Köders vorliegt.

12. Verfahren zum Kontrollieren von Insekten, bei dem man die Insekten mit einer insektizid wirksamen Menge eines insektiziden Inhaltsstoffes gemäß einem der Ansprüche 1 bis 6 in Kontakt bringt, wahlweise in einer Zusammensetzung nach einem der vorhergehenden Ansprüche.

13. Verfahren nach Anspruch 12, wobei man die insektizid wirksame Menge eines insektiziden Inhaltstoffes wachsenden Pflanzen oder einem Gebiet zuführt, wo die Pflanzen gezogen werden, bei einer Dosisrate von 10 g bis 10 kg pro Hektar.

14. Verfahren nach Anspruch 13, wobei die Applikationsrate 100 g bis 5 kg pro Hektar beträgt.

15. Verfahren nach Anspruch 12, oder 13 zum Töten von Insekten der Ordnung Lepidoptera oder Coleoptera.

**Patentansprüche für den Vertragsstaat : ES**

1. Verwendung einer insektiziden Verbindung der Formel :

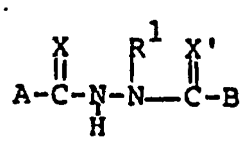

(I),

wobei

X und X' gleich oder unterschiedlich die Bedeutung O, S oder NR haben ;

$R^1$ bedeutet :

unsubstituiertes verzweigtes $(C_3-C_{10})$-Alkyl oder geradkettiges $(C_1-C_4)$-Alkyl gleich oder unterschiedlich substituiert ist mit eins oder zwei $(C_3-C_6)$-Cycloalkyl ;

A bedeutet :

unsubstituiertes oder substituiertes ($C_1$-$C_{10}$)-Alkyl mit gleich oder unterschiedlich eins bis vier aus Halo, Cyano, Nitro, Hydroxy, ($C_1$-$C_4$)-Alkoxy, Carboxy, ($C_1$-$C_4$)-Alkoxycarbonyl, ($C_1$-$C_4$)-Alkanoyloxy, $NH_2$, NHZ oder NZZ' ;

unsubstituiertes oder substituiertes ($C_3$-$C_8$)-Cycloalkyl oder ($C_3$-$C_8$)-Cycloalkyl-($C_1$-$C_4$)-alkyl mit gleich oder unterschiedlich eins bis vier aus Halo, Cyano, Nitro, Hydroxy, ($C_1$-$C_4$)-Alkyl, Halo($C_1$-$C_4$)-alkyl, ($C_1$-$C_4$)-Alkoxy, Halo-($C_1$-$C_4$)-alkoxy, Carboxy, ($C_1$-$C_4$)-Alkanoyl, ($C_1$-$C_4$)-Alkoxycarbonyl, ($C_1$-$C_4$)-Alkanoyloxy, $NH_2$, NHZ oder NZZ' ;

unsubstituiertes oder substituiertes ($C_2$-$C_8$)-Alkenyl oder unsubstituiertes oder substituiertes ($C_3$-$C_8$)-Alkadienyl mit gleich oder unterschiedlich eins bis vier aus Halo, Cyano, Nitro, Hydroxy, ($C_1$-$C_4$)-Alkyl, ($C_3$-$C_6$)-Cycloalkyl, Halo-($C_1$-$C_4$)-alkyl, ($C_1$-$C_4$)-Alkoxy, Halo-($C_1$-$C_4$)-Alkoxy, Carboxy, ($C_1$-$C_4$)-Alkoxycarbonyl, ($C_1$-$C_4$)-Alkanoyloxy, $NH_2$, NHZ oder NZZ' ;

unsubstituiertes oder substituiertes ($C_3$-$C_8$)-Cycloalkenyl oder unsubstituiertes oder substituiertes ($C_6$-$C_8$)-Cycloalkadienyl mit gleich oder unterschiedlich eins bis vier aus Halo, Cyano, Nitro, Hydroxy, ($C_1$-$C_4$)-Alkyl, Halo-($C_1$-$C_4$)-alkyl, ($C_1$-$C_4$)-Alkoxy, Halo-($C_1$-$C_4$)-alkoxy, Carboxy, ($C_1$-$C_4$)-Alkoxycarbonyl, ($C_1$-$C_4$)-Alkanoyloxy, $NH_2$, NHZ oder NZZ' ;

unsubstituiertes oder substituiertes ($C_2$-$C_8$)-Alkynyl mit gleich oder unterschiedlich eins bis vier aus Halo, Cyano, Nitro, Hydroxy, ($C_1$-$C_4$)-Alkyl, Halo-($C_1$-$C_4$)-Alkyl, ($C_1$-$C_4$)-Alkoxy, Halo-($C_1$-$C_4$)-alkoxy, Carboxy, ($C_1$-$C_4$)-Alkoxycarbonyl, ($C_1$-$C_4$)-Alkanyloxy, $NH_2$, NHZ oder NZZ' oder ;

Phenalkyl mit eins bis vier Kohlenstoffatomen in der Alkylgruppe, wobei der Phenylring unsubstituiert ist oder gleich oder unterschiedlich substituiert ist mit eins bis drei aus Halo, Cyano, Nitro, Hydroxy, ($C_1$-$C_4$)-Alkyl, Halo-($C_1$-$C_4$)-alkyl, Cyano-($C_1$-$C_4$)-alkyl, ($C_1$-$C_4$)-Alkoxy, Halo-($C_1$-$C_4$)-alkoxy, Carboxy, ($C_1$-$C_4$)-Alkoxycarbonyl, ($C_1$-$C_4$)-Alkanoyloxy, ($C_2$-$C_6$)-Alkenyl, Halo-($C_2$-$C_6$)-alkenyl, ($C_2$-$C_6$)-Alkynyl, $NH_2$, NHZ oder NZZ' ;

B bedeutet :

unsubstituiertes oder substituiertes Naphthyl, wobei die Substituenten gleich oder unterschiedlich eins bis drei aus Halo, Cyano, Nitro, Hydroxy, ($C_1$-$C_4$)-Alkoxy, ($C_1$-$C_4$)-Alkyl, Carboxy, ($C_1$-$C_4$)-Alkoxycarbonyl, ($C_1$-$C_4$)-Alkanoyloxy, $NH_2$, NHZ oder NZZ' sein können ;

unsubstituiertes oder substituiertes Phenyl, wobei die Substituenten gleich oder unterschiedlich sein können eins bis fünf aus Halo ; Nitro ; Cyano ; Hydroxy ; ($C_1$-$C_6$)-Alkyl ; Halo-($C_1$-$C_6$)-alkyl ; Cyano($C_1$-$C_6$)-Alkyl ; ($C_1$-$C_6$)-Alkoxy ; Halo-($C_1$-$C_6$)-alkoxy ; Alkoxyalkyl mit unabhängig eins bis sechs Kohlenstoffatomen in jeder Alkylgruppe ; Alkoxyalkoxy mit unabhängig eins bis sechs Kohlenstoffatomen in jeder Alkylgruppe ; $-OCO_2R-$ Gruppe ; ($C_2$-$C_6$)-Alkenyl, wahlweise substituiert mit Halo, Cyano, ($C_1$-$C_4$)-Alkyl, ($C_1$-$C_4$)-Alkoxy, Halo-($C_1$-$C_4$)-alkoxy oder ($C_1$-$C_4$)-Alkylthio ; $-RCO_2R'-$ Gruppe ; $-COR-$ Gruppe ; Halo-($C_1$-$C_6$)-alkylcarbonyl ; Cyano-($C_1$-$C_6$)-alkylcarbonyl ; Nitro-($C_1$-$C_6$)-akylcarbonyl ; $-CO_2R-$ Gruppe ; Halo-($C_1$-$C_6$)-Alkoxycarbonyl ; $-OCOR-$ Gruppe, $-NRR'-$ Gruppe ; Amino substituiert mit Hydroxy, ($C_1$-$C_4$)-Alkoxy oder ($C_1$-$C_4$)-Alkylthio-Gruppen ; Phenylamino ; Diphenylamino ; $-CONRR'-$ Gruppe ; $-OCONRR'-$ Gruppe ; $-NRCOR'-$ Gruppe ; $-NRCO_2R'$Gruppe ; $-N(COR)COR'-$ Gruppe ; $-OCONRCOR'-$ Gruppe ; Sulfhydryl ; Halothio ; ($C_1$-$C_6$)-Alkylthio ; Halo-($C_1$-$C_6$)-alkylthio ; $-SOR-$ Gruppe ; $-SO_2R-$ Gruppe ; Phenylsulfonyl ; $-OSO_2R-$ Gruppe ; Halo-($C_1$-$C_6$)-Alkylsulfonyloxy ; $-SO_2NRR'-$ Gruppe ; $-NRSOR'-$ Gruppe ; $-NRSO_2R'-$ Gruppe ; $-CSR-$ Gruppe ; $CS_2R-$ Gruppe ; $-NRCSR'-$ Gruppe ; $-SCOR-$ Gruppe ; unsubstituiertes oder substituiertes Phenyl mit gleich oder unterschiedlich eins bis drei aus Halo, Cyano, Nitro, Hydroxy, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, $NH_2$-Gruppe ; NHZ-Gruppe oder NZZ'-Gruppe ; Phenoxy, wobei der Phenylring unsubstiuiert ist oder gleich oder unterschiedlich substituiert ist mit eins bis drei aus Halo, Cyano, Nitro, Hydroxy, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, $NH_2$-Gruppe ; NHZ-Gruppe ; oder NZZ'-Gruppe ; Phenylthio, wobei der Phenylring unsubstituiert ist oder gleich oder unterschiedlich substituiert ist mit eins bis drei aus Halo, Cyano, Nitro, Hydroxy ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, oder $NH_2$-Gruppe ; NHZ-Gruppe ; oder NZZ'-Gruppe ; oder wenn zwei benachbarte Positionen am Phenylring mit Alkoxygruppen substituiert sind, diese Gruppen verbunden sein können, um zusammen mit den Kohlenstoffatomen, an denen sie beide hängen, einen 5-oder 6-gliedrigen heterocyclischen Dioxolan- oder Dioxanring zu bilden ;

wobei R, R' und R" Wasserstoff oder ($C_1$-$C_6$)-Alkyl bedeuten ; Z und Z' ($C_1$-$C_4$)-Alkyl bedeuten ; wobei "Amino" NRR' bedeutet ; oder eines landwirtschaftlich vertretbaren Salzes davon zusammen mit landwirtschaftlich vertretbarem Verdünnungs- oder Trägerstoff zur Herstellung einer insektiziden Zusammensetzung.

2. Verwendung nach Anspruch 1 einer Verbindung oder eines Salzes, wobei

X und X' die Bedeutung O oder S haben ; und/oder

R1 bedeutet:
unsubstituiertes verzweigtes ($C_3$-$C_8$)-Alkyl oder geradkettiges ($C_1$-$C_4$)-Alkyl, gleich oder unterschiedlich substituiert mit eins oder zwei ($C_3$-$C_4$)-Cycloalkyl ; und/oder
A bedeutet:
unsubstituiertes oder unsubstituiertes ($C_1$-$C_6$)-Alkyl mit gleich oder unterschiedlich eins bis drei aus Halo, Cyano, Nitro, ($C_1$-$C_4$)-Alkoxy, Carboxy, ($C_1$-$C_4$)-Alkoxycarbonyl, ($C_1$-$C_4$)-Alkanoyloxy, $NH_2$, NHZ oder NZZ' ;
($C_3$-$C_6$)-Cycloalkyl ;
unsubstituiertes oder substituiertes ($C_2$-$C_6$)-Alkenyl mit gleich oder unterschiedlich eins bis drei aus Halo, ($C_1$-$C_4$)-Alkyl, Halo-($C_1$-$C_4$)-Alkyl, ($C_1$-$C_4$)-Alkoxy oder Halo-($C_1$-$C_4$)-alkoxy ;
($C_3$-$C_6$)-Cycloalkenyl ; oder
Phenalkyl mit einem oder zwei Kohlenstoffatomen in der Alkylgruppe, wobei der Phenylring unsubstituiert ist oder gleich oder unterschiedlich substituiert ist mit eins oder zwei aus Halo, Nitro oder ($C_1$-$C_4$)-Alkyl ; und/oder
B unsubstituiertes Naphthyl bedeutet ; oder
unsubstituiertes oder substituiertes Phenyl, wobei die Substituenten gleich oder unterschiedlich sein können eins bis drei aus Halo ; Nitro ; Cyano ; ($C_1$-$C_4$)-Alkyl ; Halo-($C_1$-$C_4$)-alkyl ; Cyano-($C_1$-$C_4$)-alkyl ; ($C_1$-$C_4$)-Alkoxy ; Alkoxyalkyl mit unabhängig eins bis vier Kohlenstoffatomen in jeder Alkylgruppe ; $-COD^4-$ Gruppe ; Carboxy ; ($C_1$-$C_4$)-Alkoxycarbonyl ; ($C_1$-$C_4$)-Alkanoyloxy ; $NH_2$ ; NHZ ; NZZ' ; ($C_1$-$C_4$)-Alkylthio ; $-CSD^4-$ Gruppe ; $-CS_2D^4-$ Gruppe ; $-SCOD^4-$ Gruppe ; unsubstituiertes Phenyl mit gleich oder unterschiedlich eins bis zwei aus Halo, Nitro ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, $NH_2$, NHZ oder NZZ' sein können ; Phenoxy, wobei der Phenylring unsubstituiert ist oder gleich oder unterschiedlich substituiert ist mit eins oder zwei aus Halo, Nitro, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, $NH_2$, NHZ oder NZZ' ; oder wenn zwei benachbarte Positionen am Phenylring mit Alkoxy-Gruppen substituiert sind, diese Gruppen verbunden sein können, um zusammen mit den Kohlenstoffatomen, an denen sie hängen, ein 5- oder 6-gliedrigen heterocyclischen Dioxolan- oder Dioxanring zu bilden ;
wobei $D^4$ Wasserstoff oder ($C_1$-$C_4$)-Alkyl bedeutet ; Z und Z' ($C_1$-$C_4$)-Alkyl bedeuten.

3. Verwendung nach Anspruch 2 einer Verbindung oder eines Salzes, wobei
X und X' die Bedeutung O oder S haben ; und/oder
$R^1$ verzweigtes ($C_3$-$C_8$)-Alkyl bedeutet ; und/oder
A bedeutet:
unsubstituiertes oder substituiertes ($C_1$-$C_6$)-Alkyl mit gleich unterschiedlich eins bis drei aus Halo, Nitro, Cyano, Carboxy oder ($C_1$-$C_4$)-Alkoxycarbonyl ;
Cyclohexyl ;
Cyclohexenyl ;
unsubstituiertes oder substituiertes ($C_2$-$C_6$)-Alkenyl mit gleich oder unterschiedlich eins bis drei aus Halo oder ($C_1$-$C_4$)-Alkyl ; oder
Benzyl, wobei der Phenylring unsubstituiert ist oder gleich oder unterschiedlich substituiert ist mit eins oder zwei aus Halo, Methyl oder Ethyl ; und/oder
B bedeutet:
unsubstituiertes Naphthyl ;
unsubstituiertes oder substituiertes Phenyl mit gleich oder unterschiedlich eins bis zwei Halo ; Nitro ; Cyano ; ($C_1$-$C_4$)-Alkyl ; Halo-($C_1$-$C_4$)-Alkyl ; Cyano-($C_1$-$C_4$)-alkyl ; ($C_1$-$C_4$)-Alkoxy ; $-COD^4-$ Gruppe ; ($C_1$-$C_4$)Alkoxycarbonyl ; ($C_1$-$C_4$)-Alkanoyloxy ; oder unsubstituiertes oder substituiertes Phenyl mit gleich oder unterschiedlich eins oder zwei aus Halo, Nitro, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, $NH_2$, NHZ oder NZZ' ; wobei $D^4$ die Bedeutung Wasserstoff oder ($C_1$-$C_4$)-Alkyl hat ; Z und Z' die Bedeutung ($C_1$-$C_4$)-Alkyl haben.

4. Verwendung nach Anspruch 3 einer Verbindung eines Salzes, wobei
X und X' die Bedeutung O haben ; und/oder
$R^1$ verzweigtes ($C_4$-$C_7$)-Alkyl bedeutet ; und/oder
A bedeutet:
unsubstituiertes oder substituiertes ($C_1$-$C_6$)-Alkyl mit gleich oder unterschiedlich eins bis zwei Halo ;
Cyclohexyl ;
unsubstituiertes oder substituiertes ($C_2$-$C_6$)-Alkenyl mit gleich oder unterschiedlich eins bis drei aus Halo, Methyl oder Ethyl ;
Cyclohexenyl ; oder
Benzyl ; und/oder

B bedeutet:
unsubstituiertes oder substituiertes Phenyl, wobei die Substituenten gleich oder unterschiedlich eins oder drei aus Halo, ($C_1$-$C_4$)-Alkyl, ($C_1$-$C_4$)-Alkoxy oder Halo-($C_1$-$C_4$)-alkyl sein können.

5. Verwendung nach Anspruch 4 einer Verbindung oder eines Salzes wobei
X und X' die Bedeutung O haben ; und/oder
$R^1$ die Bedeutung t-Butyl, Neopentyl (2,2-Dimethylpropyl) oder 1,2,2-Trimethylpropyl hat ; und/oder
A bedeutet:
unsubstituiertes ($C_3$-$C_5$)-Alkyl ;
Cyclohexyl ;
($C_2$-$C_4$) unsubstituiertes oder substituiertes geradkettiges Alkenyl mit gleich oder unterschiedlich ein bis zwei aus Chlor, Brom oder Methyl ;
Cyclohexenyl ; oder
Benzyl ; und/oder
B bedeutet:
unsubstituiertes oder substituiertes Phenyl, wobei die Substituenten gleich oder unterschiedlich eins oder zwei aus Chlor, Fluor, Brom, Iod, Methyl, Ethyl, Methoxy oder Trifluormethyl sein können.

6. Verwendung nach Anspruch 1 einer Verbindung oder eines Salzes, wobei
X und X' die Bedeutung O haben ;
$R^1$ die Bedeutung t-Butyl hat ; und
B die Bedeutung Phenyl hat und A die Bedeutung Cyclohexyl, n-Butyl, n-Propyl, Benzyl, 1-Cyclohexenyl, Dichlormethyl oder Trichlormethyl hat ; oder
B die Bedeutung 4-Chlorphenyl und A n-Propyl oder t-Butyl hat ; oder
B die Bedeutung 3-Methylphenyl hat und A die Bedeutung Norbonyl, Cyclohex-3-enyl, 1-Butenyl, 3-Butenyl, Cis-3,4-Dihydroxycyclohexyl, 2,2-Dimethyl-3a, 4,5,6,7,7a-hexahydrobenzo[d]-dioxalano-5-yl, 3-Acetyl-2,2-dimethylcyclobutyl-methyl oder 2-Methylcyclohexyl-di-2,5-enyl hat ; oder
B die Bedeutung 3,5-Dimethylphenyl hat und A die Bedeutung Isopropyl oder 1-Methylethenyl hat.

7. Verwendung nach Anspruch 2 der Verbindung oder eines Salzes, wobei B unsubstituiertes Phenyl, 4-Chlorphenyl, 3-substituiertes Phenyl, 2-substituiertes Phenyl oder disubstituiertes Phenyl bedeutet.

8. Verwendung nach einem der vorhergehenden Ansprüche der Verbindung oder des Salzes in einer Menge von 0,0001 bis 99 Gew.-% der Zusammensetzung.

9. Verwendung nach Anspruch 8 der Verbindung oder des Salzes in einer Menge von 0,001 bis 99 Gew.-% der Zusammensetzung.

10. Verwendung nach Anspruch 8 der Verbindung oder des Salzes in einer Menge von 0,01 bis 99 Gew.-% der Zusammensetzung.

11. Verwendung nach einem der vorhergehenden Ansprüche der Verbindung oder des Salzes,
(a) zusammen mit einem Emulgiermittel, wobei die Zusammensetzung in Form eines emulgierbaren Konzentrats vorliegt ; oder
(b) zusammen mit einem Dispergiermittel, wobei die Zusammensetzung in Form eines benetzbaren Pulvers vorliegt ; oder
(c) zusammen mit einem flüssigen landwirtschaftlich vertretbaren Trägerstoff und einem Dispergiermittel, wobei die Zusammensetzung in Form eines Fließbaren vorliegt ; oder
(d) in Form eines Staubes ; oder
(e) zusammen mit einem Bindemittel, wobei die Zusammensetzung in Form eines Granulats vorliegt ; oder
(f) zusammen mit einem Lockstoff, wobei die Zusammensetzung in Form eines Köders vorliegt.

12. Verwendung einer Verbindung oder eines Salzes wie in einem der Ansprüche 1 bis 7 definiert, wahlweise in einer Zusammensetzung auch enthaltend landwirtschaftlich vertretbaren Verdünnungs- oder Trägerstoff, zum Kontrollieren von Insekten, bei dem man die Insekten mit einer insektizid wirksamen Menge der Verbindung oder des Salzes in Kontakt bringt.

13. Mechanisches Verfahren zur Verbesserung des Handelswertes und/oder der Rentabilität verkaufbarer Ernten aus Pflanzen, deren Wachstum von Insekten beeinflußt wird oder möglicherweise beinflußt wird, wobei man
(1) einem Behälter, einer Vernebelungsvorrichtung oder einem mechanischen Ausbreiter eine insektizide Verbindung oder einem Salz wie in einem der Ansprüche 1 bis 7 definiert zuführt, wahlweise in einer Mischung mit landwirtschaftlich vertretbaren Verdünnungs- oder Trägerstoff,
(2) den Behälter, Vernebeler oder mechanischen Ausbreiter dazu verwendet, die insektizide Verbindung oder das Salz in Form von Granulat, Staub, Rauch, Dampf oder einer tensidhaltigen Flüssigkeitszubereitung wachsenden Pflanzen oder einem Wachstumsmedium zu applizieren, in dem die Pflanzen wachsen oder gezogen werden, oder den Insekten selbst,

(3) die Dosis des Wirkstoffs während des Applizierungsschrittes so steuert, daß die Applizierungsrate der aktiven insektiziden Verbindung zur Bekämpfung der Insektizide ausreicht, jedoch nicht ausreicht zur Erzeugung einer nicht hinnehmbaren nachteiligen Auswirkung auf die in dem behandelten Gebiet wachsenden oder zu ziehenden Erntepflanzen.

14. Verwendung oder Verfahren nach Anspruch 12 oder 13, wobei man die insektizide Verbindung wachsenden Pflanzen oder einem Gebiet zuführt, wo die Pflanzen gezogen werden, bei einer Dosisrate von 10 g bis 10 kg pro Hektar.

15. Verwendung oder Verfahren nach Anspruch 14, wobei die Applikationsrate 100 g bis 5 kg pro Hektar beträgt.

16. Verwendung oder Verfahren nach Anspruch 13 oder 14 zum Töten von Insekten der Ordnung Lepidoptera oder Coleoptera.

## Revendications

### Revendications pour les Etats Contractants : BE, CH, DE, FR, GB, GR, IT, LI, LU, NL, SE.

1. Composé insecticide répondant à la formule :

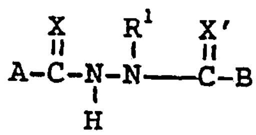

I

dans laquelle

X et X′ qui sont identiques ou différents, sont O, S OU NR ;

$R^1$ est alkyle($C_3$-$C_{10}$) ramifié, non substitué ou alkyle($C_1$-$C_4$) à chaîne droite, substitué par un ou deux cycloalkyles ($C_3$-$C_6$) identiques ou différents ;

A est alkyle($C_1$-$C_{10}$) non substitué ou substitué présentant un à quatre substituants identiques ou différents,choisis parmi halogène, cyano, nitro, hydroxy, alcoxy($C_1$-$C_4$), carboxy, alcoxy-($C_1$-$C_4$)-carbonyle, alcanoyloxy($C_1$-$C_4$), $NH_2$, NHZ ou NZZ′ ;

cycloalkyle($C_3$-$C_8$) ou cycloalkyl($C_3$-$C_8$)-alkyle-($C_1$-$C_4$), non substitué ou substitué, présentant un à quatre substituants identiques ou différents, choisis parmi halogène, cyano, nitro, hydroxy, alkyle($C_1$-$C_4$), halogéno-alkyle($C_1$-$C_4$), alcoxy($C_1$-$C_4$), halogéno-alcoxy($C_1$-$C_4$), carboxy, alcanoyle($C_1$-$C_4$), alcoxy($C_1$-$C_4$)-carbonyle, alcanoyloxy($C_1$-$C_4$), $NH_2$, NHZ ou NZZ′ ;

alcényle($C_2$-$C_8$) non substitué ou substitué ou alcadiényle($C_3$-$C_8$) non substitué ou substitué, présentant un à quatre substituants identiques ou différents, choisis parmi halogène, cyano, nitro, hydroxy, alkyle($C_1$-$C_4$), cycloalkyle($C_3$-$C_6$), halogéno-alkyle($C_1$-$C_4$), alcoxy($C_1$-$C_4$), halogéno-alcoxy($C_1$-$C_4$), carboxy, alcoxy ($C_1$-$C_4$)-carbonyle, alcanoyloxy($C_1$-$C_4$), $NH_2$, NHZ ou NZZ′ ; cycloalcényle($C_3$-$C_8$) non substitué ou substitué ou cycloalcadiényle($C_6$-$C_8$) non substitué ou substitué, présentant un à quatre substituants identiques ou différents, choisis parmi halogène, cyano, nitro, hydroxy, alkyle($C_1$-$C_4$), halogéno-alkyle($C_1$-$C_4$), alcoxy($C_1$-$C_4$), halogéno-alcoxy($C_1$-$C_4$), carboxy, alcoxy($C_1$-$C_4$)-carbo-nyle, alcanoyloxy($C_1$-$C_4$), $NH_2$, NHZ ou NZZ′.

alcynyle ($C_2$-$C_8$) non substitué ou substitué présentant un à quatre substituants identiques ou différents choisis parmi halogène, cyano, nitro, hydroxy, alkyle ($C_1$-$C_4$), halogéno-alkyle-($C_1$-$C_4$), alcoxy ($C_1$-$C_4$), halogéno-alcoxy ($C_1$-$C_4$), carboxy, alcoxy ($C_1$-$C_4$)-carbonyle, alcanoyloxy-($C_1$-$C_4$), $NH_2$, NHZ ou NZZ′; ou

phénylalkyle présentant un à quatre atomes de carbone dans le groupe alkyle et le cycle phényle est non substitué ou substitué par un à trois substituants identiques ou différents, choisis parmi halogène, cyano, nitro, hydroxy, alkyle ($C_1$-$C_4$), halogéno-alkyle ($C_1$-$C_4$), cyano-alkyle ($C_1$-$C_4$), alcoxy ($C_1$-$C_4$), halogéno-alcoxy ($C_1$-$C_4$), carboxy, alcoxy ($C_1$-$C_4$)-carbonyle, alcanoyloxy ($C_1$-$C_4$), alcényle ($C_2$-$C_6$), halogéno-alcényle ($C_2$-$C_6$), alcynyle ($C_2$-$C_6$), $NH_2$, NHZ ou NZZ′.

B est naphtyle non substitué ou substitué, où les substituants peuvent être de 1 à 3, identiques ou différents, choisis parmi halogène ; cyano ; nitro ; hydroxy ; alcoxy($C_1$-$C_4$) ; alkyle($C_1$-$C_4$) ; carboxy ; alcoxy($C_1$-$C_4$)-carbonyle ; alconoyloxy-($C_1$-$C_4$) ; $NH_2$, NHZ ou NZZ′ ;

phényle non substitué ou substitué où les substituants peuvent être de 1 à 5, identiques ou différents, choisis parmi halogène ; nitro ; cyano ; hydroxy ; alkyle ($C_1$-$C_6$), halogéno-alkyle ($C_1$-$C_6$), cyano-alkyle ($C_1$-$C_6$);

alcoxy ($C_1$-$C_6$) ; halogéno-alcoxy ($C_1$-$C_6$) ; alcoxy-alkyle ayant indépendamment 1 à 6 atomes de carbone dans chaque groupe alkyle ; alcoxy-alcoxy ayant indépendamment 1 à 6 atomes de carbone dans chaque groupe alkyle ; un groupe $-OCO_2R$ ; alcényle ($C_2$-$C_6$), éventuellement substitué par halogène, cyano, alkyle ($C_1$-$C_4$), alcoxy ($C_1$-$C_4$), halogéno-alcoxy($C_1$-$C_4$) ou alkylthio ($C_1$-$C_4$) ; le groupe $-RCO_2R'$ ; le groupe $-COR$ ; halogéno-alkyl ($C_1$-$C_6$)-carbonyle ; cyano-alkyl ($C_1$-$C_6$)-carbonyle ; nitro-alkyl ($C_1$-$C_6$)-carbonyle ; le groupe $-CO_2R$ ; halogéno-alcoxy ($C_1$-$C_6$) -carbonyle ; le groupe $-OCOR$ ; le groupe $-NRR'$ ; aminosubstitué par des groupes hydroxy, alcoxy($C_1$-$C_4$) ou alkylthio ($C_1$-$C_4$) ; phényl-amino ; diphénylamino ; le groupe $-CONRR'$ ; le groupe $-OCONRR'$ ; le groupe $-NRCOR'$, le groupe $-NRCO_2R'$ ; le groupe $-N(COR)COR'$ ; le groupe OCONRCOR' ; sulfhydryle ; halogénothio ; alkylthio ($C_1$-$C_6$) ; halogéno-alkylthio ($C_1$-$C_6$) ; le groupe $-SOR$ ; le groupe $-SO_2R$ ; phénylsulfonyle ; le groupe $-OSO_2R$ ; halogéno-alkyl ($C_1$-$C_6$)-sulfonyloxy ; le groupe $SO_2NRR'$ ; le groupe $-NRSOR'$ ; le groupe $-NRSO_2R'$ ; le groupe $-CSR$ ; le groupe $-CS_2R$ ; le groupe $-NRCSR'$ ; le groupe $-SCOR$ ; phényle non substitué ou substitué ayant un à trois substituants identiques ou différents choisis parmi halogène, cyano, nitro, hydroxy-alkyle-($C_1$-$C_4$), alcoxy ($C_1$-$C_4$), carboxy, alcoxy ($C_1$-$C_4$)-carbonyle, alcanoyloxy ($C_1$-$C_4$), le groupe $NH_2$ ; le groupe NHZ ; ou le groupe NZZ' ; phénoxy où le cycle phényle est non substitué ou substitue par un à trois substituants identiques ou différents choisis parmi halogène, cyano, nitro, hydroxy, alkyle ($C_1$-$C_4$), alcoxy($C_1$-$C_4$), carboxy, alcoxy($C_1$-$C_4$)-carbonyle, alcanoyloxy($C_1$-$C_4$), le groupe $NH_2$ ; le groupe NHZ ; ou le groupe NZZ' ; phényl-thio où le cycle phényle est non substitué ou substitué par un à trois substituants identiques ou différents choisis parmi halogène, cyano, nitro, hydroxy, alkyle($C_1$-$C_4$), alcoxy ($C_1$-$C_4$), carboxy, alcoxy($C_1$-$C_4$)-carbonyle, alcanoyloxy($C_1$-$C_4$), le groupe $NH_2$ ; le groupe NHZ ; ou le groupe NZZ' ; ou lorsque deux positions adjacentes sur le cycle phényle sont substituées par des groupes alcoxy, ces groupes peuvent être liés pour former, ensemble avec les atomes de carbone auxquels ils sont l'un et l'autre fixés, un cycle hétérocyclique dioxolano ou dioxano à 5 ou 6 membres ;

où R, R' et R" sont hydrogène ou alkyle ($C_1$-$C_6$) ; Z et Z' sont alkyle ($C_1$-$C_4$) ; "amino" signifie NRR' ; et ses sels agronomiquement acceptables.

2. Composé selon la revendication 1, dans lequel

X et X' sont O ou S ; et/ou

$R^1$ est alkyle($C_1$-$C_8$) ramifié, non substitué, ou alkyle($C_1$-$C_4$) à chaîne droite, substitué par un ou deux groupes cycloalkyle($C_3$-$C_4$) identiques ou différents ; et/ou

A est alkyle($C_1$-$C_6$) non substitué ou substitué ayant un à trois substituants indentiques ou différents, choisis parmi halogène, cyano, nitro, alcoxy($C_1$-$C_4$), carboxy, alcoxy($C_1$-$C_4$)-carbonyle, alcanoyloxy ($C_1$-$C_4$), $NH_2$, NHZ ou NZZ' ;

cycloalkyle ($C_3$-$C_6$) ;

alcenyle($C_2$-$C_6$) non substitué ou substitué ayant 1 à 3 substituants identiques ou différents, choisis parmi halogène, alkyle ($C_1$-$C_4$) ; halogéno-alkyle ($C_1$-$C_4$), alcoxy ($C_1$-$C_4$) ou halogéno-alcoxy($C_1$-$C_4$) cycloalcényle ($C_3$-$C_8$) ; ou

phénylalkyle présentant 1 ou 2 atomes de carbone dans le groupe alkyle et le cycle phényle est non substitué ou substitué par un ou deux substituants identiques ou différents, choisis parmi halogène, nitro ou alkyle($C_1$-$C_4$) ; et /ou

B est naphtyle non substitué ; ou phényle non substitué ou substitué où les substituants peuvent être de un à trois, identiques ou différents, choisis parmi halogène ; nitro ; cyano ; alkyle($C_1$-$C_4$), halogéno-alkyle($C_1$-$C_4$) ; cyano-alkyle($C_1$-$C_4$) ; alcoxy ($C_1$-$C_4$) ; alcoxy-alkyle ayant indépendamment 1 à 4 atomes de carbone dans chaque groupe alkyle ; le groupe $-COD^4$ ; carboxy ; alcoxy($C_1$-$C_4$)-carbonyle ; alcanoyloxy($C_1$-$C_4$) ; $NH_2$ ; NHZ ; NZZ' ; alkylthio($C_1$-$C_4$) ; le groupe $-CSD^4$ ; le groupe $-CS_2D^4$ ; le groupe $-SCOD^4$ ; phényle non substitué ou substitué ayant un à deux substituants identiques ou différents, choisis parmi halogène, nitro, alkyle($C_1$-$C_4$), alcoxy($C_1$-$C_4$), carboxy, alcoxy($C_1$-$C_4$)-carbonyle, alcanoyloxy ($C_1$-$C_4$), $NH_2$, NHZ ou NZZ' ; phénoxy où le cycle phényle est non substitué ou substitué par un ou deux substituants identiques ou différents, choisis parmi halogène, nitro, alkyle($C_1$-$C_4$), alcoxy($C_1$-$C_4$), carboxy, alcoxy($C_1$-$C_4$)-carbonyle, alcanoyloxy($C_1$-$C_4$), $NH_2$, NHZ ou NZZ' ; ou lorsque deux positions adjacentes sur le cycle phényle sont substituées par des groupes alcoxy, ces groupes peuvent être reliés pour former, ensemble avec les atomes de carbone auxquels ils sont fixés, un cycle hétérocyclique dioxolano ou dioxano à 5 ou 6 membres ;

où $D^4$ est hydrogène ou alkyle ($C_1$-$C_4$) ; Z et Z' sont alkyle ($C_1$-$C_4$) ; et

ses sels agronomiquement acceptables.

3. Composé selon la revendication 2, dans lequel :

X et X' sont O ou S ; et/ou

$R^1$ est alkyle($C_3$-$C_8$) ramifié ; et/ou

A est alkyle($C_1$-$C_6$) non substitué ou substitué ayant 1là 3 substituants identiques ou différents, choisis

parmi halogène, cyano, nitro, carboxy ou alcoxy($C_1$-$C_4$)-carbonyle ;
cyclohexyle ;
cyclohexènyle :
alcényle($C_2$-$C_6$) non substitué ou substitué ayant un à trois substituants identiques ou différents, choisis parmi halogène ou alkyle($C_1$-$C_4$) ; ou
benzyle où le cycle phényle est non substitué ou substitué par un ou deux substituants identiques ou différents, choisis parmi halogène, méthyle ou éthyle ; et/ou
B est naphtyle non substitué ;
phényle non substitué ou substitué ayant un à trois substituants identiques ou différents, choisis parmi halogène ; nitro ; cyano ; alkyle ($C_1$-$C_4$) ; halogénoalkyle ($C_1$-$C_4$) ; cyano-alkyle ($C_1$-$C_4$) ; alcoxy ($C_1$-$C_4$) ; le groupe $-COD^4$ ; alcoxy. ($C_1$-$C_4$)-carbonyle ; alcanoyloxy ($C_1$-$C_4$) ; ou phényle non substitué ou substitué ayant un ou deux substituants identiques ou différents, choisis parmi halogène, nitro, alkyle ($C_1$-$C_4$), alcoxy ($C_1$-$C_4$), carboxy, alcoxy ($C_1$-$C_4$)-carbonyle, alcanoyloxy($C_1$-$C_4$), $NH_2$, NHZ ou NZZ' ; où $D^4$ est hydrogène ou alkyle($C_1$-$C_4$) ; Z et Z' sont alkyle($C_1$-$C_4$) ; et
ses sels agronomiquement acceptables.

4. Composé selon la revendication 3, dans lequel
X et X' sont O ; et/ou
$R^1$ est alkyle($C_4$-$C_7$) ramifié ; et/ou
A est alkyle($C_1$-$C_6$) non substitué ou substitué ayant un à deux substituants identiques ou différents halogène ;
cyclohexyle ;
alcényle($C_2$-$C_6$) non substitué ou substitué ayant un à trois substituants identiques ou différents, choisis parmi halogène, méthyle ou éthyle ;
cyclohexényle ; ou
benzyle ; et/ou
B est phényle non substitué ou substitué où les substituants peuvent être de un à trois, identiques ou différents, choisis parmi halogène, alkyle($C_1$-$C_4$) ; alcoxy($C_1$-$C_4$) ; ou halogéno-alkyle($C_1$-$C_4$) ; et
ses sels agronomiquement acceptables.

5. Composé selon la revendication 4, dans lequel :
X et X' sont O ; et/ou
$R^1$ est t-butyle, néopentyle (2,2-diméthylpropyle) ou 1,2,2-diméthylpropyle ; et/ou
A est alkyle($C_3$-$C_5$) non substitué ;
cyclohexényle
alcényle($C_2$-$C_4$) à chaîne droite, non substitué ou substitué, ayant un à trois substituants identiques ou différents choisis parmi chlore, brome ou méthyle ;
cyclohexényle ; ou
benzyle ; et/ou
B est phényle non substitué ou substitué où les substituants peuvent être un ou deux, identiques ou différents, choisis parmi chlore, fluor, brome, iode, méthyle, éthyle, méthoxy ou trifluorométhyle ; et
ses sels agronomiquement acceptables.

6. Composé selon la revendication 1, dans lequel :
X et X' sont O ;
$R^1$ est t-butyle ; et
B est phényle et A est cyclohexyle, n-butyle, n-propyle, benzyle, 1-cyclohexényle, dichlorométhyle ou trichlorométhyle ; ou
B est 4-chlorophényle et A est n-propyle ou t-butyle ; ou
B est 3-méthyl phényle et A est norbornyle, cyclohex-3-ényle, 1-butényle, 3-butényle, cis-3,4-dihydroxycyclohexyle, 2,2-diméthyl-3a-4,5,6,7, 7a-hexahydrobenzo[d]dioxalano-5-yle, 3-acétyl-2,2-diméthylcyclobutyl-méthyle ou 2-méthylcyclohexyl-di-2,5-ényle ; ou
B est 3,5-diméthylphényle et A est isopropyle ou 1-méthyléthényle.

7. Composé tel que revendiqué à la revendication 2, dans lequel B est phényle non substitué, 4-chlorophényle, phényle substitué en position 3, phényle substitué en position 2 ou phényle disubstitué.

8. Composition insecticide comprenant un composé insecticide tel que revendiqué dans l'une quelconque des revendications précédentes ensemble avec du diluant ou excipient agronomiquement acceptable.

9. Composition selon la revendication 8, dans laquelle ledit composé insecticide est présent à raison de 0,0001 à 99% en poids de la composition.

10. Composition selon la revendication 8, dans laquelle ledit composé insecticide est présent à raison de 0,001 à 99% en poids de la composition.

11. Composition selon la revendication 8, dans laquelle ledit composé insecticide est présent à raison de 0,01 à 99% en poids de la composition.

12. Composition insecticide selon l'une quelconque des revendications 8 à 11, (a) contenant en outre un agent émulsifiant, ladite composition étant sous la forme d'un concentré émulsifiable ; ou (b) contenant en outre un agent dispersant, ladite composition étant sous la forme d'une poudre mouillable ; ou (c) contenant un support ou excipient liquide agronomiquement acceptable et un dispersant, ladite composition étant sous une forme apte à l'écoulement ; ou (d) sous la forme d'une poussière ; ou (e) contenant en outre un liant, ladite composition étant sous la forme d'une granule ; ou (f) contenant en outre un agent attractif, ladite composition étant sous la forme d'un appât.

13. Procédé pour contrôler les insectes qui comprend la mise en contact desdits insectes avec une quantité efficace au plan insecticide d'un composé selon l'une quelconque des revendications 1 à 7, éventuellement dans une composition selon l'une quelconque des revendications 8 à 12.

14. Procédé selon la revendication 13, dans lequel le composé insecticide est appliqué à des plantes en culture ou à une zone où des plantes doivent être cultivées, à une posologie de 10 g à 10 kg par hectare.

15. Procédé selon la revendication 14, dans lequel le taux d'application est de 100 g à 5 kg par hectare.

16. Procédé selon la revendication 13, 14 ou 15 pour tuer les insectes de l'ordre des lépidoptères ou des coléoptères.

**Revendications pour l'Etat Contractant : AT.**

1. Composition insecticide comprenant, en tant qu'ingrédient insecticide actif, un composé ayant la formule :

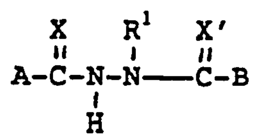

I

dans laquelle

X et X' qui sont identiques ou différents, sont O, S ou NR ;

$R^1$ est alkyle($C_3$-$C_{10}$) ramifié, non substitué ou alkyle($C_1$-$C_4$) à chaîne droite, substitué par un ou deux cycloalkyles($C_3$-$C_6$) identiques ou différents ;

A est alkyle($C_1$-$C_{10}$) non substitué ou substitué, ayant un à quatre substituants identiques ou différents, choisis parmi halogène, cyano, nitro, hydroxy, alcoxy($C_1$-$C_4$), carboxy, alcoxy($C_1$-$C_4$)-carbonyle, alcanoyloxy($C_1$-$C_4$), $NH_2$, NHZ ou NZZ' ;

cycloalkyle ($C_3$-$C_8$) ou cycloalkyl ($C_3$-$C_8$) -alkyle ($C_1$-$C_4$) non substitué ou substitué, ayant un à quatre substituants identiques ou différents, choisis parmi halogène, cyano, nitro, hydroxy, alkyle ($C_1$-$C_4$), halogéno-alkyle ($C_1$-$C_4$), alcoxy($C_1$-$C_4$), halogéno-alcoxy ($C_1$-$C_4$), carboxy, alcanoyle ($C_1$-$C_4$), alcoxy ($C_1$-$C_4$) -carbonyle, alcano-yloxy ($C_1$-$C_4$), $NH_2$, NHZ ou NZZ'

alcényle($C_2$-$C_8$) non substitué ou substitué ou alcadiényle($C_3$-$C_8$) non substitué ou substitué ayant un à quatre substituants identiques ou différents, choisis parmi halogène, cyano, nitro, hydroxy, alkyle($C_1$-$C_4$), cycloalkyle ($C_3$-$C_6$), halogéno-alkyle($C_1$-$C_4$), alcoxy($C_1$-$C_4$), halogéno-alcoxy($C_1$-$C_4$), carboxy, alcoxy($C_1$-$C_4$)-carbonyle, alcanoyloxy($C_1$-$C_4$), $NH_2$, NHZ ou NZZ' ; cycloalcényle($C_3$-$C_8$) non substitué ou substitué ou cycloalcadiényle($C_6$-$C_8$) non substitué ou substitué, ayant un à quatre substituants identiques ou différents, choisis parmi halogène, cyano, nitro, hydroxy, alkyle($C_1$-$C_4$), halogéno-alkyle($C_1$-$C_4$), alcoxy($C_1$-$C_4$), halogénoalcoxy($C_1$-$C_4$), carboxy, alcoxy($C_1$-$C_4$)-carbonyle, alcanoyloxy($C_1$-$C_4$), $NH_2$, NHZ ou NZZ ;

alcynyle($C_2$-$C_8$) non substitué ou substitué ayant un à quatre substituants identiques ou différents, choisis parmi halogène, cyano, nitro, hydroxy, alkyle($C_1$-$C_4$), halogéno-alkyle($C_1$-$C_4$), alcoxy($C_1$-$C_4$), halogéno-alcoxy($C_1$-$C_4$), carboxy, alcoxy($C_1$-$C_4$)-carbonyle, alcanoyloxy ($C_1$-$C_4$), $NH_2$, NHZ ou NZZ' ; ou phénylalkyle ayant un à quatre atomes de carbone dans le groupe alkyle et le cycle phényle est non substitué ou substitué par un à trois substituants identiques ou différents, choisis parmi halogène, cyano, nitro, hydroxy, alkyle ($C_1$-$C_4$), halogéno-alkyle ($C_1$-$C_4$), cyanoalkyle ($C_1$-$C_4$), alcoxy ($C_1$-$C_4$), halogéno-alcoxy-($C_1$-$C_4$), carboxy, alcoxy ($C_1$-$C_4$)-carbonyle, alcanoyloxy ($C_1$-$C_4$), alcényle ($C_2$-$C_6$), halogéno-alcényle($C_2$-$C_6$), alcynyle($C_2$-$C_6$), $NH_2$, NHZ ou NZZ' ;

B est naphtyle non substitué ou substitué, où les substituants peuvent être de 1 à 3, identiques ou différents, choisis parmi halogène ; cyano ; nitro ; hydroxy ; alcoxy ($C_1$-$C_4$) ; alkyle ($C_1$-$C_4$) ; carboxy ; alcoxy ($C_1$-$C_4$)

-carbonyle ; alcanoyloxy-($C_1$-$C_4$) ; $NH_2$, NHZ ou NZZ' ;

phényle non substitué ou substitué où les substituants peuvent être de 1 à 5, identiques ou différents, choisis parmi halogène ; nitro ; cyano ; hydroxy ; alkyle ($C_1$-$C_6$) ; halogéno-alkyle ($C_1$-$C_6$) ; cyano-alkyle ($C_1$-$C_6$) ; alcoxy ($C_1$-$C_6$) ; halogéno-alcoxy ($C_1$-$C_6$) ; alcoxy-alkyle ayant indépendamment 1 à 6 atomes de carbone dans chaque groupe alkyle ; alcoxy-alcoxy ayant indépendamment 1 à 6 atomes de carbone dans chaque groupe alkyle ; le groupe $-OCO_2R$ ; alcényle ($C_2$-$C_6$) éventuellement substitué par halogène, cyano, alkyle ($C_1$-$C_4$), alcoxy ($C_1$-$C_4$), halogéno-alcoxy ($C_1$-$C_4$) ou alkylthio ($C_1$-$C_4$) ; le groupe $-RCO_2R'$ ; le groupe $-COR$ ; halogéno-alkyl ($C_1$-$C_6$)-carbonyle ; cyano-alkyl ($C_1$-$C_6$)-carbonyle ; nitro-alkyl ($C_1$-$C_6$)-carbonyle ; le groupe $-CO_2R$ ; halogéno-alcoxy ($C_1$-$C_6$) -carbonyle ; le groupe $-OCOR$ ; le groupe $-NRR'$ ; amino substitué par des groupes hydroxy, alcoxy($C_1$-$C_4$) ou alkylthio ($C_1$-$C_4$) ; phényl -amino ; diphénylamino ; le groupe $-CONRR'$ ; le groupe $-OCONRR'$ ; le groupe $-NRCOR'$ ; le groupe $-NRCO_2R'$ ; le groupe $-N(COR)COR'$ ; le groupe $-OCONRCOR'$ ; sulfhydryle ; halogéno-thio ; alkylthio ($C_1$-$C_6$) ; halogéno-alkylthio ($C_1$-$C_6$) ; le groupe

$-SOR$ ; le groupe $-SO_2R$ ; phénylsulfonyle ; le groupe $-OSO_2R$ ; halogéno-alkyl ($C_1$-$C_6$) -sulfonyloxy; le groupe $-SO_2NRR'$ ; le groupe $-NRSOR'$ ; le groupe $-NRSO_2R'$ ; le groupe $-CSR$ ; le groupe $-CS_2R$ ; le groupe $-NRCSR'$ ; le groupe $-SCOR$ ; phényle non substitué ou substitué ayant un à trois substituants identiques ou différents, choisis parmi halogène, cyano, nitro, hydroxy-alkyle ($C_1$-$C_4$), alcoxy ($C_1$-$C_4$), carboxy, alcoxy ($C_1$-$C_4$) -carbonyle, alcanoyloxy ($C_1$-$C_4$), le groupe $NH_2$ ; le groupe NHZ; ou le groupe NZZ' ; phénoxy où le cycle phényle est non substitué ou substitué par un à trois substituants identiques ou différents choisis parmi halogène, cyano, nitro, hydroxy, alkyle($C_1$-$C_4$), alcoxy($C_1$-$C_4$), carboxy, alcoxy($C_1$-$C_4$)-carbonyle, alcanoyloxy($C_1$-$C_4$), le groupe $NH_2$ ; le groupe NHZ ; ou le groupe NZZ' ; phényl-thio où le cycle phényle est non substitué ou substitué par un à trois substituants identiques ou différents choisis parmi halogène, cyano, nitro, hydroxy, alkyle($C_1$-$C_4$) alcoxy($C_1$-$C_4$) carboxy alcoxy($C_1$-$C_4$)-carbonyle, alcanoyloxy($C_1$-$C_4$), le groupe $NH_2$ ; le groupe NHZ ; ou le groupe NZZ'; ou lorsque deux positions adjacentes du cycle phényle sont substituées par des groupes alcoxy, ces groupes peuvent être reliés pour former, ensemble avec les atomes de carbone auxquels ils sont l'un et l'autre fixés, un cycle hétérocyclique dioxolano ou dioxano à 5 ou 6 membres ;

où R, R' et R" sont hydrogène ou alkyle($C_1$-$C_6$) ; Z et Z' sont alkyle($C_1$-$C_4$) ; "amino" signifie NRR' ; ou un sel agronomiquement acceptable de celui-ci, ensemble avec du diluant ou du support ou excipient agronomiquement acceptable.

2. Composition selon la revendication 1, dans laquelle :

X et X' sont O ou ; S et/ou

$R^1$ est alkyle($C_3$-$C_8$) ramifié, non substitué, ou alkyle($C_1$-$C_4$) à chaîne droite, substitué par un ou deux cycloalkyles($C_3$-$C_4$) identiques ou différents ; et/ou

A est alkyle($C_1$-$C_8$) non substitué ou substitué ayant un à trois substituants identiques ou différents, choisis parmi halogène, cyano, nitro, alcoxy($C_1$-$C_4$), carboxy, alcoxy($C_1$-$C_4$)-carbonyle, alcanoyloxy($C_1$-$C_4$), $NH_2$, NHZ ou NZZ' ;

cycloalkyle($C_2$-$C_6$) ;

alcényle ($C_2$-$C_6$) non substitué ou substitué, ayant 1 à 3 substituants identiques ou différents, choisis parmi halogène, alkyle ($C_1$-$C_4$) ; halogéno-alkyle ($C_1$-$C_4$), alcoxy ($C_1$-$C_4$) ou halogéno-alcoxy ($C_1$-$C_4$) ; cycloalcényle($C_3$-$C_6$) ; ou

phénylalkyle ayant 1 ou 2 atomes de carbone dans le groupe alkyle et le cycle phényle est non substitué ou substitué par un ou deux substituants identiques ou différents, choisis parmi halogène, nitro ou alkyle($C_1$-$C_4$) ; et/ou

B est naphtyle non substitué ; ou phényle non substitué ou substitué où les substituants peuvent être de un à trois, identiques ou différents, choisis parmi halogène ; nitro ; cyano ; alkyle($C_1$-$C_4$) ; halogéno-alkyle($C_1$-$C_4$) ; cyano-alkyle($C_1$-$C_4$) ; alcoxy($C_1$-$C_4$) ; alcoxy-alkyle ayant indépendamment 1 à 4 atomes de carbone dans chaque groupe alkyle ; le groupe $-COD^4$ ; carboxy ; alcoxy($C_1$-$C_4$)-carbonyle ; alcanoyloxy($C_1$-$C_4$) ; $NH_2$ ; NHZ ; NZZ' ; alkylthio($C_1$-$C_4$) ; le groupe $-CSD^4$ ; le groupe $-CS_2D^4$ ; le groupe $-SCOD^4$ ; phényle non substitué ou substitué ayant un à deux substituants identiques ou différents, choisis parmi halogène, nitro, alkyle($C_1$-$C_4$), alcoxy($C_1$-$C_4$), carboxy, alcoxy($C_1$-$C_4$)-carbonyle, alcanoyloxy($C_1$-$C_4$), $NH_2$, NHZ ou NZZ' ; phénoxy où le cycle phényle est non substitué ou substitué par un ou deux substituants identiques ou différents, choisis parmi halogène, nitro, alkyle($C_1$-$C_4$), alcoxy($C_1$-$C_4$), carboxy, alcoxy($C_1$-$C_4$)-carbonyle, alcanoyloxy($C_1$-$C_4$), $NH_2$, NHZ ou NZZ' ; ou lorsque deux positions adjacentes sur le cycle phényle sont substituées par des groupes alcoxy, ces groupes peuvent être reliés pour former, ensemble avec les atomes de carbone auxquels ils sont liés, un cycle hétérocyclique dioxolano ou dioxano à 5 ou 6 membres ;

où $D^4$ est hydrogène ou alkyle ($C_1$-$C_4$) ; Z et Z' sont alkyle ($C_1$-$C_4$).

3. Composition, selon la revendication 2, dans laquelle :
X et X' sont O ou S ; et/ou
$R^1$ est alkyle($C_3$-$C_8$) ramifié ; et/ou
A est alkyle($C_1$-$C_8$) non substitué ou substitué ayant 1 à 3 substituants identiques ou différents, choisis parmi halogène, cyano, nitro, carboxy ou alcoxy($C_1$-$C_4$)-carbonyle ;
cyclohexyle ;
cyclohexényle :
alcényle($C_2$-$C_6$) non substitué ou substitué ayant un à trois substituants identiques ou différents,choisis parmi halogène ou alkyle($C_1$-$C_4$) ; ou
benzyle où le cycle phényle est non substitué ou substitué par un ou deux substituants identiques ou différents, choisis parmi halogène, méthyle ou éthyle ; et/ou
B est naphtyle non substitué ; phényle non substitué ou substitué ayant un à trois substituants identiques ou différents, choisis parmi halogène ; nitro ; cyano ; alkyle($C_1$-$C_4$) ; halogénoalkyle ($C_1$-$C_4$) ; cyano-alkyle ($C_1$-$C_4$) ; alcoxy($C_1$-$C_4$) ; le groupe $-COD^4$ ; alcoxy($C_1$-$C_4$)-carbonyle ; alcanoyloxy ($C_1$-$C_4$) ; ou phényle non substitué ou substitué ayant un ou deux substituants identiques ou différents, choisis parmi halogène, nitro, alkyle ($C_1$-$C_4$), alcoxy($C_1$-$C_4$), carboxy, alcoxy ($C_1$-$C_4$)-carbonyle, alcanoyloxy ($C_1$-$C_4$), $NH_2$, NHZ ou NZZ', où $D^4$ est hydrogène ou alkyle($C_1$-$C_4$) ; Z et Z' sont alkyle($C_1$-$C_4$).
4. Composition selon la revendication 3, dans laquelle :
X et X' sont O ; et/ou
$R^1$ est alkyle($C_4$-$C_7$) ramifié ; et/ou
A est alkyle($C_1$-$C_6$) non substitué ou substitué ayant un à deux halogènes identiques ou différents ; cyclohexyle ;
alcényle($C_2$-$C_6$) non substitué ou substitué ayant un à trois substituants identiques ou différents, choisis parmi halogène, méthyle ou éthyle ;
cyclohexènyle ; ou
benzyle ; et/ou
B est phényle non substitué ou substitué où les substituants peuvent être de un à trois, identiques ou différents, choisis parmi halogène, alkyle ($C_1$-$C_4$), alcoxy ($C_1$-$C_4$), ou halogéno-alkyle ($C_1$-$C_4$).
5. Composition selon la revendication 4, dans laquelle :
X et X' sont O ; et/ou
$R^1$ est t-butyle, néopentyle (2,2-diméthylpropyle) ou 1,2,2-triméthylpropyle ; et/ou
A est alkyle($C_3$-$C_5$) non substitué ;
cyclohexyle ;
alcényle($C_2$-$C_4$) à chaîne droite, non substitué ou substitué, ayant un à trois substituants identiques ou différents, choisis parmi chlore, brome ou méthyle ;
cyclohexényle ; ou
benzyle ; et/ou
B est phényle non substitué ou substitué où les substituants peuvent être un ou deux, identiques ou différents, choisis parmi chlore, fluor, brome, iode, méthyle, éthyle, méthoxy ou trifluorométhyle.
6. Composition selon la revendication 1, dans laquelle :
X et X' sont O ;
$R^1$ est t-butyle ; et
B est phényle et A est cyclohexyle, n-butyle, n-propyle, benzyle, 1-cyclohexényle, dichlorométhyle ou trichlorométhyle ; ou
B est 4-chlorophényle et A est n-propyle ou t-butyle ; ou
B est 3-méthylphényle et A est norbornyle, cyclohex-3-ényle, 1-butényle, 3-butényle, cis-3,4-dihydroxycyclohexyle, 2,2-diméthyl-3a-4,5,6,7, 7a-hexahydrobenzo[d]dioxalano-5-yle, 3-acétyl-2,2-diméthyl-cyclobutyl-méthyle ou 2-méthylcyclohexyl-di-2,5-ényle ; ou
B est 3,5-diméthylphényle et A est isopropyle ou 1-méthyléthényle.

7. Composition telle que revendiquée à la revendication 2, dans laquelle B est phényle non substitué, 4-chlorophényle, phényle substitué en position 3, phényle substitué en position 2 ou phényle disubstitué.

8. Composition selon l'une quelconque des revendications précédentes, dans laquelle ledit ingrédient insecticide est présent à raison de 0,0001 à 99% en poids de la composition.

9. Composition selon la revendication 8, dans laquelle ledit ingrédient insecticide est présent à raison de 0,001 à 99% en poids de la composition.

10. Composition selon la revendication 8, dans laquelle ledit ingrédient insecticide est présent à raison de de 0,01 à 99% en poids de la composition.

11. Composition insecticide selon l'une quelconque des revendications précédentes, (a) contenant en

outre un agent émulsifiant, ladite composition étant sous la forme d'un concentré émulsifiable ; ou (b) contenant en outre un agent dispersant, ladite composition étant sous la forme d'une poudre mouillable ; ou (c) contenant un support ou excipient liquide agronomiquement acceptable et un agent dispersant, ladite composition étant sous une forme apte à s'écouler ; ou (d) sous la forme d'une poussière ; ou (e) contenant en outre un liant, ladite composition étant sous la forme d'un granule ; ou (f) contenant en outre un agent d'attraction, ladite composition étant sous la forme d'un appât.

12. Procédé pour contrôler les insectes, qui comprend la mise en contact desdits insectes avec une quantité efficace au plan insecticide d'ingrédient insecticide tel que défini dans l'une quelconque des revendications 1 à 6, éventuellement dans une composition selon l'une quelconque des revendications précédentes.

13. Procédé selon la revendication 12, dans lequel l'ingrédient insecticide est appliqué à des plantes cultivées ou à une zone où des plantes doivent être cultivées, à une posologie de 10 g à 10 kg par hectare.

14. Procédé selon la revendication 13, dans lequel le taux d'application est de 100 g à 5 kg par hectare.

15. Procédé selon la revendication 12 ou 13 pour tuer des insectes de l'ordre des lepidoptères ou des coléoptères.

## Revendications pour l'Etat Contractant : ES

1. Utilisation d'un composé insecticide ayant la formule :

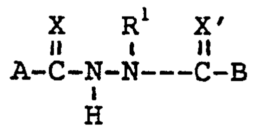

I

dans laquelle

X et X' qui sont identiques ou différents sont O, S ou NR ;

$R^1$ est alkyle($C_3$-$C_{10}$) ramifié, non substitué ou alkyle($C_1$-$C_4$) à chaîne droite, substitué par un ou deux substituants identiques ou différents cycloalkyles($C_3$-$C_6$) ;

A est alkyle($C_1$-$C_{10}$) substitué ou non substitué, ayant un à quatre substituants identiques ou différents, choisis parmi halogène, cyano, nitro, hydroxy, alcoxy($C_1$-$C_4$), carboxy, alcoxy-($C_1$-$C_4$)-carbonyle, alcanoyloxy($C_1$-$C_4$), $NH_2$, NHZ ou NZZ' ;

cycloalkyle($C_3$-$C_8$) ou cycloalkyl($C_3$-$C_8$)-alkyle-($C_1$-$C_4$), non substitué ou substitué, ayant un à quatre substituants identiques ou différents, choisis parmi halogène, cyano, nitro, hydroxy, alkyle($C_1$-$C_4$), halogéno-alkyle($C_1$-$C_4$), alcoxy. ($C_1$-$C_4$), halogéno-alcoxy($C_1$-$C_4$), carboxy, alcanoyle($C_1$-$C_4$), alcoxy($C_1$-$C_4$)-carbonyle, alcanoyloxy($C_1$-$C_4$), $NH_2$, NHZ ou NZZ' ;

alcényle($C_2$-$C_8$) non substitué ou substitué ou alcadiényle($C_3$-$C_8$) non substitué ou substitué ayant un à quatre substituants identiques ou différents, choisis parmi halogène, cyano, nitro, hydroxy, alkyle($C_1$-$C_4$), cycloalkyle($C_3$-$C_6$), halogéno-alkyle($C_1$-$C_4$), alcoxy($C_1$-$C_4$), halogéno-alcoxy($C_1$-$C_4$), carboxy, alcoxy($C_1$-$C_4$)-carbonyle, alcanoyloxy($C_1$-$C_4$), $NH_2$, NHZ ou NZZ' ; cycloalcényle($C_3$-$C_8$) non substitué ou substitué ou cycloalcadiényle($C_6$-$C_8$) non substitué ou substitué, ayant un à quatre substituants identiques ou différents, choisis parmi halogène, cyano, nitro, hydroxy, alkyle($C_1$-$C_4$), halogéno-alkyle($C_1$-$C_4$), alcoxy($C_1$-$C_4$), halogénoalcoxy($C_1$-$C_4$), carboxy, alcoxy($C_1$-$C_4$)-carbo nyle, alcanoyloxy($C_1$-$C_4$), $NH_2$, NHZ ou NZZ'.

Alcynyle($C_2$-$C_8$) non substitué ou substitué, ayant un à quatre substituants identiques ou différents/choisis parmi halogène, cyano, nitro, hydroxy, alkyle($C_1$-$C_4$), halogéno-alkyle($C_1$-$C_4$), alcoxy($C_1$-$C_4$), halogéno-alcoxy($C_1$-$C_4$), carboxy, alcoxy($C_1$-$C_4$)-carbonyle, alcanoyloxy($C_1$-$C_4$), $NH_2$, NHZ ou NZZ' ; ou phénylalkyle ayant un à quatre atomes de carbone dans le groupe alkyle et le cycle phényle est non substitué ou substitué par un à trois substituants identiques ou différents, choisis parmi halogène, cyano, nitro, hydroxy, alkyle($C_1$-$C_4$), halogéno-alkyle($C_1$-$C_4$), cyano-alkyle($C_1$-$C_4$), alcoxy($C_1$-$C_4$), halogéno-alcoxy($C_1$-$C_4$), carboxy, alcoxy($C_1$-$C_4$)-carbonyle, alcanoyloxy($C_1$-$C_4$), alcényle($C_2$-$C_6$), halogénoalcényle($C_2$-$C_6$), alcynyle($C_2$-$C_6$), $NH_2$, NHZ ou NZZ' ;

B est naphtyle non substitué ou substitué, où les substituants peuvent être de 1 à 3, identiques ou différents, choisis parmi halogène ; cyano ; nitro ; hydroxy ; alcoxy($C_1$-$C_4$) ; alkyle($C_1$-$C_4$) ; carboxy ; alcoxy($C_1$-$C_4$)-carbonyle ; alcanoyloxy-($C_1$-$C_4$), $NH_2$, NHZ ou NZZ' ;

phényle non substitué ou substitué où les substituants peuvent être de 1 à 5, identiques ou différents, choisis parmi halogène ; nitro ; cyano ; hydroxy ; alkyle($C_1$-$C_6$) ; halogéno-alkyle-($C_1$-$C_6$) ; cyano-alkyle($C_1$-$C_6$) ; alcoxy($C_1$-$C_6$) ; halogéno-alcoxy($C_1$-$C_6$) ; alcoxy-alkyle ayant indépendamment 1 à 6 atomes de carbone dans chaque groupe alkyle ; alcoxy-alcoxy ayant indépendamment 1 à 6 atomes de carbone dans chaque groupe alkyle ; le groupe $-OCO_2R$ ; alcényle($C_2$-$C_6$) éventuellement substitué par halogène, cyano, alkyle($C_1$-$C_4$), alcoxy($C_1$-$C_4$), halogéno-alcoxy($C_1$-$C_4$) ou alkylthio($C_1$-$C_4$) ; le groupe $-RCO_2R'$ ; le groupe $-COR$ ; halogénoalkyl($C_1$-$C_6$)-carbonyle ; cyano-alkyl($C_1$-$C_6$)-carbonyle ; nitro-alkyl($C_1$-$C_6$)-carbonyle, le groupe $-CO_2R$ ; halogéno-alcoxy($C_1$-$C_6$)-carbonyle, le groupe $-OCOR$ ; le groupe $-NRR'$ ; aminosubstitué par des groupes hydroxy, alcoxy($C_1$-$C_4$) ou alkylthio($C_1$-$C_4$) ; phénylamino ; diphénylamino ; le groupe $-CONRR'$ ; le groupe $-OCONRR'$ ; un groupe $-NRCOR'$, le groupe $-NRCO_2R'$ ; le groupe $-N(COR)COR'$ ; le groupe $-OCONRCOR'$ ; sulphydryle ; halogénothio ; alkylthio($C_1$-$C_6$) ; halogéno-alkylthio($C_1$-$C_6$) ; le groupe $-SOR$ ; le groupe $-SO_2R$ ; phénylsulfonyle ; le groupe $-OSO_2R$ ; halogéno-alkylsulfonyloxy($C_1$-$C_6$) ; le groupe $-SO_2NRR'$ ; le groupe $-NRSOR'$ ; le groupe $-NRSO_2R'$ ; le groupe $-CSR$ ; le groupe $-CS_2R$ ; le groupe $-NRCSR'$ ; le groupe $-SCOR$ ; phényle non substitué ou substitué ayant un à trois substituants identiques ou différents, choisis parmi halogène, cyano, nitro, hydroxyalkyle($C_1$-$C_4$), alcoxy($C_1$-$C_4$), carboxy, alcoxy($C_1$-$C_4$)-carbonyle, alcanoyloxy($C_1$-$C_4$), le groupe $NH_2$ ; le groupe NHZ ou le groupe NZZ' ; phénoxy où le cycle phényle est non substitué ou substitué par un à trois substituants identiques ou différents, choisis parmi halogène, cyano, nitro, hydroxy, alkyle($C_1$-$C_4$), alcoxy($C_1$-$C_4$), carboxy, alcoxy($C_1$-$C_4$)-carbo nyle, alcanoyloxy($C_1$-$C_4$), le groupe $NH_2$ ; le groupe NHZ ; ou le groupe NZZ' ; phénylthio où le cycle phényle est non substitué ou substitué par un à trois substituants identiques ou différents, choisis parmi halogène, cyano, nitro, hydroxy, alkyle($C_1$-$C_4$), alcoxy($C_1$-$C_4$), carboxy, alcoxy($C_1$-$C_4$)-carbonyle, alcanoyloxy ($C_1$-$C_4$), le groupe $NH_2$ ; le groupe NHZ ; ou le groupe NZZ' ; ou, lorsque deux positions adjacentes du cycle phényle sont substituées par des groupes alcoxy, ces groupes peuvent être reliés pour former, ensemble avec les atomes de carbone auxquels ils sont l'un et l'autre fixés, un cycle hétérocyclique dioxolano ou dioxano à 5 ou 6 membres ;

où R, R' et R" sont hydrogène ou alkyle($C_1$-$C_6$) ; Z et Z' sont alkyle($C_1$-$C_4$) ; "amino" signifie NRR' ; ou un sel agronomiquement acceptable de celui-ci, ensemble avec du diluant ou du support ou excipient agronomiquement acceptable, dans la fabrication d'une composition insecticide.

2. Utilisation selon la revendication 1, d'un composé ou d'un sel dans lequel :

X et X' sont O ou S ; et /ou

$R^1$ est alkyle($C_3$-$C_8$) ramifié, non substitué ou alkyle($C_1$-$C_4$) à chaîne droite, substitué par un ou deux substituants identiques ou différents cycloalkyle ($C_3$-$C_4$) ; et/ou

A est alkyle($C_1$-$C_6$) non substitué ou substitué ayant un à trois substituants identiques ou différents, choisis parmi halogène, cyano, nitro, alcoxy ($C_1$-$C_4$), carboxy, alcoxy ($C_1$-$C_4$) -carbonyle, alcanoyloxy ($C_1$-$C_4$), $NH_2$, NHZ ou NZZ' ; cycloalkyle ($C_3$-$C_6$) ;

alcényle ($C_2$-$C_6$) non substitué ou substitué ayant 1 à 3 substituants identiques ou différents choisis parmi halogène, alkyle ($C_1$-$C_4$) ; halogéno-alkyle ($C_1$-$C_4$), alcoxy ($C_1$-$C_4$) ou halogénoalcoxy ($C_1$-$C_4$) ; cycloalcényle($C_3$-$C_6$) ; ou

phénylalkyle ayant 1 ou 2 atomes de carbone dans le groupe alkyle et le cycle phényle est non substitué ou substitué par un ou deux substituants identiques ou différents, choisis parmi halogène, nitro ou alkyle($C_1$-$C_4$) ; et /ou

B est naphtyle non substitué ; ou phényle non substitué ou substitué où les substituants peuvent être de un à trois, identiques ou différents, choisis parmi halogène ; nitro ; cyano ; alkyle($C_1$-$C_4$) ; halogéno-alkyle($C_1$-$C_4$) ; cyano-alkyle($C_1$-$C_4$) ; alcoxy($C_1$-$C_4$) ; alcoxy-alkyle ayant indépendamment 1 à 4 atomes de carbone dans chaque groupe alkyle ; le groupe $-COD^4$ ; carboxy ; alcoxy($C_1$-$C_4$)-carbonyle ; alcanoyloxy($C_1$-$C_4$) ; $NH_2$ ; NHZ ; NZZ' ; alkylthio($C_1$-$C_4$) ; le groupe $-CSD^4$ ; le groupe $-CS_2D^4$ ; le groupe $-SCOD^4$ ; phényle non substitué ou substitué ayant un à deux substituants identiques ou différents, choisis parmi halogène, nitro, alkyle($C_1$-$C_4$), alcoxy($C_1$-$C_4$), carboxy, alcoxy($C_1$-$C_4$)-carbonyle, alcanoyloxy. ($C_1$-$C_4$), $NH_2$, NHZ ou NZZ' ; phénoxy où le cycle phényle est non substitué ou substitué par un ou deux substituants identiques ou différents, choisis parmi halogène, nitro, alkyle($C_1$-$C_4$), alcoxy($C_1$-$C_4$), carboxy, alcoxy($C_1$-$C_4$)-carbo nyle, alcanoyloxy($C_1$-$C_4$), $NH_2$, NHZ ou NZZ' ; ou lorsque deux positions adjacentes sur le cycle phényle sont substituées par des groupes alcoxy, ces groupes peuvent être reliés pour former, ensemble avec les atomes de carbone auxquels ils sont fixés, un cycle hétérocyclique dioxolano ou dioxano à 5 ou 6 membres ;

où $D^4$ est hydrogène ou alkyle ($C_1$-$C_4$) ; Z et Z' sont alkyle ($C_1$-$C_4$).

3. Utilisation, selon la revendication 2, d'un composé ou sel, dans lequel :

X et X' sont O ou S ; et/ou

$R^1$ est alkyle($C_3$-$C_8$) ramifié ; et/ou

A est alkyle($C_1$-$C_6$) non substitué ou substitué ayant 1 à 3 substituants identiques ou différents, choisis parmi halogène, cyano, nitro, carboxy ou alcoxy($C_1$-$C_4$)-carbonyle ;

cyclohexyle ;
cyclohexényle :
alcényle ($C_2$-$C_6$) non substitué ou substitué ayant un à trois substituants identiques ou différents, choisis parmi halogène ou alkyle ($C_1$-$C_4$) ; ou
benzyle où le cycle phényle est non substitué ou substitué par un ou deux substituants identiques ou différents, choisis parmi halogène, méthyle ou éthyle ; et/ou
B est naphtyle non substitué ; phényle non substitué ou substitué ayant un à trois substituants identiques ou différents, choisis parmi halogène ; nitro ; cyano ; alkyle($C_1$-$C_4$) ; halogénoalkyle($C_1$-$C_4$) ; cyano-alkyle($C_1$-$C_4$) ; alcoxy-($C_1$-$C_4$) ; le groupe $-COD^4$ ; alcoxy($C_1$-$C_4$)-carbonyle ; alcanoyloxy ($C_1$-$C_4$) ; ou phényle non substitué ou substitué ayant un ou deux substituants identiques ou différents, choisis parmi halogène, nitro, alkyle ($C_1$-$C_4$), alcoxy ($C_1$-$C_4$), carboxy, alcoxy ($C_1$-$C_4$)-carbonyle, alcanoyloxy ($C_1$-$C_4$), $NH_2$, NHZ ou NZZ' ; où $D^4$ est hydrogène ou alkyle($C_1$-$C_4$) ; Z et Z' sont alkyle ($C_1$-$C_4$).
4. Utilisation, selon la revendication 3, d'un composé ou sel dans lequel
X et X' sont O ; et/ou
$R^1$ est alkyle($C_4$-$C_7$) ramifié ; et/ou
A est alkyle($C_1$-$C_6$) non substitué ou substitué ayant un à deux halogènes identiques ou différents ;
cyclohexyle ;
alcényle($C_2$-$C_6$) non substitué ou substitué ayant un à trois substituants identiques ou différents, choisis parmi halogène, méthyle ou éthyle ;
cyclohexènyle ; ou
benzyle ; et/ou
B est phényle non substitué ou substitué où les substituants peuvent être de un à trois, identiques ou différents, choisis parmi halogène, alkyle($C_1$-$C_4$) ; alcoxy($C_1$-$C_4$) ; ou halogéno-alkyle($C_1$-$C_4$).
5. Utilisation, selon la revendication 4, d'un composé ou sel dans lequel :
X et X' sont O ; et/ou
$R^1$ est t-butyle, néopentyle (2,2-diméthyl-propyle) ou 1,2,2-triméthylpropyle ; et/ou
A est alkyle($C_3$-$C_5$) non substitué ;
cyclohexyle ;
alcényle($C_2$-$C_4$) non substitué ou substitué à chaîne droite, ayant un à trois substituants identiques ou différents choisis parmi chlore, brome ou méthyle ;
cyclohexényle ; ou
benzyle ; et/ou
B est phényle non substitué ou substitué où les substituants peuvent être un ou deux, identiques ou différents, choisis parmi chlore, fluor, brome, iode, méthyle, éthyle, méthoxy ou trifluorométhyle.
6. Utilisation, selon la revendication 1, d'un composé ou sel dans lequel :
X et X' sont O ;
$R^1$ est t-butyle ; et
B est phényle et A est cyclohexyle, n-butyle, n-propyle, benzyle, 1-cyclohexényle, dichlorométhyle ou trichlorométhyle ; ou
B est 4-chlorophényle et A est n-propyle ou t-butyle ; ou
B est 3-méthylphényle et A est norbornyle, cyclohex-3-ényle, 1-butényle, 3-butényle, cis-3,4-dihydroxycyclohexyle, 2,2-diméthyl-3a-4,5,6,7, 7a-hexahydrobenzo[d]dioxalano-5-yle, 3-acétyl-2,2-diméthyl-cyclobutyl-méthyle ou 2-méthylcyclohexyl-di-2,5-ényle ; ou
B est 3,5-diméthylphényle et A est isopropyle ou 1-méthyléthényle.

7. Utilisation selon la revendication 2 du composé ou sel, dans lequel B est phényle non substitué, 4-chlorophényle, phényle substitué en position 3, phényle substitué en position 2 ou phényle disubstitué.

8. Utilisation selon l'une quelconque des revendications précédentes, du composé ou sel en une quantité de 0,0001 à 99% en poids de la composition.

9. Utilisation selon la revendication 8, du composé ou sel en une quantité de 0,001 à 99% en poids de la composition

10. Utilisation selon la revendication 8, du composé ou sel en une quantité de 0,01 à 99% en poids de la composition.

11. Utilisation selon l'une des revendications précédentes du composé ou sel (a) ensemble avec de l'agent émulsifiant, dans la fabrication d'un concentré émulsifiable ; ou (b) ensemble avec un agent dispersant dans la fabrication d'une poudre mouillable ; ou (c) ensemble avec un support ou excipient liquide agronomiquement acceptable et un agent dispersant dans la fabrication d'une composition apte à s'écouler ; ou (d) sous la forme d'une poussière ; ou (e) ensemble avec un liant dans la fabrication d'une formulation granulaire ; ou (f) ensemble avec un agent d'attraction dans la fabrication d'un appât.

12. Utilisation d'un composé ou sel tel que défini dans l'une quelconque des revendications 1 à 7, éventuellement dans une composition contenant également du diluant ou du support ou excipient agronomiquement acceptable, pour le contrôle des insectes par mise en contact desdits insectes avec une quantité efficace au plan insecticide du composé ou sel.

13. Procédé mécanique pour améliorer la valeur commerciale et/ou le caractère avantageux de récoltes vendables de plantes dont la croissance est affectée ou susceptible d'être affectée par des insectes comprenant (1) le chargement dans un conteneur, un dispositif de fumigation ou un dispositif de dissémination mécanique d'un composé ou sel insecticide tel que défini dans l'une quelconque des revendications 1 à 7, éventuellement dans un mélange avec du diluant ou du support ou excipient agronomiquement acceptable, (2) l'utilisation du conteneur, fumigateur ou dispositif mécanique de dissémination pour appliquer le composé ou sel insecticide, sous forme de granules, de poussière, de fumée, de vapeur ou de préparation liquide contenant de l'agent tensio-actif à des plantes en culture ou à un milieu de culture où les plantes sont en culture ou doivent être mises en culture, ou aux insectes eux-mêmes, (3) le contrôle ou la régulation de la dose de l'ingrédient actif pendant cette étape d'application de sorte que le taux d'application du composé insecticide actif soit suffisant pour combattre les insectes mais insuffisant pour provoquer un effet nocif inacceptable sur les plantes récoltées croissant ou devant croître dans la zone traitée.

14. Utilisation ou procédé selon la revendication 12 ou 13, où le composé ou sel insecticide est appliqué à des plantes cultivées ou à une surface où les plantes doivent être cultivées à une posologie de 10 g à 10 kg par hectare.

15. Utilisation ou procédé selon la revendication 14, où le taux d'application est de 100 g à 5 kg par hectare.

16. Utilisation ou procédé selon la revendication 13 ou 14 pour tuer des insectes de l'ordre des lepidoptères ou des coléoptères.